# EUROPEAN PATENT SPECIFICATION

(11) **EP 3 049 078 B1**
(45) Date of publication and mention of the grant of the patent: **27.06.2018**
(21) Application number: 14849624.3
(22) Date of filing: 26.09.2014
(51) Int. Cl.: A61K 31/404

(54) **TREATMENT OF CANCER**
KREBSBEHANDLUNG
TRAITEMENT DU CANCER

(30) Priority: 27.09.2013 US 201361883487 P
(43) Date of publication of application: 03.08.2016
(73) Proprietor: BlueLink Pharmaceuticals, Inc., Ames, Iowa 50010 (US)
(72) Inventor: GIACCIA, Amato, Stanford, CA 94305-5152 (US); NOE, Jeanne, Chapel Hill, NC 27517 (US); SANOFF, Hanna, Chapel Hill, NC 27517 (US); TEPPER, Joel, E., Chapel Hill, NC 27517 (US); WANG, Andrew, Z., Chapel Hill, NC 27517 (US)
(74) Representative: Simpson, Tobias Rutger
(86) International application number: PCT/US2014/057749
(87) International publication number: WO 2015/048467

(56) References cited:
- WO-A1-2012/125232
- WO-A1-2013/037789
- WO-A1-2013/059651
- WO-A1-2014/055913
- WO-A2-00/38717
- US-A1- 2007 036 717
- US-A1- 2007 036 717
- US-A1- 2013 164 282
- US-A1- 2013 164 282
- US-A1- 2013 209 518
- HOFHEINZ R-D ET AL: "PHASE I TRIAL OF CAPECITABINE AND WEEKLY IRINOTECAN IN COMBINATION WITH RADIOTHERAPY FOR NEOADJUVANT THERAPY OF RECTAL CANCER", JOURNAL OF CLINICAL ONCOLOGY, AMERICAN SOCIETY OF CLINICAL ONCOLOGY, US, vol. 23, no. 7, 1 March 2005 (2005-03-01), pages 1350-1357, XP009052136, ISSN: 0732-183X, DOI: 10.1200/JCO.2005.04.171
- G KLAUTKE ET AL: "Concurrent chemoradiation with capecitabine and weekly irinotecan as preoperative treatment for rectal cancer: results from a phase I/II study", BRITISH JOURNAL OF CANCER, vol. 94, no. 7, 10 April 2006 (2006-04-10) , pages 976-981, XP055353819, GB ISSN: 0007-0920, DOI: 10.1038/sj.bjc.6603053
- GLEN J. WEISS ET AL: "First-in-human phase 1/2a trial of CRLX101, a cyclodextrin-containing polymer-camptothecin nanopharmaceutical in patients with advanced solid tumor malignancies", INVESTIGATIONAL NEW DRUGS., vol. 31, no. 4, 9 February 2013 (2013-02-09), pages 986-1000, XP055353714, US ISSN: 0167-6997, DOI: 10.1007/s10637-012-9921-8
- KIM ET AL.: 'Update and Debate Issues in Surgical Treatment of Middle and Low Rectal Cancer' J KOREAN SOC COLOPROCTOL vol. 28, no. 5, 31 October 2012, pages 230 - 241, XP055329383

## Description

### Claim of Priority

This application claims priority to U.S.S.N. 61/883,487 filed September 27, 2013, the entire disclosure of which is incorporated herein by reference.

### Background of the Invention

Drug delivery and dosing of small molecule therapeutic agents, such as camptothecin, can be problematic due to a number issues including half-life, toxicity, distribution etc.

### Summary of the Invention

In one aspect, the disclosure features, a method of treating a cancer, e.g. colorectal cancer, e.g., rectal cancer, in a subject with a cyclodextrin containing polymer ("CDP") - camptothecin conjugate, particle or composition or camptothecin derivative conjugate, particle or composition described herein, e.g., CRLX101. The method comprises:
providing an initial administration of a CDP-camptothecin conjugate, particle or composition or camptothecin derivative conjugate, particle or composition described herein, e.g., CRLX101, to said subject at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m², 18 mg/m², (wherein said dosage is expressed in mg of drug, as opposed to mg of conjugate),
providing one or more subsequent administrations of said CDP-camptothecin conjugate, particle or composition or camptothecin derivative conjugate, particle or composition described herein, e.g., CRLX101, at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m², 18 mg/m², wherein each subsequent administration is provided, independently, between 12, 13, 14, 15 or 16 days, after the previous, e.g., the initial, administration, and
providing multiple radiation treatments, e.g., pelvic radiation treatments, wherein an initial radiation treatment, e.g., pelvic radition treatment, is administered with the administration of said CDP-camptothecin conjugate, particle or composition or camptothecin derivative conjugate, particle or composition described herein, e.g., CRLX101, e.g., with the initial administration, of said CDP-camptothecin conjugate, particle or composition or camptothecin derivative conjugate, particle or composition described herein, e.g., CRLX101, and said radiation treatments, e.g., pelvic radiation treatment, are administered daily five days a week, e.g., on weekdays, e.g., every day of the week except Saturday and Sunday, e.g., for at least 20 to 40 days, e.g., 25 to 35 days, e.g., 25, 26, 27, 28, 29, 30, 31, 32, 33, 34, 35 days, to thereby treat the cancer, e.g. colorectal cancer, e.g., rectal cancer.

In one embodiment, said initial radiation treatment, e.g., pelvic radiation treatment, is administered within 24 hours, e.g., within about 24 hours, within about 22 hours, within about 20 hours, within about 18 hours, within about 16 hours, within about 14 hours, within about 12 hours, within about 10 hours, within about 8 hours, within about 6 hours, within about 4 hours, within about 2 hours, within about 1 hour, of administration of said CDP-camptothecin conjugate, particle or composition or camptothecin derivative conjugate, particle or composition described herein, e.g., CRLX101.

In one embodiment, said initial administration and/or one or more subsequent administrations of said CDP-camptothecin conjugate, particle or composition or camptothecin derivative conjugate, particle or composition described herein, e.g., CRLX101, is administered at a dosage of 9 mg/m², 10 mg/m², 12 mg/m², or 15 mg/m² (wherein said dosage is expressed in mg of drug, as opposed to mg of conjugate).

In an embodiment, the dosage of at least 2, 3, 4, 5, 6, 7, 8, 9, 10, 12, 15 or 20 administrations of the CDP-camptothecin conjugate, particle or composition or camptothecin derivative conjugate, particle or composition described herein, e.g., CRLX101, is the same.

In an embodiment, the time between at least 2, 3, 4, 5, 6, 7, 8, 9, 10, 12, 15, or 20 administrations of the CDP-camptothecin conjugate, particle or composition or camptothecin derivative conjugate, particle or composition described herein, e.g., CRLX101, is the same.

In an embodiment, each subsequent administration of the CDP-camptothecin conjugate, particle or composition or camptothecin derivative conjugate, particle or composition described herein, e.g., CRLX101, is administered 9-15 days, e.g., 9 days, 12 days or 14 days, after the previous administration.

In an embodiment, at least 2, 3, 4, 5, 6, 7, 8, 9, 10, 12, 15, 20, 50 or 100 administrations of the CDP-camptothecin conjugate, particle or composition or camptothecin derivative conjugate, particle or composition described herein, e.g., CRLX101, are administered to said subject.

In one embodiment, the CDP-camptothecin or camptothecin derivative, a CDP-camptothecin or camptothecin derivative conjugate, particle or composition described herein, e.g., CRLX101, is administered by intravenous administration over a period equal to or less than about 30 minutes, 45 minutes, 60 minutes, 90 minutes, 120 minutes, 150 minutes, or 180 minutes. In one embodiment, the CDP-camptothecin or camptothecin derivative conjugate, particle or composition, e.g., the CDP-camptothecin or camptothecin derivative conjugate, particle or composition described herein, e.g. CRLX101, is administered at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m², or 18 mg/m², by intravenous administration over a period equal to or less than about 30 minutes, 45 minutes, 60 minutes or 90 minutes, e.g., a period equal to or less than 30 minutes, 45 minutes or 60 minutes.

In some embodiments, the radiation treatment, e.g., pelvic radiation, is administered at a dosage of about 160 cGy to about 200 cGy, e.g., about 170 cGy to about 190 cGy, e.g., about 180 cGy per treatment.

In an embodiment, the dosage of at least 2, 3, 4, 5, 6, 7, 8, 9, 10, 11, 12, 13, 14, 15, 16, 17, 18, 19, 20, 21, 22, 23,24, 25, 26, 27, 28, 29, 30, 31, 32, 33, 34 or 35 of the radiation treatments, e.g., pelvic raditation, are the same.

In an embodiment, at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 radiation treatments, e.g., pelvic radition, are administered to said subject.

In an embodiment, the total amount of radiation, e.g., pelvic radition, given during the multiple radiation treatments is 6,000 cGy to about 4,000 cGy, about 5,900 cGy to about 4,100 cGy, about 5,800 cGy to about 4,200 cGy, about 5,700 cGy to about 4,300 cGy, about 5,600 cGy to about 4,400 cGy, e.g., about 5,400 cGy to about 4,500 cGy.

In some embodiments, the cancer, e.g., colorectal cancer, e.g., rectal cancer, is an adenocarcinoma. In some embodiments, the adenocarcinoma is a mucinous adenocarcinoma or a signet ring cell adenocarcinoma. Exemplary adenocarcinomas include: gastrointestinal carcinoid tumors; gastrointestinal stromal tumors; primary colorectal lymphoma; leiomyosarcoma; melanoma and squamous cell carcinoma. In an embodiment, the cancer is rectal cancer, e.g., locally advanced rectal cancer (e.g., stage cT3-4N0 or cT1-4N+).

In one embodiment, the CDP-camptothecin or camptothecin derivative conjugate, particle or composition, e.g., a CDP-camptothecin or camptothecin derivative conjugate, particle or composition described herein, e.g., CRLX101, is administered in combination with one or more additional chemotherapeutic agent, e.g., a chemotherapeutic agent (such as a pyrimidine analogue (e.g., capecitabine, cytarabine, gemcitabine, 5FU, floxuridine, 6-azauracil)) or combination of chemotherapeutic agents described herein.

In one embodiment, the CDP-camptothecin or camptothecin derivative conjugate, particle or composition, e.g., a CDP-camptothecin or camptothecin derivative conjugate, particle or composition described herein, e.g., CRLX101, is administered prior to surgery, after surgery or before and after surgery to remove the cancer, e.g., to remove the primary tumor and/or a metastases.

In one embodiment, the CDP-camptothecin or camptothecin derivative conjugate, particle or composition described herein, e.g., CRLX101, is administered prior to surgery to remove the cancer, e.g., to remove the primary tumor and/or a metastases.

In an embodiment, the method further comprises administering to said subject a treatment that reduces one or more side effects associated with administration of a CDP-camptothecin or camptothecin derivative conjugate, particle or composition, e.g., a treatment described herein.

In some embodiments, the method further comprises administering an agent which ameliorates bladder toxicity associated with therapy, e.g., an agent which increases urinary excretion and/or neutralizes one or more urinary metabolite. In one embodiment, the agent which ameliorates bladder toxicity associated with therapy, e.g., the agent which increases urinary excretion and/or neutralizes one or more urinary metabolite, is administered prior to, concurrently with and/or after administration with the CDP-camptothecin or camptothecin derivative conjugate, particle or composition. In one embodiment, the agent which ameliorates bladder toxicity associated with therapy, e.g., the agent which increases urinary excretion and/or neutralizes one or more urinary metabolite, is administered prior to and after administration with the CDP-camptothecin or camptothecin derivative conjugate, particle or composition. In one embodiment, the agent which ameliorates bladder toxicity associated with therapy is saline, e.g., intravenous saline, D5 half normal saline or D5 water. In one embodiment, the agent which increases urinary excretion and/or neutralizes one or more urinary metabolite is 2-mercaptoethane sulfonate sodium (MESNA). In one embodiment, the agent which ameliorates bladder toxicity associated with therapy is 2-mercaptoethane sulfonate sodium (MESNA) and the MESNA is administered intravenously at a dose of about 10%, 20%, 30% the dose of the camptothecin or camptothecin derivative and/or the MESNA is administered orally at a dose of about 20%, 30%, 40%, 50% the dose of the camptothecin or camptothecin derivative.

In one embodiment, the method further comprises administering an agent which reduces or inhibits one or more symptom of hypersensitivity to the subject. Symptoms of hypersensitivity include: injection site reaction, dyspnea, hypotension, angioedema, urticaria, bronchospasm and erythema. In one embodiment, the agent which reduces or inhibits one or more symptoms of hypersensitivity can be one or more of a corticosteroid (e.g., dexamethasone), an antihistamine (e.g., diphenhydramine), and an H2 antagonist (e.g., ranitidine or famotidine). In one embodiment, the agent is a corticosteroid (e.g., dexamethasone) and the corticosteroid is administered at 5, 10, 15, 20, 25 or 30 mg. In one embodiment, the corticosteroid is administered about 12, 11, 10, 9, 8, 7, 6, 5, 4, and/or 3 hours before administration of the CDP-camptothecin or camptothecin derivative conjugate, particle or composition, or the corticosteroid is administered intravenously about 40, 30, 20 minutes before the CDP-camptothecin or camptothecin derivative conjugate, particle or composition. In one embodiment, the agent is an antihistamine (e.g., diphenhydramine) and the antihistamine is administered at 15, 20, 25, 30, 35, 40, 45, 50, 55, 60, 65 or 70 mg. In one embodiment, the antihistamine is administered intravenously about 40, 30, 20, 10 minutes before the CDP-camptothecin or camptothecin derivative conjugate, particle or composition. In one embodiment, the agent is an H2 antagonist (e.g., ranitidine or famotidine) and the H2 antagonist is administered at 10, 15, 20, 25, 30, 35, 40, 45, 50, 55, 60, 65 or 70 mg. In one embodiment the H2 antagonist is administered intravenously about 70, 60, 50, 40, 30, 20, 10 minutes before the CDP-camptothecin or camptothecin derivative conjugate, particle or composition.

In one embodiment, the method further comprises administering an agent which reduces or inhibits nausea and/or vomiting, e.g., an antiemetic, e.g., an antiemetic described herein.

In another embodiment, the method further comprises administering to said subject a treatment that reduces one or more side effects associated with administration of radiation treatment. For example, in one embodiment, the method further comprises administering an agent that ameliorates a side effect associated with radiation treatment. In one embodiment, the agent that ameliorates a side effect associated with radiation treatment, is administered prior to, concurrently with and/or after administration of the radiation treatment. In one embodiment, the agent that ameliorates a side effect associated with radiation treatment, is administered prior to and after administration of the radiation treatment.

In one embodiment, the agent that ameliorates a side effect associated with radiation treatment is a radiation protector. In another embodiment, the agent that ameliorates a side effect associated with radiation treatment is a corticosteroid ((e.g., dexamethasone). In another embodiment, the agent that ameliorates a side effect associated with radiation treatment is an agent (e.g., a topical agent) that treats radiation damage to the skin such as, e.g., sucralfate. In another embodiment, the agent that ameliorates a side effect associated with radiation treatment is an agent that stimulates growth of epithelial cells such as, e.g., keratinocyte growth factor (KGF, palifermin). In another embodiment, the agent that ameliorates a side effect associated with radiation treatment is an antioxidant that reduces radiation-induced tissue injury such as, e.g., an antioxidant (e.g., Cu/Zn superoxide dismutase (SOD)). In another embodiment, the agent that ameliorates a side effect associated with radiation treatment is an agent that stimulates platelet recovery in the subject such as, e.g., interleukin 11. In another embodiment, the agent that ameliorates a side effect associated with radiation therapy is an agent that treats inflammation and wounds such as, e.g., prostaglandin (e.g., misoprostol).

In one embodiment, the subject has not been administered a CDP-camptothecin or camptothecin derivative conjugate, particle or composition, a CDP-camptothecin or camptothecin derivative conjugate, particle or composition described herein, e.g., CRLX101, prior to the initial administration.

In an embodiment, the CDP-camptothecin or camptothecin derivative conjugate, particle or composition, a CDP-camptothecin or camptothecin derivative conjugate, particle or composition described herein, e.g., CRLX101, is administered as a first line treatment for the cancer, e.g. colorectal cancer, e.g., rectal cancer.

In an embodiment, the CDP-camptothecin or camptothecin derivative conjugate, particle or composition, a CDP-camptothecin or camptothecin derivative conjugate, particle or composition described herein, e.g., CRLX101, is administered as a second, third or fourth line treatment for the cancer, e.g. colorectal cancer, e.g., rectal cancer.

In an embodiment, the cancer, e.g. colorectal cancer, e.g., rectal cancer, is sensitive to one or more chemotherapeutic agents, e.g., a platinum based agent, a taxane, an alkylating agent, an anthracycline (e.g., doxorubicin (e.g., liposomal doxorubicin)), an antimetabolite and/or a vinca alkaloid. In an embodiment, the cancer, e.g. colorectal cancer, e.g., rectal cancer, is a refractory, relapsed or resistant to one or more chemotherapeutic agents, e.g., a platinum based agent, a taxane, an alkylating agent, an antimetabolite and/or a vinca alkaloid.

In one embodiment, the method further comprises obtaining a sample, e.g., a biopsy sample, from the subject after an intital course of treatment, and determining if the subject has a pCR. In one embodiment, the subject has a pathological complete response (pCR), e.g., after one course of treatment. In one embodiment, the subject has a pCR after one course of treatment and the subject is administered one or more additional courses of treatment. In one embodiment, the subject does not have a pathological complete response (pCR), e.g., after one course of treatment. In one embodiment, the subject does not have a pCR after one course of treatment and the subject is administered one or more additional courses of treatment. In one embodiment, if the subject does not have a pCR after two courses of treatment, the subject is not given any further courses of treatment. In another embodiment, the subject does not have a pCR, e.g., after one course of treatment, and the subject is administered a chemotherapeutic agent other than the CDP-camptothecin or camptothecin derivative conjugate, particle or composition. In one embodiment, the subject does not have a pCR, e.g., after two courses of treatment, and the subject is administered a chemotherapeutic agent other than the CDP-camptothecin or camptothecin derivative conjugate, particle or composition.

In another aspect, the disclosure features, a method of treating rectal cancer (e.g., locally advanced rectal cancer (e.g., stage cT3-4N0 or cT1-4N+)), in a subject with a CDP-camptothecin conjugate, particle or composition or camptothecin derivative conjugate, particle or composition described herein, e.g., CRLX101. The method comprises:
providing an initial administration of a CDP-camptothecin conjugate, particle or composition or camptothecin derivative conjugate, particle or composition described herein, e.g., CRLX101, to said subject at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m², 18 mg/m², (wherein said dosage is expressed in mg of drug, as opposed to mg of conjugate),
providing one or more subsequent administrations of said CDP-camptothecin conjugate, particle or composition or camptothecin derivative conjugate, particle or composition described herein, e.g., CRLX101, at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m², 18 mg/m², wherein each subsequent administration is provided, independently, between 12, 13, 14, 15 or 16 days, after the previous, e.g., the initial, administration,
providing multiple radiation treatments, e.g., pelvic raditation, wherein an initial radiation treatment, e.g., pelvic radiation, is administered with the administration of said CDP-camptothecin conjugate, particle or composition or camptothecin derivative conjugate, particle or composition described herein, e.g., CRLX101, e.g., with the initial administration, of said CDP-camptothecin conjugate, particle or composition or camptothecin derivative conjugate, particle or composition described herein, e.g., CRLX101, and said radiation treatments, e.g., pelvic radiation treatments, are administered daily five days a week, e.g., on weekdays, e.g., every day of the week except Saturday and Sunday, for at least 25 to 35 days, e.g., 25, 26, 27, 28, 29, 30, 31, 32, 33, 34, 35 days, to thereby treat the rectal cancer, and
administering multiple doses of a pyrimidine analogue, e.g., capecitabine or 5FU, to thereby treat the rectal cancer.

In one embodiment, said initial radiation treatment, e.g., pelvic radiation treatment, is administered within 24 hours, e.g., within about 24 hours, within about 22 hours, within about 20 hours, within about 18 hours, within about 16 hours, within about 14 hours, within about 12 hours, within about 10 hours, within about 8 hours, within about 6 hours, within about 4 hours, within about 2 hours, within about 1 hour, of administration of said CDP-camptothecin conjugate, particle or composition or camptothecin derivative conjugate, particle or composition described herein, e.g., CRLX101.

In one embodiment, said doses of the pyrimidine analogue, e.g., capecitabine, are administered twice daily five days a week, e.g., on weekdays, e.g., every day of the week except Saturday and Sunday. In one embodiment, an initial dose of the pyrimidine analogue, e.g., capecitabine, is administered with the initial radition treatment, e.g., said initial dose of the pyrimidine analogue, e.g., capecitiabine, is administered within 24 hours, e.g., within about 24 hours, within about 22 hours, within about 20 hours, within about 18 hours, within about 16 hours, within about 14 hours, within about 12 hours, within about 10 hours, within about 8 hours, within about 6 hours, within about 4 hours, within about 2 hours, within about 1 hour, of the initial radiation treatment.

In one embodiment, said initial administration and/or one or more subsequent administrations of CDP-camptothecin conjugate, particle or composition or camptothecin derivative conjugate, particle or composition described herein, e.g., CRLX101, is administered at a dosage of 9 mg/m², 10 mg/m², 12 mg/m², or 15 mg/m² (wherein said dosage is expressed in mg of drug, as opposed to mg of conjugate).

In an embodiment, the dosage of at least 2, 3, 4, 5, 6, 7, 8, 9, 10, 12, 15 or 20 administrations of the CDP-camptothecin conjugate, particle or composition or camptothecin derivative conjugate, particle or composition described herein, e.g., CRLX101, is the same.

In an embodiment, the time between at least 2, 3, 4, 5, 6, 7, 8, 9, 10, 12, 15, or 20 administrations of the CDP-camptothecin conjugate, particle or composition or camptothecin derivative conjugate, particle or composition described herein, e.g., CRLX101, is the same.

In an embodiment, each subsequent administration of the CDP-camptothecin conjugate, particle or composition or camptothecin derivative conjugate, particle or composition described herein, e.g., CRLX101, is administered 9-15 days, e.g., 9 days, 12 days or 14 days, after the previous administration.

In an embodiment, at least 2, 3, 4, 5, 6, 7, 8, 9, 10, 12, 15, 20, 50 or 100 administrations of the CDP-camptothecin conjugate, particle or composition or camptothecin derivative conjugate, particle or composition described herein, e.g., CRLX101, are administered to said subject.

In one embodiment, the CDP-camptothecin or camptothecin derivative, a CDP-camptothecin or camptothecin derivative conjugate, particle or composition described herein, e.g., CRLX101, is administered by intravenous administration over a period equal to or less than about 30 minutes, 45 minutes, 60 minutes, 90 minutes, 120 minutes, 150 minutes, or 180 minutes. In one embodiment, the CDP-camptothecin or camptothecin derivative conjugate, particle or composition, e.g., the CDP-camptothecin or camptothecin derivative conjugate, particle or composition described herein, e.g. CRLX101, is administered at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m², or 18 mg/m², by intravenous administration over a period equal to or less than about 30 minutes, 45 minutes, 60 minutes or 90 minutes, e.g., a period equal to or less than 30 minutes, 45 minutes or 60 minutes.

In some embodiments, the radiation treatment, e.g., pelvic administration, is administered at a dosage of about 160 cGy to about 200 cGy, e.g., about 170 cGy to about 190 cGy, e.g., about 180 cGy per treatment.

In an embodiment, the dosage of at least 2, 3, 4, 5, 6, 7, 8, 9, 10, 11, 12, 13, 14, 15, 16, 17, 18, 19, 20, 21, 22, 23,24, 25, 26, 27, 28, 29, 30, 31, 32, 33, 34 or 35 of the radiation treatments, e.g., pelvic administration, are the same.

In an embodiment, at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 radiation treatments, e.g., pelvic administration treatments, are administered to said subject.

In an embodiment, the total amount of radiation, e.g., pelvic radiation, given during the multiple radition treatments is 6,000 cGy to about 4,000 cGy, about 5,900 cGy to about 4,100 cGy, about 5,800 cGy to about 4,200 cGy, about 5,700 cGy to about 4,300 cGy, about 5,600 cGy to about 4,400 cGy, e.g., about 5,400 cGy to about 4,500 cGy.

In an embodiment, the doses of the pyrimidine analogue, e.g., capecitiabine, are administered for at least 20 to 40 days, e.g., 25 to 35 days, e.g., 25, 26, 27, 28, 29, 30, 31, 32, 33, 34, 35 days.

In some embodiments, the pyrimidine analog, e.g., capecitabine, is administered at a dosage of about 600 mg/m², about 625 mg/m², about 650 mg/m², about 675 mg/m², about 700 mg/m², about 725 mg/m², about 750 mg/m², about 775 mg/m², about 800 mg/m², about 825 mg/m², about 850 mg/m², about 875 mg/m², about 900 mg/m², about 925 mg/m², about 950 mg/m², about 975 mg/m², by oral administration twice daily.

In one embodiment, the CDP-camptothecin or camptothecin derivative conjugate, particle or composition, e.g., a CDP-camptothecin or camptothecin derivative conjugate, particle or composition described herein, e.g., CRLX101, is administered prior to surgery, after surgery or before and after surgery to remove the cancer, e.g., to remove the primary tumor and/or a metastases.

In one embodiment, the CDP-camptothecin or camptothecin derivative conjugate, particle or composition described herein, e.g., CRLX101, is administered prior to surgery to remove the cancer, e.g., to remove the primary tumor and/or a metastases.

In an embodiment, the method further comprises administering to said subject a treatment that reduces one or more side effects associated with administration of a CDP-camptothecin or camptothecin derivative conjugate, particle or composition, e.g., a treatment described herein.

In some embodiments, the method further comprises administering an agent which ameliorates bladder toxicity associated with therapy, e.g., an agent which increases urinary excretion and/or neutralizes one or more urinary metabolite. In one embodiment, the agent which ameliorates bladder toxicity associated with therapy, e.g., the agent which increases urinary excretion and/or neutralizes one or more urinary metabolite, is administered prior to, concurrently with and/or after administration with the CDP-camptothecin or camptothecin derivative conjugate, particle or composition. In one embodiment, the agent which ameliorates bladder toxicity associated with therapy, e.g., the agent which increases urinary excretion and/or neutralizes one or more urinary metabolite, is administered prior to and after administration with the CDP-camptothecin or camptothecin derivative conjugate, particle or composition. In one embodiment, the agent which ameliorates bladder toxicity associated with therapy is saline, e.g., intravenous saline, D5 half normal saline or D5 water. In one embodiment, the agent which increases urinary excretion and/or neutralizes one or more urinary metabolite is 2-mercaptoethane sulfonate sodium (MESNA). In one embodiment, the agent which ameliorates bladder toxicity associated with therapy is 2-mercaptoethane sulfonate sodium (MESNA) and the MESNA is administered intravenously at a dose of about 10%, 20%, 30% the dose of the camptothecin or camptothecin derivative and/or the MESNA is administered orally at a dose of about 20%, 30%, 40%, 50% the dose of the camptothecin or camptothecin derivative.

In one embodiment, the method further comprises administering an agent which reduces or inhibits one or more symptom of hypersensitivity to the subject. Symptoms of hypersensitivity include: injection site reaction, dyspnea, hypotension, angioedema, urticaria, bronchospasm and erythema. In one embodiment, the agent which reduces or inhibits one or more symptoms of hypersensitivity can be one or more of a corticosteroid (e.g., dexamethasone), an antihistamine (e.g., diphenhydramine), and an H2 antagonist (e.g., ranitidine or famotidine). In one embodiment, the agent is a corticosteroid (e.g., dexamethasone) and the corticosteroid is administered at 5, 10, 15, 20, 25 or 30 mg. In one embodiment, the corticosteroid is administered about 12, 11, 10, 9, 8, 7, 6, 5, 4, and/or 3 hours before administration of the CDP-camptothecin or camptothecin derivative conjugate, particle or composition, or the corticosteroid is administered intravenously about 40, 30, 20 minutes before the CDP-camptothecin or camptothecin derivative conjugate, particle or composition. In one embodiment, the agent is an antihistamine (e.g., diphenhydramine) and the antihistamine is administered at 15, 20, 25, 30, 35, 40, 45, 50, 55, 60, 65 or 70 mg. In one embodiment, the antihistamine is administered intravenously about 40, 30, 20, 10 minutes before the CDP-camptothecin or camptothecin derivative conjugate, particle or composition. In one embodiment, the agent is an H2 antagonist (e.g., ranitidine or famotidine) and the H2 antagonist is administered at 10, 15, 20, 25, 30, 35, 40, 45, 50, 55, 60, 65 or 70 mg. In one embodiment the H2 antagonist is administered intravenously about 70, 60, 50, 40, 30, 20, 10 minutes before the CDP-camptothecin or camptothecin derivative conjugate, particle or composition.

In one embodiment, the method further comprises administering an agent which reduces or inhibits nausea and/or vomiting, e.g., an antiemetic, e.g., an antiemetic described herein.

In another embodiment, the method further comprises administering to said subject a treatment that reduces one or more side effects associated with administration of radiation treatment. For example, in one embodiment, the method further comprises administering an agent that ameliorates a side effect associated with radiation treatment. In one embodiment, the agent that ameliorates a side effect associated with radiation treatment, is administered prior to, concurrently with and/or after administration of the radiation treatment. In one embodiment, the agent that ameliorates a side effect associated with radiation treatment, is administered prior to and after administration of the radiation treatment.

In one embodiment, the agent that ameliorates a side effect associated with radiation treatment is a radiation protector. In another embodiment, the agent that ameliorates a side effect associated with radiation treatment is a corticosteroid ((e.g., dexamethasone). In another embodiment, the agent that ameliorates a side effect associated with radiation treatment is an agent (e.g., a topical agent) that treats radiation damage to the skin such as, e.g., sucralfate. In another embodiment, the agent that ameliorates a side effect associated with radiation treatment is an agent that stimulates growth of epithelial cells such as, e.g., keratinocyte growth factor (KGF, palifermin). In another embodiment, the agent that ameliorates a side effect associated with radiation treatment is an antioxidant that reduces radiation-induced tissue injury such as, e.g., an antioxidant (e.g., Cu/Zn superoxide dismutase (SOD)). In another embodiment, the agent that ameliorates a side effect associated with radiation treatment is an agent that stimulates platelet recovery in the subject such as, e.g., interleukin 11. In another embodiment, the agent that ameliorates a side effect associated with radiation therapy is an agent that treats inflammation and wounds such as, e.g., prostaglandin (e.g., misoprostol).

In another embodiment, the method further comprises administering to said subject a treatment that reduces one or more side effects associated with administration of a pyrimidine analogue, e.g., capecitabine. For example, the method further comprises administering an agent that ameliorates one or more side effects associated with the administration of a pyrimidine analogue, e.g., capecitabine. In one embodiment, the agent that ameliorates the side effects associate with administration of a pyrimidine analogue, e.g., capecitabine, is a cardiovasular agent, e.g., an agent that treats myocardial infarction or angina such as, e.g., a calcium channel blocker, e.g., diltiazem. In one embodiment, the agent that ameliorates the side effect associate with administration of a pyrimidine analogue, e.g., capecitabine, is an agent that treats hand-foot syndrome.

In one embodiment, the agent that ameliorates the side effects associate with administration of a pyrimidine analogue, e.g., capecitabine, an antiemetic, e.g., an antiemetic described herein.

In one embodiment, the CDP-camptothecin or camptothecin derivative conjugate, particle or composition, e.g., a CDP-camptothecin or camptothecin derivative conjugate, particle or composition described herein, e.g., CRLX101, is administered in combination with one or more additional chemotherapeutic agent, e.g., as described herein.

In one embodiment, the subject has not been administered a CDP-camptothecin or camptothecin derivative conjugate, particle or composition, a CDP-camptothecin or camptothecin derivative conjugate, particle or composition described herein, e.g., CRLX101, prior to the initial administration.

In an embodiment, the CDP-camptothecin or camptothecin derivative conjugate, particle or composition, a CDP-camptothecin or camptothecin derivative conjugate, particle or composition described herein, e.g., CRLX101, is administered as a first line treatment for the rectal cancer.

In an embodiment, the CDP-camptothecin or camptothecin derivative conjugate, particle or composition, a CDP-camptothecin or camptothecin derivative conjugate, particle or composition described herein, e.g., CRLX101, is administered as a second, third or fourth line treatment for the rectal cancer.

In an embodiment, the rectal cancer is sensitive to one or more chemotherapeutic agents, e.g., a platinum based agent, a taxane, an alkylating agent, an anthracycline (e.g., doxorubicin (e.g., liposomal doxorubicin)), an antimetabolite and/or a vinca alkaloid. In an embodiment, the cancer is a refractory, relapsed or resistant to one or more chemotherapeutic agents, e.g., a platinum based agent, a taxane, an alkylating agent, an antimetabolite and/or a vinca alkaloid.

In one embodiment, the CDP-camptothecin or camptothecin derivative conjugate, particle or composition, e.g., a CDP-camptothecin or camptothecin derivative conjugate, particle or composition described herein, e.g., CRLX101, is administered prior to surgery, after surgery or before and after surgery to remove the rectal cancer, e.g., to remove the primary tumor and/or a metastases.

In one embodiment, the method further comprises obtaining a sample, e.g., a biopsy sample, from the subject after an intital course of treatment, and determining if the subject has a pCR.

In one embodiment, the subject has a pathological complete response (pCR), e.g., after one course of treatment. In one embodiment, the subject has a pCR after one course of treatment and the subject is administered one or more additional courses of treatment.

In one embodiment, the subject does not have a pathological complete response (pCR), e.g., after one course of treatment. In one embodiment, the subject does not have a pCR after one course of treatment and the subject is administered one or more additional courses of treatment. In one embodiment, if the subject does not have a pCR after two courses of treatment, the subject is not given any further courses of treatment. In another embodiment, the subject does not have a pCR, e.g., after one course of treatment, and the subject is administered a chemotherapeutic agent other than the CDP-camptothecin or camptothecin derivative conjugate, particle or composition. In one embodiment, the subject does not have a pCR, e.g., after two courses of treatment, and the subject is administered a chemotherapeutic agent other than the CDP-camptothecin or camptothecin derivative conjugate, particle or composition.

The details of one or more embodiments of the invention are set forth in the description below. Other features, objects, and advantages of the invention will be apparent from the description and the drawings, and from the claims.

### Brief Description of the Drawings

FIG. 1 depicts the structure and description of an exemplary CDP-camptothecin conjugate referred to as "CRLX101" throughout this application. CRLX101 is used interchangeably with the term IT-101.
FIG. 2 depicts tumor volume and survival in a mouse model of head and neck cancer receiving CRLX101, radiation or a combination of CRLX101 and radiation. Radiation was administered starting one day after the initial dose of CRLX101.

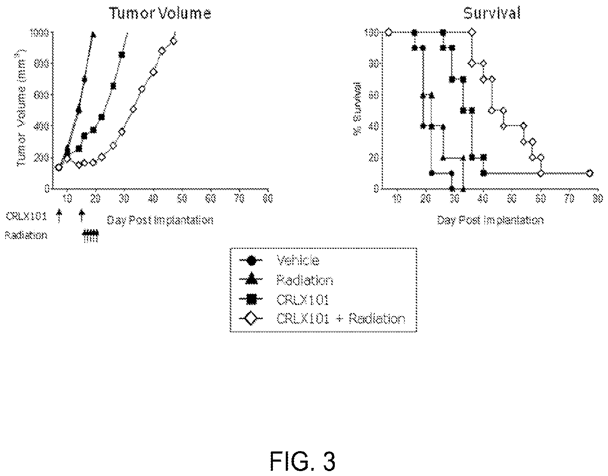

FIG. 3 depicts tumor volume and survival in a mouse model of head and neck cancer receiving CRLX101, radiation or a combination of CRLX101 and radiation. Radiation was administered starting one day after the second dose of CRLX101.

### Detailed Description of the Invention

The present invention relates to compositions of therapeutic cyclodextrin-containing polymers ("CDP") designed for drug delivery of a camptothecin or a camptothecin derivative. In certain embodiments, these cyclodextrin-containing polymers improve drug stability and/or solubility, and/or reduce toxicity, and/or improve efficacy of the camptothecin or camptothecin derivative when used *in vivo.*

Furthermore, by selecting from a variety of linker groups that link or couple CDP to a camptothecin or a camptothecin derivative, and/or targeting ligands, the rate of drug release from the polymers can be attenuated for controlled delivery.

More generally, the present invention provides water-soluble, biocompatible polymer conjugates comprising a water-soluble, biocompatible polymer covalently attached to a camptothecin or a camptothecin derivative through attachments that are cleaved under biological conditions to release the camptothecin or camptothecin derivative.

Polymeric conjugates featured in the methods described herein may be useful to improve solubility and/or stability of a bioactive/therapeutic agent, such as camptothecin, reduce drug-drug interactions, reduce interactions with blood elements including plasma proteins, reduce or eliminate immunogenicity, protect the agent from metabolism, modulate drug-release kinetics, improve circulation time, improve drug half-life (e.g., in the serum, or in selected tissues, such as tumors), attenuate toxicity, improve efficacy, normalize drug metabolism across subjects of different species, ethnicities, and/or races, and/or provide for targeted delivery into specific cells or tissues.

The term "camptothecin derivative", as used herein, includes camptothecin analogues and metabolites of camptothecin. For example, camptothecin derivatives can have the following structure: 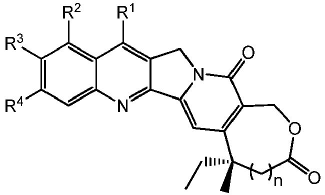 wherein
R¹ is H, OH, optionally substituted alkyl (e.g., optionally substituted with NR^{a}₂ or ORₐ, or SiR^{a}₃), or SiR^{a}₃; or R¹ and R² may be taken together to form an optionally substituted 5- to 8-membered ring (e.g., optionally substituted with NR^{a}₂ or OR^{a});
R² is H, OH, NH₂, halo, nitro, optionally substituted alkyl (e.g., optionally substituted with NR^{a}₂ or OR^{a}, NR^{a}₂, OC(=O)NR^{a}₂, or OC(=O)OR^{a});
R³ is H, OH, NH₂, halo, nitro, NR^{a}₂, OC(=O)NR^{a}₂, or OC(=O)OR^{a}
R⁴ is H, OH, NH₂, halo, CN, or NR^{a}₂; or R³ and R⁴ taken together with the atoms to which they are attached form a 5- or 6-membered ring (e.g. forming a ring including - OCH₂O- or -OCH₂CH₂O-);
each R^{a} is independently H or alkyl; or two R^{a}s, taken together with the atom to which they are attached, form a 4- to 8-membered ring (e.g., optionally containing an O or NR^{b})
R_{b} is H or optionally substituted alkyl (e.g., optionally substituted with OR^{c} or NR^{c}₂);
R^{c} is H or alkyl; or, two R^{c}s, taken together with the atom to which they are attached, form a 4- to 8-membered ring; and
n = 0 or 1.

In some embodiments, the camptothecin or camptothecin derivative is the compound as provided below.

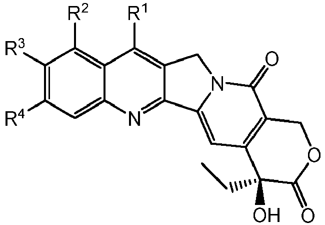

In one embodiment, R¹, R², R³ and R⁴ of the camptothecin derivative are each H, and n is 0.

In one embodiment, R¹, R², R³ and R⁴ of the camptothecin derivative are each H, and n is 1.

In one embodiment, R¹ of the camptothecin derivative is H, R² is -CH₂N(CH₃)₂, R³ is -OH, R⁴ is H; and n is 0.

In one embodiment, R¹ of the camptothecin derivative is -CH₂CH₃, R² is H, R³ is: 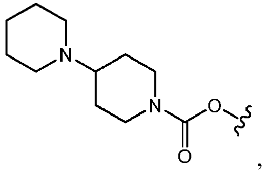 R⁴ is H, and n is 0.

In one embodiment, R¹ of the camptothecin derivative is -CH₂CH₃, R² is H, R³ is -OH, R⁴ is H, and n is 0.

In one embodiment, R¹ of the camptothecin derivative is *tert*-butyldimethylsilyl, R² is H, R³ is -OH and R⁴ is H, and n is 0.

In one embodiment, R¹ of the camptothecin derivative is *tert*-butyldimethylsilyl, R² is hydrogen, R³ is -OH and R⁴ is hydrogen, and n is 1.

In one embodiment, R¹ of the camptothecin derivative is *tert*-butyldimethylsilyl, R², R³ and R⁴ are each H, and n is 0.

In one embodiment, R¹ of the camptothecin derivative is *tert*-butyldimethylsilyl, R², R³ and R⁴ are each H, and n is 1.

In one embodiment, R¹ of the camptothecin derivative is -CH₂CH₂Si(CH₃)₃ and R², R³ and R⁴ are each H.

In one embodiment, R¹ and R² of the camptothecin derivative are taken together with the carbons to which they are attached to form an optionally substituted ring. In one embodiment, R¹ and R² of the camptothecin derivative are taken together with the carbons to which they are attached to form a substituted 6-membered ring. In one embodiment, the camptothecin derivative has the following formula: 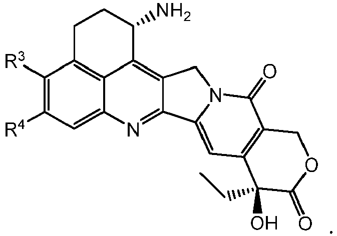 In one embodiment, R³ is methyl and R⁴ is fluoro.

In one embodiment, R³ and R⁴ are taken together with the carbons to which they are attached to form an optionally substituted ring. In one embodiment, R³ and R⁴ are taken together with the carbons to which they are attached to form a 6-membered heterocyclic ring. In one embodiment, the camptothecin derivative has the following formula: 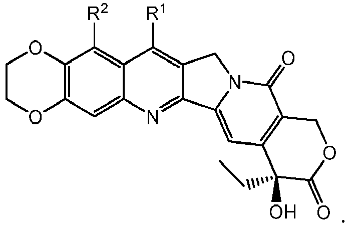 In one embodiment, R¹ is: 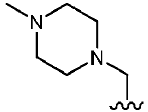 and R² is hydrogen.

In one embodiment, the camptothecin derivative has the following formula: 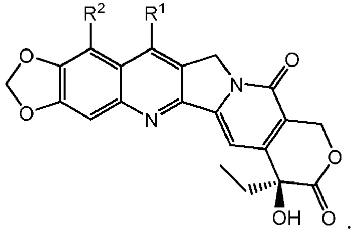 In one embodiment, R¹ is: 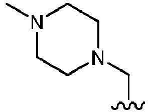 and R² is hydrogen.

In one embodiment, R¹ is: 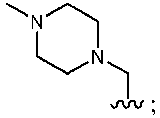 R² is H, R³ is methyl, R⁴ is chloro; and n is 1.

In one embodiment, R¹ is -CH=NOC(CH₃)₃, R², R³ and R⁴ are each H, and n is 0.

In one embodiment, R¹ is -CH₂CH₂NHCH(CH₃)₂, R², R³ and R⁴ are each H; and n is 0.

In one embodiment, R¹ and R² are H, R³ and R⁴ are fluoro, and n is 1.

In one embodiment, each of R¹, R³, and R⁴ is H, R² is NH₂, and n is 0.

In one embodiment, each of R¹, R³, and R⁴ is H, R² is NO₂, and n is 0.

An "effective amount" or "an amount effective" refers to an amount of the CDP-camptothecin or camptothecin derivative conjugate, particle or composition which is effective, upon single or multiple dose administrations to a subject, in treating a cell, or curing, alleviating, relieving or improving a symptom of a disorder. An effective amount of the conjugate, particle or composition may vary according to factors such as the disease state, age, sex, and weight of the individual, and the ability of the compound to elicit a desired response in the individual. An effective amount is also one in which any toxic or detrimental effects of the conjugate, particle or composition is outweighed by the therapeutically beneficial effects.

As used herein, the term "subject" is intended to include human and non-human animals. Exemplary human subjects include a human patient having a disorder, e.g., a disorder described herein, or a normal subject. The term "non-human animals" includes all vertebrates, e.g., non-mammals (such as chickens, amphibians, reptiles) and mammals, such as non-human primates, domesticated and/or agriculturally useful animals, e.g., sheep, dog, cat, cow, pig, etc.

As used herein, the term "treat" or "treating" a subject having a disorder refers to subjecting the subject to a regimen, e.g., the administration of a CDP-camptothecin or camptothecin derivative conjugate, particle or composition, such that at least one symptom of the disorder is cured, healed, alleviated, relieved, altered, remedied, ameliorated, or improved. Treating includes administering an amount effective to alleviate, relieve, alter, remedy, ameliorate, improve or affect the disorder or the symptoms of the disorder. The treatment may inhibit deterioration or worsening of a symptom of a disorder.

An amount of a CDP-camptothecin or camptothecin derivative conjugate, particle or composition, e.g., a CDP-camptothecin or camptothecin derivative conjugate, particle or composition described herein, e.g., CRLX101 effective to prevent a disorder, or "a prophylactically effective amount" of the conjugate, particle or composition as used in the context of the administration of an agent to a subject, refers to subjecting the subject to a regimen, e.g., the administration of a CDP-camptothecin or camptothecin derivative conjugate, particle or composition, e.g., a CDP-camptothecin or camptothecin derivative conjugate, particle or composition described herein, e.g., CRLX101, particle or composition such that the onset of at least one symptom of the disorder is delayed as compared to what would be seen in the absence of the regimen.

### CDP-Camptothecin and CDP-Camptothecin Derivative Conjugates, Particles and Compositions

Described herein are cyclodextrin containing polymer ("CDP")-camptothecin or camptothecin derivative conjugates, wherein one or more camptothecin or camptothecin derivative are covalently attached to the CDP (e.g., either directly or through a linker). The CDP-camptothecin or CDP-camptothecin derivative conjugates include linear or branched cyclodextrin-containing polymers and polymers grafted with cyclodextrin. Exemplary cyclodextrin-containing polymers that may be modified as described herein are taught in U.S. Patent Nos. 7,270,808, 6,509,323, 7,091,192, 6,884,789, U.S. Publication Nos. 20040087024, 20040109888 and 20070025952.

In certain such embodiments, the CDP comprises cyclic moieties alternating with linker moieties that connect the cyclic structures, e.g., into linear or branched polymers, preferably linear polymers. The cyclic moieties may be any suitable cyclic structures, such as cyclodextrins, crown ethers (e.g., 18-crown-6, 15-crown-5, 12-crown-4, etc.), cyclic oligopeptides (e.g., comprising from 5 to 10 amino acid residues), cryptands or cryptates (e.g., cryptand [2.2.2], cryptand-2,1,1, and complexes thereof), calixarenes, or cavitands, or any combination thereof. Preferably, the cyclic structure is (or is modified to be) water-soluble. In certain embodiments, e.g., for the preparation of a linear polymer, the cyclic structure is selected such that under polymerization conditions, exactly two moieties of each cyclic structure are reactive with the linker moieties, such that the resulting polymer comprises (or consists essentially of) an alternating series of cyclic moieties and linker moieties, such as at least four of each type of moiety. Suitable difunctionalized cyclic moieties include many that are commercially available and/or amenable to preparation using published protocols. In certain embodiments, conjugates are soluble in water to a concentration of at least 0.1 g/mL, preferably at least 0.25 g/mL.

Thus, in certain embodiments, the invention relates to novel compositions of therapeutic cyclodextrin-containing polymeric compounds designed for drug delivery of a camptothecin or camptothecin derivative. In certain embodiments, these CDPs improve drug stability and/or solubility, and/or reduce toxicity, and/or improve efficacy of the camptothecin or camptothecin derivative when used *in vivo.* Furthermore, by selecting from a variety of linker groups, and/or targeting ligands, the rate of camptothecin or camptothecin derivative release from the CDP can be attenuated for controlled delivery.

In certain embodiments, the CDP comprises a linear cyclodextrin-containing polymer, e.g., the polymer backbone includes cyclodextrin moieties. For example, the polymer may be a water-soluble, linear cyclodextrin polymer produced by providing at least one cyclodextrin derivative modified to bear one reactive site at each of exactly two positions, and reacting the cyclodextrin derivative with a linker having exactly two reactive moieties capable of forming a covalent bond with the reactive sites under polymerization conditions that promote reaction of the reactive sites with the reactive moieties to form covalent bonds between the linker and the cyclodextrin derivative, whereby a linear polymer comprising alternating units of cyclodextrin derivatives and linkers is produced. Alternatively the polymer may be a water-soluble, linear cyclodextrin polymer having a linear polymer backbone, which polymer comprises a plurality of substituted or unsubstituted cyclodextrin moieties and linker moieties in the linear polymer backbone, wherein each of the cyclodextrin moieties, other than a cyclodextrin moiety at the terminus of a polymer chain, is attached to two of said linker moieties, each linker moiety covalently linking two cyclodextrin moieties. In yet another embodiment, the polymer is a water-soluble, linear cyclodextrin polymer comprising a plurality of cyclodextrin moieties covalently linked together by a plurality of linker moieties, wherein each cyclodextrin moiety, other than a cyclodextrin moiety at the terminus of a polymer chain, is attached to two linker moieties to form a linear cyclodextrin polymer.

In some embodiments, the CDP-camptothecin or CDP-camptothecin derivative conjugates comprises a water soluble linear polymer conjugate comprising: cyclodextrin moieties; comonomers which do not contain cyclodextrin moieties (comonomers) (e.g., polyethylene glycol containing moieties); and a plurality of camptothecin or camptothecin derivative; wherein the CDP-camptothecin or CDP-camptothecin derivative conjugate comprises at least four, five six, seven, eight, etc., cyclodextrin moieties and at least four, five six, seven, eight, etc., comonomers. The camptothecin or camptothecin derivative can be attached to the CDP via a functional group such as a hydroxyl group, or where appropriate, an amino group.

In some embodiments, the least four cyclodextrin moieties and at least four comonomers alternate in the CDP-camptothecin or CDP-camptothecin derivative conjugate. In some embodiments, the camptothecin or camptothecin derivatives are cleaved from said CDP-camptothecin or CDP-camptothecin derivative conjugate under biological conditions to release the camptothecin or camptothecin derivatives. In some embodiments, the cyclodextrin moieties comprise linkers to which camptothecin or camptothecin derivatives are linked. In some embodiments, the camptothecin or camptothecin derivatives are attached via linkers.

In some embodiments, the comonomer comprises residues of at least two functional groups through which reaction and linkage of the cyclodextrin monomers was achieved. In some embodiments, the functional groups, which may be the same or different, terminal or internal, of each comonomer comprise an amino, acid, imidazole, hydroxyl, thio, acyl halide, -HC=CH-, -C≡C- group, or derivative thereof. In some embodiments, the two functional groups are the same and are located at termini of the comonomer precursor. In some embodiments, a comonomer contains one or more pendant groups with at least one functional group through which reaction and thus linkage of a camptothecin or camptothecin derivative was achieved. In some embodiments, the functional groups, which may be the same or different, terminal or internal, of each comonomer pendant group comprise an amino, acid, imidazole, hydroxyl, thiol, acyl halide, ethylene, ethyne group, or derivative thereof. In some embodiments, the pendant group is a substituted or unsubstituted branched, cyclic or straight chain C1-C10 alkyl, or arylalkyl optionally containing one or more heteroatoms within the chain or ring. In some embodiments, the cyclodextrin moiety comprises an alpha, beta, or gamma cyclodextrin moiety. In some embodiments, the camptothecin or camptothecin derivative is at least 5%, 10%, 15%, 20%, 25%, 30%, or 35% by weight of CDP-camptothecin or camptothecin derivative conjugate, particle or composition, e.g., the CDP-camptothecin or camptothecin derivative conjugate, particle or composition described herein, e.g., CRLX101.

In some embodiments, the comonomer comprises polyethylene glycol of molecular weight of about 2 to about 5 kDa (e.g., from about 2 to about 4.5 kDa, from about 3 to about 4 kDa, or less than about 4 kDa, (e.g., about 3.4 kDa ± 10%, e.g., about 3060 Da to about 3740 Da)), the cyclodextrin moiety comprises beta-cyclodextrin, the theoretical maximum loading of the camptothecin or camptothecin derivative on the CDP conjugate is 13% by weight, and the camptothecin or camptothecin derivative is 6-10% by weight of CDP-camptothecin or camptothecin derivative conjugate.

In some embodiments, the camptothecin or camptothecin derivative is attached to the CDP via a second compound.

In some embodiments, administration of the CDP-camptothecin or CDP-camptothecin derivative conjugate to a subject results in release of the camptothecin or camptothecin derivative over a period of at least 6 hours. In some embodiments, administration of the CDP-camptothecin or CDP-camptothecin derivative conjugate to a subject results in release of the camptothecin or camptothecin derivative over a period of 2 hours, 3 hours, 5 hours, 6 hours, 8 hours, 10 hours, 15 hours, 20 hours, 1 day, 2 days, 3 days, 4 days, 7 days, 10 days, 14 days, 17 days, 20 days, 24 days, 27 days up to a month. In some embodiments, upon administration of the CDP-camptothecin or CDP-camptothecin derivative conjugate to a subject, the rate of camptothecin or camptothecin derivative release is dependent primarily upon the rate of hydrolysis as opposed to enzymatic cleavage.

In some embodiments, the CDP-camptothecin or CDP-camptothecin derivative conjugate has a molecular weight of 10,000-500,000. In some embodiments, the cyclodextrin moieties make up at least about 2%, 5%, 10%, 20%, 30%, 50% or 80% of the CDP-camptothecin or CDP-camptothecin derivative conjugate by weight.

In some embodiments, the CDP-camptothecin or CDP-camptothecin derivative conjugate is made by a method comprising providing cyclodextrin moiety precursors modified to bear one reactive site at each of exactly two positions, and reacting the cyclodextrin moiety precursors with comonomer precursors having exactly two reactive moieties capable of forming a covalent bond with the reactive sites under polymerization conditions that promote reaction of the reactive sites with the reactive moieties to form covalent bonds between the comonomers and the cyclodextrin moieties, whereby a CDP comprising alternating units of a cyclodextrin moiety and a comonomer is produced. In some embodiments, the cyclodextrin moiety precursors are in a composition, the composition being substantially free of cyclodextrin moieties having other than two positions modified to bear a reactive site (e.g., cyclodextrin moieties having 1, 3, 4, 5, 6, or 7 positions modified to bear a reactive site).

In some embodiments, a comonomer of the CDP-camptothecin or CDP-camptothecin derivative conjugate comprises a moiety selected from the group consisting of: an alkylene chain, polysuccinic anhydride, poly-L-glutamic acid, poly(ethyleneimine), an oligosaccharide, and an amino acid chain. In some embodiments, a CDP-camptothecin or CDP-camptothecin derivative conjugate comonomer comprises a polyethylene glycol chain. In some embodiments, a comonomer comprises a moiety selected from: polyglycolic acid and polylactic acid chain. In some embodiments, a comonomer comprises a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-, -C(=O)O, -NR₁-, -NR₁CO-,-C(O)NR₁-, -S(O)ₙ- (wherein n is 0, 1, or 2), -OC(O)-NR₁, -NR₁-C(O)-NR₁-, -NR₁-C(NR₁)-NR₁-, and -B(OR₁)-; and R₁, independently for each occurrence, represents H or a lower alkyl.

In some embodiments, the CDP-camptothecin or camptothecin derivative conjugate is a polymer having attached thereto a plurality of D moieties of the following formula: 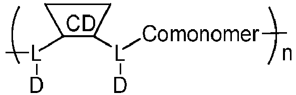 wherein each L is independently a linker, and each D is independently a camptothecin or camptothecin derivative, or absent; and each comonomer is independently a comonomer described herein, and n is at least 4, 5, 6, 7, 8, 9, 10, 11, 12, 13, 14, 15, 16, 17, 18, 19 or 20, provided that the polymer comprises at least one camptothecin or camptothecin derivative and in some embodiments, at least two camptothecin or camptothecin derivative moieties. In some embodiments, the molecular weight of the comonomer is from about 2000 to about 5000 Da (e.g., from about 3000 to about 4000 Da (e.g., about 3400 Da).

In some embodiments, the camptothecin or camptothecin derivative can be attached to the CDP via a functional group such as a hydroxyl group, or where appropriate, an amino group.

In some embodiments, the CDP-camptothecin or CDP-camptothecin derivative conjugate is a polymer having attached thereto a plurality of D moieties of the following formula: 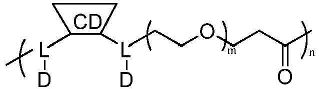 wherein each L is independently a linker, and each D is a camptothecin or camptothecin derivative, or absent, provided that the polymer comprises at least one camptothecin or camptothecin derivative and in some embodiments, at least two camptothecin or camptothecin derivative moieties; and wherein the group 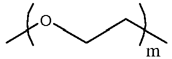 has a Mw of about 2 to about 5 kDa (e.g., from about 2 to about 4.5 kDa, from about 3 to about 4 kDa, or less than about 4 kDa, (e.g., about 3.4 kDa ± 10%, e.g., about 3060 Da to about 3740 Da)) and n is at least 4, 5, 6, 7, 8, 9, 10, 11, 12, 13, 14, 15, 16, 17, 18, 19 or 20.

In some embodiments, the camptothecin or camptothecin derivative can be attached to the CDP via a functional group such as a hydroxyl group, or where appropriate, an amino group.

In some embodiments, less than all of the L moieties are attached to D moieties, meaning in some embodiments, at least one D is absent. In some embodiments, the loading of the D moieties on CDP-camptothecin or CDP-camptothecin derivative conjugate is from about 1 to about 50% (e.g., from about 1 to about 25%, from about 5 to about 20% or from about 5 to about 15%). In some embodiments, each L independently comprises an amino acid or a derivative thereof. In some embodiments, each L independently comprises a plurality of amino acids or derivatives thereof. In some embodiments, each L is independently a dipeptide or derivative thereof. In one embodiment, L is one or more of: alanine, arginine, histidine, lysine, aspartic acid, glutamic acid, serine, threonine, asparganine, glutamine, cysteine, glycine, proline, isoleucine, leucine, methionine, phenylalanine, tryptophan, tyrosine and valine.

In some embodiments, the CDP-camptothecin or CDP-camptothecin derivative conjugate is a polymer having attached thereto a plurality of L-D moieties of the following formula: 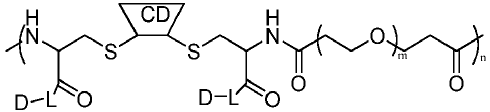 wherein each L is independently a linker or absent and each D is independently a camptothecin or camptothecin derivative, or absent and wherein the group 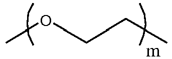 has a Mw of about 2 to about 5 kDa (e.g., from about 2 to about 4.5 kDa, from about 3 to about 4 kDa, or less than about 4 kDa, (e.g., about 3.4 kDa ± 10%, e.g., about 3060 Da to about 3740 Da)) and n is at least 4, 5, 6, 7, 8, 9, 10, 11, 12, 13, 14, 15, 16, 17, 18, 19 or 20, provided that the polymer comprises at least one camptothecin or camptothecin derivative and in some embodiments, at least two camptothecin or camptothecin derivative moieties.

In some embodiments, less than all of the C(=O) moieties are attached to L-D moieties, meaning in some embodiments, at least one L and/or D is absent. In some embodiments, the loading of the L, D and/or L-D moieties on the CDP-camptothecin or CDP-camptothecin derivative conjugate is from about 1 to about 50% (e.g., from about 1 to about 25%, from about 5 to about 20% or from about 5 to about 15%). In some embodiments, each L is independently an amino acid or derivative thereof. In some embodiments, each L is glycine or a derivative thereof.

In some embodiments, the CDP-camptothecin or camptothecin derivative conjugate is a polymer having the following formula:

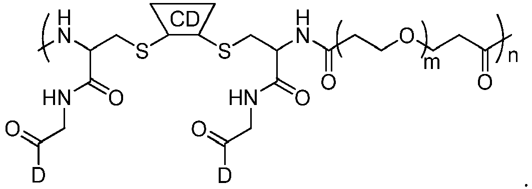

In some embodiments, less than all of the C(=O) moieties are attached to 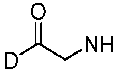 moieties, meaning in some embodiments, 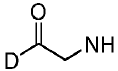 is absent, provided that the polymer comprises at least one camptothecin or camptothecin derivative and in some embodiments, at least two camptothecin or camptothecin derivative moieties. In some embodiments, the loading of the 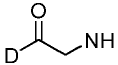 moieties on the CDP-camptothecin or CDP-camptothecin derivative conjugate is from about 1 to about 50% (e.g., from about 1 to about 25%, from about 5 to about 20% or from about 5 to about 15%).

### Cyclodextrins

In certain embodiments, the cyclodextrin moieties make up at least about 2%, 5% or 10% by weight, up to 20%, 30%, 50% or even 80% of the CDP by weight. In certain embodiments, the camptothecin or camptothecin derivative, or targeting ligands make up at least about 1%, 5%, 10% or 15%, 20%, 25%, 30% or even 35% of the CDP by weight. Number-average molecular weight (Mₙ) may also vary widely, but generally fall in the range of about 1,000 to about 500,000 daltons, preferably from about 5000 to about 200,000 daltons and, even more preferably, from about 10,000 to about 100,000. Most preferably, Mₙ varies between about 12,000 and 65,000 daltons. In certain other embodiments, Mₙ varies between about 3000 and 150,000 daltons. Within a given sample of a subject polymer, a wide range of molecular weights may be present. For example, molecules within the sample may have molecular weights that differ by a factor of 2, 5, 10, 20, 50, 100, or more, or that differ from the average molecular weight by a factor of 2, 5, 10, 20, 50, 100, or more. Exemplary cyclodextrin moieties include cyclic structures consisting essentially of from 7 to 9 saccharide moieties, such as cyclodextrin and oxidized cyclodextrin. A cyclodextrin moiety optionally comprises a linker moiety that forms a covalent linkage between the cyclic structure and the polymer backbone, preferably having from 1 to 20 atoms in the chain, such as alkyl chains, including dicarboxylic acid derivatives (such as glutaric acid derivatives, succinic acid derivatives, and the like), and heteroalkyl chains, such as oligoethylene glycol chains.

Cyclodextrins are cyclic polysaccharides containing naturally occurring D-(+)-glucopyranose units in an α-(1,4) linkage. The most common cyclodextrins are alpha ((α)-cyclodextrins, beta (β)-cyclodextrins and gamma (γ)-cyclodextrins which contain, respectively six, seven, or eight glucopyranose units. Structurally, the cyclic nature of a cyclodextrin forms a torus or donut-like shape having an inner apolar or hydrophobic cavity, the secondary hydroxyl groups situated on one side of the cyclodextrin torus and the primary hydroxyl groups situated on the other. Thus, using (β)-cyclodextrin as an example, a cyclodextrin is often represented schematically as follows.

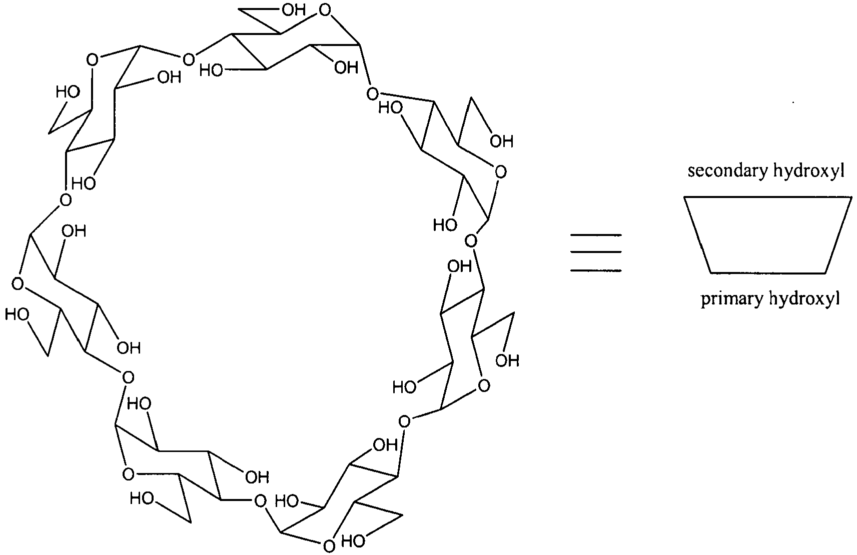

The side on which the secondary hydroxyl groups are located has a wider diameter than the side on which the primary hydroxyl groups are located. The present invention contemplates covalent linkages to cyclodextrin moieties on the primary and/or secondary hydroxyl groups. The hydrophobic nature of the cyclodextrin inner cavity allows for host-guest inclusion complexes of a variety of compounds, e.g., adamantane. (Comprehensive Supramolecular Chemistry, Volume 3, J.L. Atwood et al., eds., Pergamon Press (1996); T. Cserhati, Analytical Biochemistry, 225:328-332(1995); Husain et al., Applied Spectroscopy, 46:652-658 (1992); FR 2 665 169). Additional methods for modifying polymers are disclosed in Suh, J. and Noh, Y., Bioorg. Med. Chem. Lett. 1998, 8, 1327-1330.

In certain embodiments, the compounds comprise cyclodextrin moieties and wherein at least one or a plurality of the cyclodextrin moieties of the CDP-camptothecin or camptothecin derivative conjugate is oxidized. In certain embodiments, the cyclodextrin moieties of P alternate with linker moieties in the polymer chain.

### Comonomers

In addition to a cyclodextrin moiety, the CDP can also include a comonomer, for example, a comonomer described herein. In some embodiments, a comonomer of the CDP-camptothecin or camptothecin derivative conjugate comprises a moiety selected from the group consisting of: an alkylene chain, polysuccinic anhydride, poly-L-glutamic acid, poly(ethyleneimine), an oligosaccharide, and an amino acid chain. In some embodiments, a CDP- camptothecin or camptothecin derivative conjugate comonomer comprises a polyethylene glycol chain. In some embodiments, a comonomer comprises a moiety selected from: polyglycolic acid and polylactic acid chain. In some embodiments, a comonomer comprises a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-, -C(=O)O, -NR₁-, -NR₁CO-,-C(O)NR₁-, -S(O)ₙ- (wherein n is 0, 1, or 2), -OC(O)-NR₁, -NR₁-C(O)-NR₁-, -NR₁1-C(NR₁)-NR₁-, and -B(OR₁)-; and R₁, independently for each occurrence, represents H or a lower alkyl.

In some embodiments, a comonomer can be and/or can comprise a linker such as a linker described herein.

### Exemplary CDP- camptothecin or camptothecin derivative conjugates, particles and compositions

The compositions described herein comprise a CDP- camptothecin or camptothecin derivative conjugate or a plurality of CDP- camptothecin or camptothecin derivative conjugates. The composition can also comprise a particle or a plurality of particles described herein.

In one embodiment, the CDP- camptothecin or camptothecin derivative conjugate containing the inclusion complex forms a particle, e.g., a nanoparticle. The nanoparticle ranges in size from 10 to 300 nm in diameter, e.g., 20 to 280, 30 to 250, 40 to 200, 20 to 150, 30 to 100, 20 to 80, 30 to 70, 40 to 60 or 40 to 50 nm diameter. In one embodiment, the particle is 50 to 60 nm, 20 to 60 nm, 30 to 60 nm, 35 to 55 nm, 35 to 50 nm or 35 to 45 nm in diameter.

In one embodiment, the surface charge of the molecule is neutral, or slightly negative. In some embodiments, the zeta potential of the particle surface is from about - 80 mV to about 50 mV, about -20 mV to about 20 mV, about -20 mV to about -10 mV, or about -10 mV to about 0.

In some embodiments, the CDP-camptothecin or CDP-camptothecin derivative conjugate is a polymer having the following formula C: 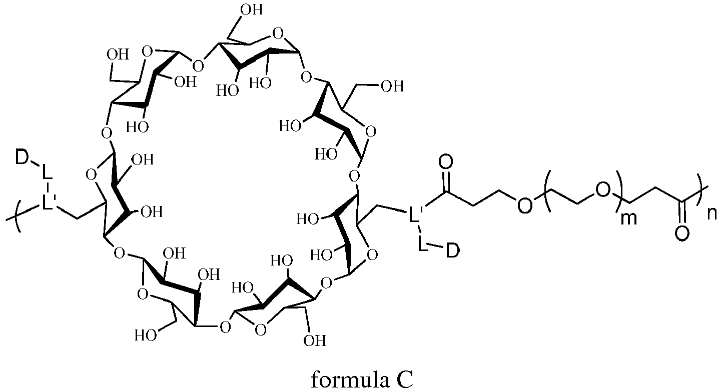 wherein L and L' independently for each occurrence, is a linker, a bond, or -OH and D, independently for each occurrence, is a camptothecin ("CPT"), a camptothecin derivative or absent, and
wherein the group 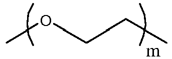 has a Mw of about 2 to about 5 kDa (e.g., from about 2 to about 4.5 kDa, from about 3 to about 4 kDa, or less than about 4 kDa, (e.g., about 3.4 kDa ± 10%, e.g., about 3060 Da to about 3740 Da)) and n is at least 4, provided that at least one D is CPT or a camptothecin derivative. In some embodiments, at least 2 D moieties are CPT and/or a camptothecin derivative.

In some embodiments, each L', for each occurrence, is a cysteine. In some embodiments, the cysteine is attached to the cyclodextrin via a sulfide bond. In some embodiments, the cysteine is attached to the PEG containing portion of the polymer via an amide bond.

In some embodiments, the L is a linker (e.g., an amino acid such as glycine). In some embodiments, L is absent. In some embodiments, D-L together form

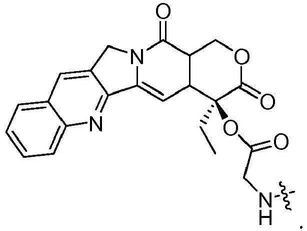

In some embodiments, a plurality of D moieties are absent and at the same position on the polymer, the corresponding L is -OH.

In some embodiments, less than all of the C(=O) moieties of the cysteine residue in the polymer backbone are attached to 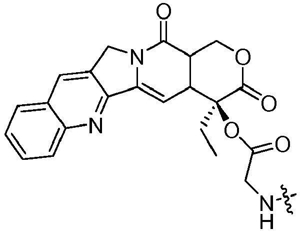 moieties, meaning in some embodiments, 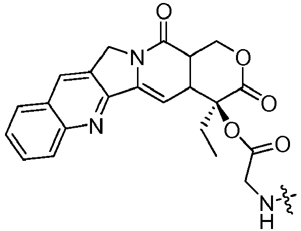 is absent in one or more positions of the polymer backbone, provided that the polymer comprises at least one 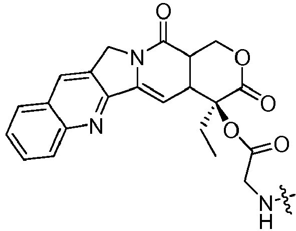 and in some embodiments, at least two 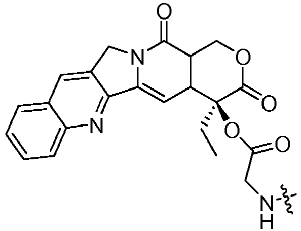 moieties. In some embodiments, the loading of the 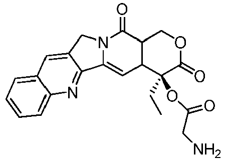 moieties on the CDP- camptothecin or camptothecin derivative conjugate is from about 1 to about 50% (e.g., from about 1 to about 25%, from about 5 to about 20% or from about 5 to about 15%, e.g., from about 6 to about 10%). In some embodiments, the loading of 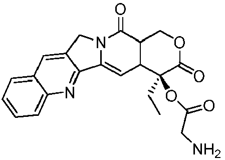 on the CDP is from about 6% to about 10% by weight of the total polymer.

In some embodiments, the CDP- camptothecin or camptothecin derivative conjugate of formula C is a polymer having the following formula: 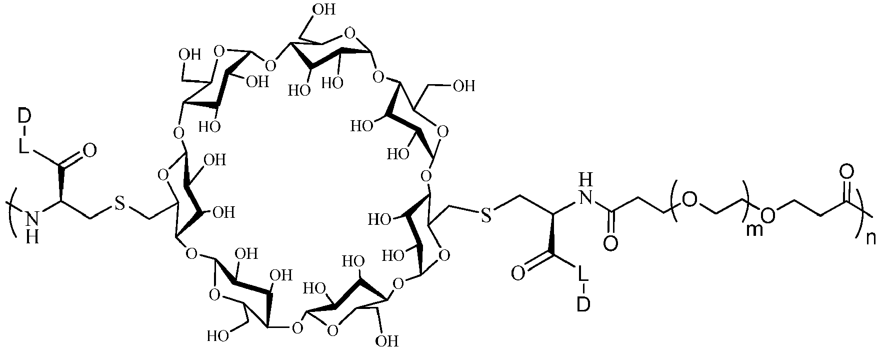 wherein L, independently for each occurrence, is a linker, a bond, or -OH and D, independently for each occurrence, is camptothecin, a camptothecin derivative or absent, and wherein the group 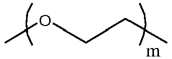 has a Mw of about 2 to about 5 kDa (e.g., from about 2 to about 4.5 kDa, from about 3 to about 4 kDa, or less than about 4 kDa, (e.g., about 3.4 kDa ± 10%, e.g., about 3060 Da to about 3740 Da)) and n is at least 4, provided that at least one D is CPT or a camptothecin derivative. In some embodiments, at least 2 D moieties are CPT and/or a camptothecin derivative.

In some embodiments, the CDP-camptothecin conjugate of formula C is a polymer of the following formula: 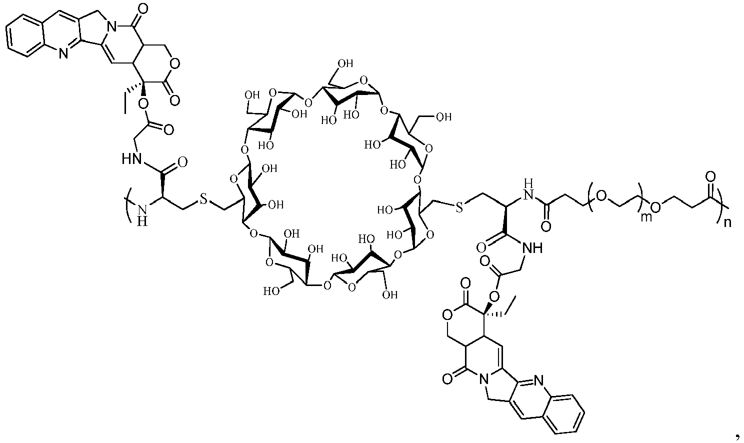 wherein m and n are as defined above, and wherein less than all of the C(=O) sites of the cysteine of the polymer backbone are occupied as indicated above with the CPT-Gly, but instead are free acids, meaning, the theoretical loading of the polymer is less than 100%.

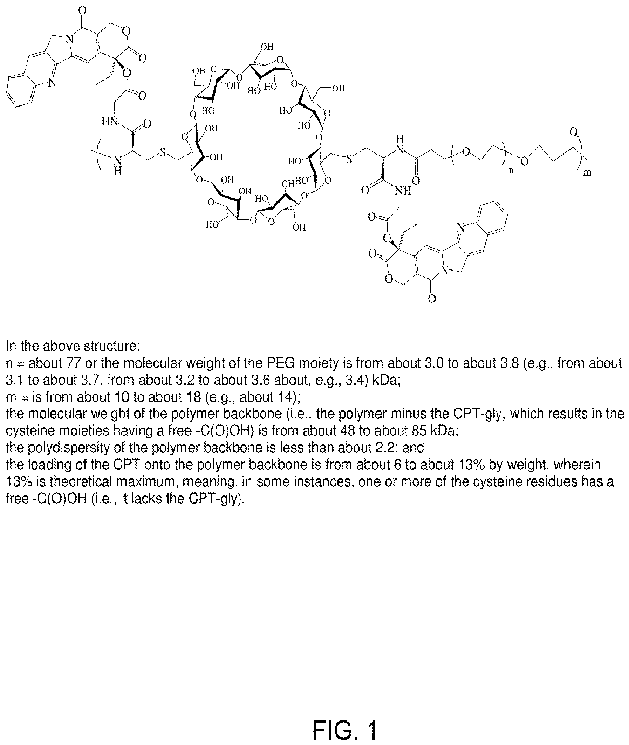

In some embodiments, the CDP-camptothecin conjugate is as provided in FIG. 1, and shown below, which is referred to herein as "CRLX101." 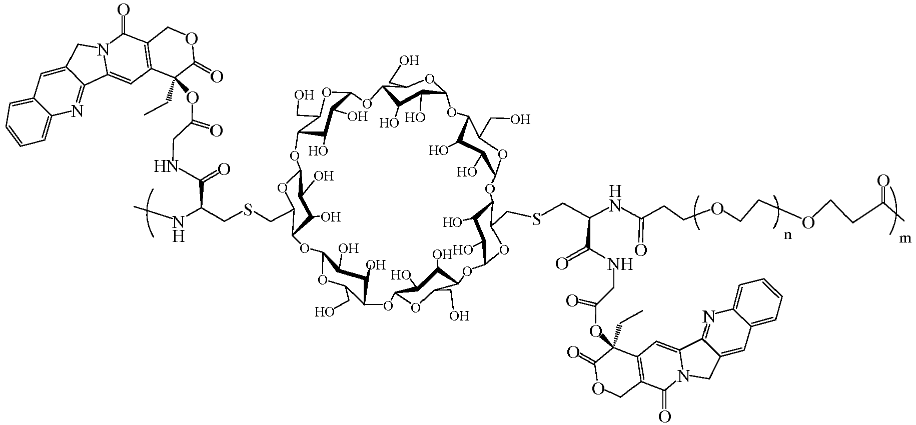 In the above structure:
n = about 77 or the molecular weight of the PEG moiety is from about 3.0 to about 3.8 (e.g., from about 3.1 to about 3.7, from about 3.2 to about 3.6, e.g., about 3.4) kDa;
m = is from about 10 to about 18 (e.g., about 14);
the molecular weight of the polymer backbone (i.e., the polymer minus the CPT-gly, which results in thecysteine moieties having a free -C(O)OH) is from about 48 to about 85 kDa;
the polydispersity of the polymer backbone is less than about 2.2; and
   the loading of the CPT onto the polymer backbone is from about 6 to about 13% by weight, wherein 13% is theoretical maximum, meaning, in some instances, one or more of the cysteine residues has a free -C(O)OH (i.e., it lacks the CPT-gly).

In some embodiments, the polydispersity of the PEG component in the above structure is less than about 1.1.

In some embodiments, a CDP-camptothecin conjugate described herein has a terminal amine and/or a terminal carboxylic acid.

### Linkers/tethers

The CDPs described herein can include one or more linkers. In some embodiments, a linker can link camptothecin or camptothecin derivative to a CDP. In some embodiments, for example, when referring to a linker that links camptothecin or camptothecin derivative to the CDP, the linker can be referred to as a tether.

In certain embodiments, a plurality of the linker moieties are attached to camptothecin or camptothecin derivative or prodrug thereof and are cleaved under biological conditions.

In some embodiments, a linker may be and/or comprise an alkylene chain, a polyethylene glycol (PEG) chain, polysuccinic anhydride, poly-L-glutamic acid, poly(ethyleneimine), an oligosaccharide, an amino acid (e.g., glycine or cysteine), an amino acid chain, or any other suitable linkage. In certain embodiments, the linker group itself can be stable under physiological conditions, such as an alkylene chain, or it can be cleavable under physiological conditions, such as by an enzyme (e.g., the linkage contains a peptide sequence that is a substrate for a peptidase), or by hydrolysis (e.g., the linkage contains a hydrolyzable group, such as an ester or thioester). The linker groups can be biologically inactive, such as a PEG, polyglycolic acid, or polylactic acid chain, or can be biologically active, such as an oligo- or polypeptide that, when cleaved from the moieties, binds a receptor, deactivates an enzyme, etc. Various oligomeric linker groups that are biologically compatible and/or bioerodible are known in the art, and the selection of the linkage may influence the ultimate properties of the material, such as whether it is durable when implanted, whether it gradually deforms or shrinks after implantation, or whether it gradually degrades and is absorbed by the body. The linker group may be attached to the moieties by any suitable bond or functional group, including carbon-carbon bonds, esters, ethers, amides, amines, carbonates, carbamates, sulfonamides, etc.

In certain embodiments, the linker group(s) of the present invention represent a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-, -C(=O)O, -NR₁-, -NR₁CO-, -C(O)NR₁-, -S(O)ₙ- (wherein n is 0, 1, or 2), -OC(O)-NR₁, -NR₁-C(O)-NR₁-, -NR₁-C(NR₁)-NR₁-, and -B(OR₁)-; and R₁, independently for each occurrence, represents H or a lower alkyl.

In certain embodiments, the linker group represents a derivatized or non-derivatized amino acid (e.g., glycine or cysteine). In certain embodiments, linker groups with one or more terminal carboxyl groups may be conjugated to the polymer. In certain embodiments, one or more of these terminal carboxyl groups may be capped by covalently attaching them to a therapeutic agent, a targeting moiety, or a cyclodextrin moiety via an (thio)ester or amide bond. In still other embodiments, linker groups with one or more terminal hydroxyl, thiol, or amino groups may be incorporated into the polymer. In preferred embodiments, one or more of these terminal hydroxyl groups may be capped by covalently attaching them to a therapeutic agent, a targeting moiety, or a cyclodextrin moiety via an (thio)ester, amide, carbonate, carbamate, thiocarbonate, or thiocarbamate bond. In certain embodiments, these (thio)ester, amide, (thio)carbonate or (thio)carbamates bonds may be biohydrolyzable, i.e., capable of being hydrolyzed under biological conditions.

In certain embodiments, a linker group represents a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-, -C(=O)O, -NR₁-, -NR₁CO-, -C(O)NR₁-, -S(O)ₙ- (wherein n is 0, 1, or 2), -OC(O)-NR₁, -NR₁-C(O)-NR₁-, -NR₁-C(NR₁)-NR₁-, and -B(OR₁)-; and R₁, independently for each occurrence, represents H or a lower alkyl.

In certain embodiments as disclosed herein, the camptothecin or camptothecin derivative is covalently bonded to the linker group via a biohydrolyzable bond (e.g., an ester, amide, carbonate, carbamate, or a phosphate).

In certain embodiments as disclosed herein, the CDP comprises cyclodextrin moieties that alternate with linker moieties in the polymer chain.

In certain embodiments, the linker moieties are attached to a camptothecin or camptothecin derivatives or prodrugs thereof that are cleaved under biological conditions.

In certain embodiments, any of the linker groups may independently be or include an alkyl chain, a polyethylene glycol (PEG) chain, polysuccinic anhydride, poly-L-glutamic acid, poly(ethyleneimine), an oligosaccharide, an amino acid chain, or any other suitable linkage. In certain embodiments, the linker group itself can be stable under physiological conditions, such as an alkyl chain, or it can be cleavable under physiological conditions, such as by an enzyme (e.g., the linkage contains a peptide sequence that is a substrate for a peptidase), or by hydrolysis (e.g., the linkage contains a hydrolyzable group, such as an ester or thioester). The linker groups can be biologically inactive, such as a PEG, polyglycolic acid, or polylactic acid chain, or can be biologically active, such as an oligo- or polypeptide that, when cleaved from the moieties, binds a receptor, deactivates an enzyme, etc. Various oligomeric linker groups that are biologically compatible and/or bioerodible are known in the art, and the selection of the linkage may influence the ultimate properties of the material, such as whether it is durable when implanted, whether it gradually deforms or shrinks after implantation, or whether it gradually degrades and is absorbed by the body. The linker group may be attached to the moieties by any suitable bond or functional group, including carbon-carbon bonds, esters, ethers, amides, amines, carbonates, carbamates, sulfonamides, etc.

In certain embodiments, any of the linker groups may independently be an alkyl group wherein one or more methylene groups is optionally replaced by a group Y (provided that none of the Y groups are adjacent to each other), wherein each Y, independently for each occurrence, is selected from aryl, heteroaryl, carbocyclyl, heterocyclyl, or -O-, C(=X) (wherein X is NR¹, O or S), -OC(O)-, -C(=O)O-, -NR¹-, -NR¹CO-, -C(O)NR¹-, -S(O)ₙ- (wherein n is 0, 1, or 2), -OC(O)-NR¹-, -NR¹-C(O)-NR¹-, -NR¹-C(NR¹)-NR¹-, and -B(OR¹)-; and R¹, independently for each occurrence, is H or lower alkyl.

In certain embodiments, the present invention contemplates a CDP, wherein a plurality of camptothecin or camptothecin derivatives are covalently attached to the polymer through attachments that are cleaved under biological conditions to release the therapeutic agents as discussed above, wherein administration of the polymer to a subject results in release of the therapeutic agent over a period of at least 2, 3, 5, 6, 8, 10, 15, 20, 24, 36, 48 or even 72 hours.

In some embodiments, the conjugation of the camptothecin or camptothecin derivative to the CDP improves the aqueous solubility of the camptothecin or camptothecin derivative and hence the bioavailability. Accordingly, in one embodiment of the invention, the camptothecin or camptothecin derivative has a log P >0.4, >0.6, >0.8, >1, >2, >3, >4, or even >5.

The CDP-camptothecin or CDP-camptothecin derivative conjugate of the disclosure preferably has a molecular weight in the range of 10,000 to 500,000; 30,000 to 200,000; or even 70,000 to 150,000 amu.

In certain embodiments, the present invention contemplates attenuating the rate of release of the camptothecin or camptothecin derivative by introducing various tether and/or linking groups between the therapeutic agent and the polymer. Thus, in certain embodiments, the CDP-camptothecin or CDP-camptothecin derivative conjugates of disclosure are compositions for controlled delivery of the camptothecin or camptothecin derivative.

### CDP-Camptothecin or CDP-Camptothecin Derivative Conjugate Characteristics

In some embodiments, the CDP-camptothecin or camptothecin derivative conjugates, particles or compositions, e.g., a CDP-camptothecins or camptothecin derivative conjugates, particles or compositions described herein, e.g., CRLX101, as described herein have polydispersities less than about 3, or even less than about 2.

One embodiment of the disclosure provides an improved delivery of certain a camptothecin or a camptothecin derivative by covalently attaching one or more camptothecin or camptothecin derivatives to a CDP. Such conjugation can improve the aqueous solubility and hence the bioavailability of the camptothecin or camptothecin derivative.

The CDP-camptothecin or camptothecin derivative conjugates, particles or compositions, e.g., a CDP-camptothecins or camptothecin derivative conjugates, particles or compositions described herein, e.g., CRLX101 described herein preferably have molecular weights in the range of 10,000 to 500,000; 30,000 to 200,000; or even 70,000 to 150,000 amu. In certain embodiments as disclosed herein, the compound has a number average (Mₙ) molecular weight between 1,000 to 500,000 amu, or between 5,000 to 200,000 amu, or between 10,000 to 100,000 amu. One method to determine molecular weight is by gel permeation chromatography ("GPC"), e.g., mixed bed columns, CH₂Cl₂ solvent, light scattering detector, and off-line dn/dc. Other methods are known in the art.

In certain embodiments as disclosed herein, the CDP-camptothecin or camptothecin derivative conjugate, particle or composition, e.g., a CDP-camptothecin or camptothecin derivative conjugate, particle or composition described herein, e.g., CRLX101, is biodegradable or bioerodable.

In certain embodiments as disclosed herein, the camptothecin, camptothecin derivative, or prodrug thereof makes up at least 3% (e.g., at least about 5%) by weight of the polymer. In certain embodiments, the camptothecin, camptothecin derivative or prodrug thereof makes up at least 20% by weight of the compound. In certain embodiments, the camptothecin, camptothecin derivative or prodrug thereof makes up at least 5%, 10%, 15%, or at least 20% by weight of the compound.

CDP-camptothecin or camptothecin derivative conjugate, particle or composition, e.g., a CDP-camptothecin or camptothecin derivative conjugate, particle or composition described herein, e.g., CRLX101, described herein may be useful to improve solubility and/or stability of the camptothecin or camptothecin derivative, reduce drug-drug interactions, reduce interactions with blood elements including plasma proteins, reduce or eliminate immunogenicity, protect the camptothecin or camptothecin derivative from metabolism, modulate drug-release kinetics, improve circulation time, improve camptothecin or camptothecin derivative half-life (e.g., in the serum, or in selected tissues, such as tumors), attenuate toxicity, improve efficacy, normalize a camptothecin or camptothecin derivative metabolism across subjects of different species, ethnicities, and/or races, and/or provide for targeted delivery into specific cells or tissues.

In other embodiments, the CDP-camptothecin or camptothecin derivative conjugate, particle or composition, e.g., a CDP-camptothecin or camptothecin derivative conjugate, particle or composition described herein, e.g., CRLX101, may be a flexible or flowable material. When the CDP used is itself flowable, the CDP composition of the invention, even when viscous, need not include a biocompatible solvent to be flowable, although trace or residual amounts of biocompatible solvents may still be present.

While it is possible that the biodegradable polymer or the biologically active agent may be dissolved in a small quantity of a solvent that is non-toxic to more efficiently produce an amorphous, monolithic distribution or a fine dispersion of the biologically active agent in the flexible or flowable composition, it is an advantage of the invention that, in a preferred embodiment, no solvent is needed to form a flowable composition. Moreover, the use of solvents is preferably avoided because, once a polymer composition containing solvent is placed totally or partially within the body, the solvent dissipates or diffuses away from the polymer and must be processed and eliminated by the body, placing an extra burden on the body's clearance ability at a time when the illness (and/or other treatments for the illness) may have already deleteriously affected it.

However, when a solvent is used to facilitate mixing or to maintain the flowability of the CDP- camptothecin or camptothecin derivative conjugate, particle or composition, it should be non-toxic, otherwise biocompatible, and should be used in relatively small amounts. Solvents that are toxic should not be used in any material to be placed even partially within a living body. Such a solvent also must not cause substantial tissue irritation or necrosis at the site of administration.

Examples of suitable biocompatible solvents, when used, include N-methyl-2-pyrrolidone, 2-pyrrolidone, ethanol, propylene glycol, acetone, methyl acetate, ethyl acetate, methyl ethyl ketone, dimethylformamide, dimethylsulfoxide, tetrahydrofuran, caprolactam, oleic acid, or 1-dodecylazacylcoheptanone. Preferred solvents include N-methylpyrrolidone, 2-pyrrolidone, dimethylsulfoxide, and acetone because of their solvating ability and their biocompatibility.

In certain embodiments, the CDP- camptothecin or camptothecin derivative conjugates, particles and compositions are soluble in one or more common organic solvents for ease of fabrication and processing. Common organic solvents include such solvents as chloroform, dichloromethane, dichloroethane, 2-butanone, butyl acetate, ethyl butyrate, acetone, ethyl acetate, dimethylacetamide, N-methylpyrrolidone, dimethylformamide, and dimethylsulfoxide.

In certain embodiments, the CDP- camptothecin or camptothecin derivative conjugates, particles and compositions described herein, upon contact with body fluids, undergo gradual degradation. The life of a biodegradable polymer in vivo depends upon, among other things, its molecular weight, crystallinity, biostability, and the degree of crosslinking. In general, the greater the molecular weight, the higher the degree of crystallinity, and the greater the biostability, the slower biodegradation will be.

If a subject composition is formulated with a camptothecin or camptothecin derivative or other material, release of the camptothecin or camptothecin derivative or other material for a sustained or extended period as compared to the release from an isotonic saline solution generally results. Such release profile may result in prolonged delivery (over, say 1 to about 2,000 hours, or alternatively about 2 to about 800 hours) of effective amounts (e.g., about 0.0001 mg/kg/hour to about 10 mg/kg/hour, e.g., 0.001 mg/kg/hour, 0.01 mg/kg/hour, 0.1 mg/kg/hour, 1.0 mg/kg/hour) of the camptothecin or camptothecin derivative or any other material associated with the polymer.

A variety of factors may affect the desired rate of hydrolysis of CDP-camptothecin or camptothecin derivative conjugates, particles and compositions, the desired softness and flexibility of the resulting solid matrix, rate and extent of bioactive material release. Some of such factors include the selection/identity of the various subunits, the enantiomeric or diastereomeric purity of the monomeric subunits, homogeneity of subunits found in the polymer, and the length of the polymer. For instance, the present invention contemplates heteropolymers with varying linkages, and/or the inclusion of other monomeric elements in the polymer, in order to control, for example, the rate of biodegradation of the matrix.

To illustrate further, a wide range of degradation rates may be obtained by adjusting the hydrophobicities of the backbones or side chains of the polymers while still maintaining sufficient biodegradability for the use intended for any such polymer. Such a result may be achieved by varying the various functional groups of the polymer. For example, the combination of a hydrophobic backbone and a hydrophilic linkage produces heterogeneous degradation because cleavage is encouraged whereas water penetration is resisted.

One protocol generally accepted in the field that may be used to determine the release rate of a therapeutic agent such as a camptothecin or camptothecin derivative or other material loaded in the CDP- camptothecin or camptothecin derivative conjugates, particles or compositions of the present invention involves degradation of any such matrix in a 0.1 M PBS solution (pH 7.4) at 37 °C, an assay known in the art. For purposes of the present invention, the term "PBS protocol" is used herein to refer to such protocol.

In certain instances, the release rates of different CDP- camptothecin or camptothecin derivative conjugates, particles and compositions of the present invention may be compared by subjecting them to such a protocol. In certain instances, it may be necessary to process polymeric systems in the same fashion to allow direct and relatively accurate comparisons of different systems to be made. For example, the present invention teaches several different methods of formulating the CDP- camptothecin or camptothecin derivative conjugates, particles and compositions. Such comparisons may indicate that any one CDP- camptothecin or camptothecin derivative conjugate, particle or composition releases incorporated material at a rate from about 2 or less to about 1000 or more times faster than another polymeric system.

Alternatively, a comparison may reveal a rate difference of about 3, 5, 7, 10, 25, 50, 100, 250, 500 or 750 times. Even higher rate differences are contemplated by the present invention and release rate protocols.

In certain embodiments, when formulated in a certain manner, the release rate for CDP- camptothecin or camptothecin derivative conjugates, particles and compositions of the present invention may present as mono- or bi-phasic.

Release of any material incorporated into the polymer matrix, which is often provided as a microsphere, may be characterized in certain instances by an initial increased release rate, which may release from about 5 to about 50% or more of any incorporated material, or alternatively about 10, 15, 20, 25, 30 or 40%, followed by a release rate of lesser magnitude.

The release rate of any incorporated material may also be characterized by the amount of such material released per day per mg of polymer matrix. For example, in certain embodiments, the release rate may vary from about 1 ng or less of any incorporated material per day per mg of polymeric system to about 500 or more ng/day/mg. Alternatively, the release rate may be about 0.05, 0.5, 5, 10, 25, 50, 75, 100, 125, 150, 175, 200, 250, 300, 350, 400, 450, or 500 ng/day/mg. In still other embodiments, the release rate of any incorporated material may be 10,000 ng/day/mg, or even higher. In certain instances, materials incorporated and characterized by such release rate protocols may include therapeutic agents, fillers, and other substances.

In another aspect, the rate of release of any material from any CDP- camptothecin or camptothecin derivative conjugate, particle or composition of the present invention may be presented as the half-life of such material in the matrix.

In addition to the embodiment involving protocols for in vitro determination of release rates, in vivo protocols, whereby in certain instances release rates for polymeric systems may be determined in vivo, are also contemplated by the present invention. Other assays useful for determining the release of any material from the polymers of the present system are known in the art.

### Physical Structures of the CDP- Camptothecin or Camptothecin Derivative Conjugates, Particles and Compositions

The CDP-camptothecin or camptothecin derivative conjugate, particle or composition, e.g., a CDP-camptothecin or camptothecin derivative conjugate, particle or composition described herein, e.g., CRLX101, may be formed in a variety of shapes. For example, in certain embodiments, CDP-camptothecin or camptothecin derivative conjugate, particle or composition, e.g., a CDP-camptothecin or camptothecin derivative conjugate, particle or composition described herein, e.g., CRLX101, may be presented in the form of microparticles or nanoparticles. Microspheres typically comprise a biodegradable polymer matrix incorporating a drug. Microspheres can be formed by a wide variety of techniques known to those of skill in the art. Examples of microsphere forming techniques include, but are not limited to, (a) phase separation by emulsification and subsequent organic solvent evaporation (including complex emulsion methods such as oil in water emulsions, water in oil emulsions and water-oil-water emulsions); (b) coacervation-phase separation; (c) melt dispersion; (d) interfacial deposition; (e) in situ polymerization; (f) spray drying and spray congealing; (g) air suspension coating; and (h) pan and spray coating. These methods, as well as properties and characteristics of microspheres are disclosed in, for example, U.S. Patent No. 4,438,253; U.S. Patent No. 4,652,441; U.S. Patent No. 5,100,669; U.S. Patent No. 5,330,768; U.S. Patent No. 4,526,938; U.S. Patent No. 5,889,110; U.S. Patent No. 6,034,175; and European Patent 0258780, the entire disclosures of which are incorporated by reference herein in their entireties.

To prepare microspheres, several methods can be employed depending upon the desired application of the delivery vehicles. Suitable methods include, but are not limited to, spray drying, freeze drying, air drying, vacuum drying, fluidized-bed drying, milling, co-precipitation and critical fluid extraction. In the case of spray drying, freeze drying, air drying, vacuum drying, fluidized-bed drying and critical fluid extraction; the components (stabilizing polyol, bioactive material, buffers, etc.) are first dissolved or suspended in aqueous conditions. In the case of milling, the components are mixed in the dried form and milled by any method known in the art. In the case of co-precipitation, the components are mixed in organic conditions and processed as described below. Spray drying can be used to load the stabilizing polyol with the bioactive material. The components are mixed under aqueous conditions and dried using precision nozzles to produce extremely uniform droplets in a drying chamber. Suitable spray drying machines include, but are not limited to, Buchi, NIRO, APV and Lab-plant spray driers used according to the manufacturer's instructions.

The shape of microparticles and nanoparticles may be determined by scanning electron microscopy. Spherically shaped nanoparticles are used in certain embodiments, for circulation through the bloodstream. If desired, the particles may be fabricated using known techniques into other shapes that are more useful for a specific application.

In addition to intracellular delivery of a camptothecin or camptothecin derivative, it also possible that particles of the CDP- camptothecin or camptothecin derivative conjugates, such as microparticles or nanoparticles, may undergo endocytosis, thereby obtaining access to the cell. The frequency of such an endocytosis process will likely depend on the size of any particle.

In one embodiment, the surface charge of the molecule is neutral, or slightly negative. In some embodiments, the zeta potential of the particle surface is from about - 80 mV to about 50 mV.

### CDPs, Methods of Making Same, and Methods of Conjugating CDPs to Camptothecin or Camptothecin Derivatives

Generally, the CDP-camptothecin or camptothecin derivative conjugate, particle or composition, e.g., a CDP-camptothecin or camptothecin derivative conjugate, particle or composition described herein, e.g., CRLX101, can be prepared in one of two ways: monomers bearing camptothecin or camptothecin derivative, targeting ligands, and/or cyclodextrin moieties can be polymerized, or polymer backbones can be derivatized with camptothecin or camptothecin derivatives, targeting ligands, and/or cyclodextrin moieties. Exemplary methods of making CDPs and CDP-camptothecin or camptothecin derivative conjugate, particle or composition, e.g., a CDP-camptothecin or camptothecin derivative conjugate, particle or composition described herein, e.g., CRLX101, for example, in U.S. Patent No.: 7,270,808, the contents of which is incorporated herein by reference in its entirety.

The CDPs described herein can be made using a variety of methods including those described herein. In some embodiments, a CDP can be made by: providing cyclodextrin moiety precursors; providing comonomer precursors which do not contain cyclodextrin moieties (comonomer precursors); and copolymerizing the said cyclodextrin moiety precursors and comonomer precursors to thereby make a CDP wherein CDP comprises at least four cyclodextrin moieties and at least four comonomers.

In some embodiments, the at least four cyclodextrin moieties and at least four comonomers alternate in the water soluble linear polymer. In some embodiments, the method includes providing cyclodextrin moiety precursors modified to bear one reactive site at each of exactly two positions, and reacting the cyclodextrin moiety precursors with comonomer precursors having exactly two reactive moieties capable of forming a covalent bond with the reactive sites under polymerization conditions that promote reaction of the reactive sites with the reactive moieties to form covalent bonds between the comonomers and the cyclodextrin moieties, whereby a CDP comprising alternating units of a cyclodextrin moiety and a comonomer is produced.

In some embodiments, the cyclodextrin momomers comprise linkers to which the camptothecin or camptothecin derivative may be further linked.

In some embodiments, the comonomer precursor is a compound containing at least two functional groups through which reaction and thus linkage of the cyclodextrin moieties is achieved. In some embodiments, the functional groups, which may be the same or different, terminal or internal, of each comonomer precursor comprise an amino, acid, imidazole, hydroxyl, thio, acyl halide, -HC=CH-, -C≡C- group, or derivative thereof. In some embodiments, the two functional groups are the same and are located at termini of the comonomer precursor. In some embodiments, a comonomer contains one or more pendant groups with at least one functional group through which reaction and thus linkage of a therapeutic agent can be achieved. In some embodiments, the functional groups, which may be the same or different, terminal or internal, of each comonomer pendant group comprise an amino, acid, imidazole, hydroxyl, thiol, acyl halide, ethylene, ethyne group, or derivative thereof. In some embodiments, the pendant group is a substituted or unsubstituted branched, cyclic or straight chain C1-C10 alkyl, or arylalkyl optionally containing one or more heteroatoms within the chain or ring.

In some embodiments, the cyclodextrin moiety comprises an alpha, beta, or gamma cyclodextrin moiety.

In some embodiments, the CDP is suitable for the attachment of sufficient camptothecin or camptothecin derivative such that up to at least 3%, 5%, 10%, 11%, 12%, 13%, 14%, 15%, 20%, 25%, 30%, or even 35% by weight of the CDP, when conjugated, is camptothecin or a camptothecin derivative.

In some embodiments, the CDP has a molecular weight of 10,000-500,000 amu. In some embodiments, the cyclodextrin moieties make up at least about 2%, 5%, 10%, 20%, 30%, 50% or 80% of the CDP by weight.

In some embodiments, a CDP of the following formula can be made by the scheme below: 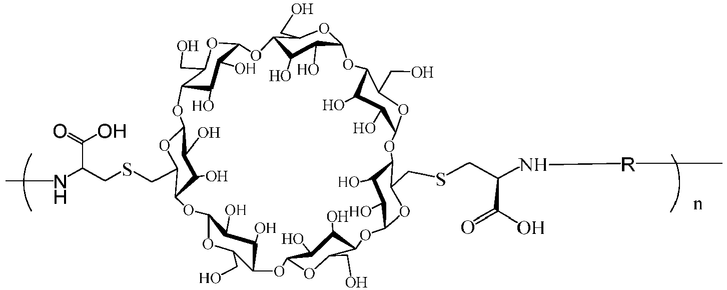 wherein R is of the form: 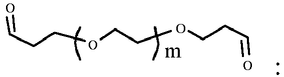 comprising the steps of:
reacting a compound of the formula below: 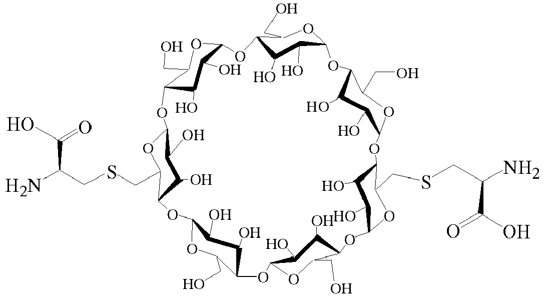 with a compound of the formula below: 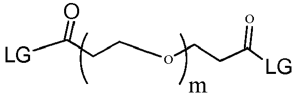 wherein the group 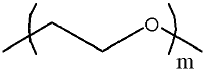 has a Mw of about 2 to about 5 kDa (e.g., from about 2 to about 4.5 kDa, from about 3 to about 4 kDa, or less than about 4 kDa, (e.g., about 3.4 kDa ± 10%, e.g., about 3060 Da to about 3740 Da)) and n is at least four,
in the presence of a non-nucleophilic organic base in a solvent.

In some embodiments, 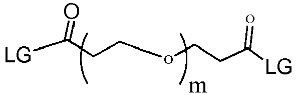 is

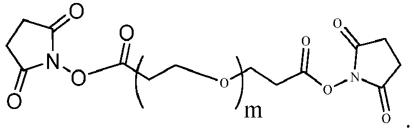

In some embodiments, the solvent is a polar aprotic solvent. In some embodiments, the solvent is DMSO.

In some embodiments, the method also includes the steps of dialysis; and lyophylization.

In some embodiments, a CDP provided below can be made by the following scheme: 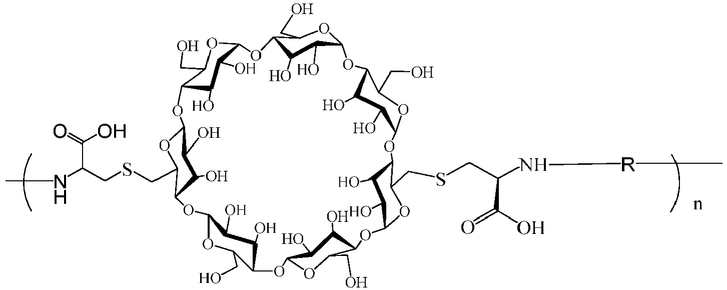 wherein R is of the form:
with a compound provided below: 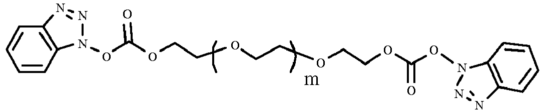 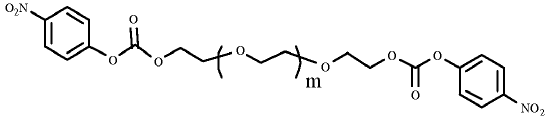 wherein the group 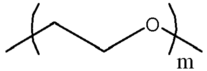 has a Mw of about 2 to about 5 kDa (e.g., from about 2 to about 4.5 kDa, from about 3 to about 4 kDa, or less than about 4 kDa, (e.g., about 3.4 kDa ± 10%, e.g., about 3060 Da to about 3740 Da));
in the presence of a non-nucleophilic organic base in DMSO;
and dialyzing and lyophilizing the following polymer

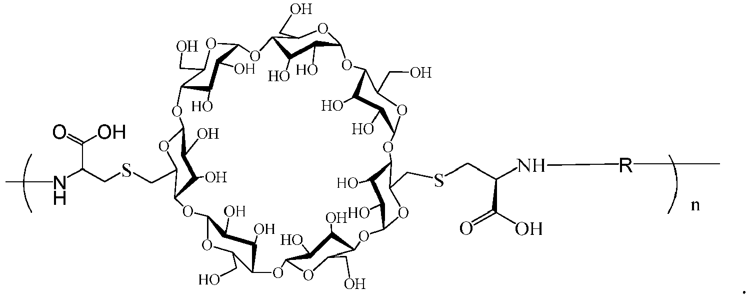

The present invention further contemplates CDPs and CDP-conjugates synthesized using CD-biscysteine monomer and a di-NHS ester such as PEG-DiSPA or PEG-BTC as shown in Scheme I. 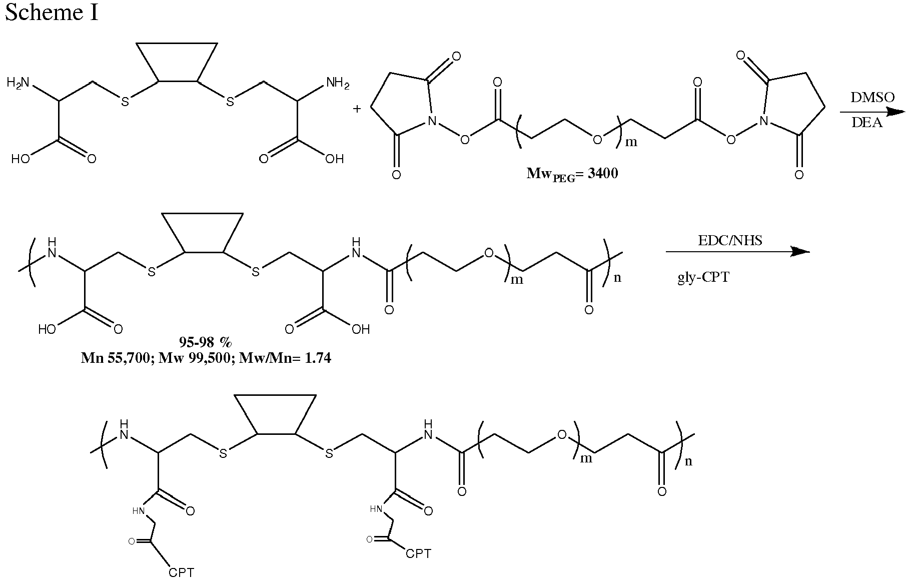 Scheme XIII, as provided above, includes embodiments where gly-CPT is absent in one or more positions as provided above. This can be achieved, for example, when less than 100% yield is achieved when coupling the CPT to the polymer and/or when less than an equivalent amount of CPT is used in the reaction. Accordingly, the loading of the camptothecin or camptothecin derivative, by weight of the polymer, can vary. Therefore, while Scheme XIII depicts CPT at each cysteine residue of each polymer subunit, the CDP-CPT conjugate can have less than 2 CPT molecules attached to any given polymer subunit of the CDP. For example, in one embodiment, the CDP-CPT conjugate includes several polymer subunits and each of the polymer subunits can independently include two, one or no CPT attached at each cysteine residue of the polymer subunit. In addition, the particles and compositions can include CDP-CPT conjugates having two, one or no CPT attached at each cysteine residue of each polymer subunit of the CDP-CPT conjugate and the conjugates include a mixture of CDP-CPT conjugates that can vary as to the number of CPTs attached to the gly at each of the polymer subunits of the conjugates in the particle or composition.

In some embodiments, a CDP-camptothecin or camptothecin derivative conjugate can be made by providing a CDP comprising cyclodextrin moieties and comonomers which do not contain cyclodextrin moieties (comonomers), wherein the cyclodextrin moieties and comonomers alternate in the CDP and wherein the CDP comprises at least four cyclodextrin moieties and at least four comonomers; and attaching a camptothecin or camptothecin derivative to the CDP.

In some embodiments, one or more of the camptothecin or camptothecin derivative moieties in the CDP-camptothecin or camptothecin derivative conjugate can be replaced with another therapeutic agent, e.g., another anticancer agent or antiinflammatory agent.

In some embodiments, the camptothecin or camptothecin derivative is attached to the water soluble linear polymer via a linker. In some embodiments, the camptothecin or camptothecin derivative is attached to the water soluble linear polymer through an attachment that is cleaved under biological conditions to release the camptothecin or camptothecin derivative. In some embodiments, the camptothecin or camptothecin derivative is attached to the water soluble linear polymer at a cyclodextrin moiety or a comonomer. In some embodiments, the camptothecin or camptothecin derivative is attached to the water soluble linear polymer via an optional linker to a cyclodextrin moiety or a comonomer.

In some embodiments, the cyclodextrin moieties comprise linkers to which therapeutic agents are linked.

In some embodiments, the CDP is made by a process comprising: providing cyclodextrin moiety precursors, providing comonomer precursors, and copolymerizing said cyclodextrin moiety precursors and comonomer precursors to thereby make a CDP comprising cyclodextrin moieties and comonomers. In some embodiments, the CDP is conjugated with a camptothecin to provide a CDP-camptothecin or camptothecin derivative conjugate.

In some embodiments, the method includes providing cyclodextrin moiety precursors modified to bear one reactive site at each of exactly two positions, and reacting the cyclodextrin moiety precursors with comonomer precursors having exactly two reactive moieties capable of forming a covalent bond with the reactive sites under polymerization conditions that promote reaction of the reactive sites with the reactive moieties to form covalent bonds between the comonomers and the cyclodextrin moieties, whereby a CDP comprising alternating units of a cyclodextrin moiety and a comonomer is produced.

In some embodiments, the camptothecin or camptothecin derivative is attached to the CDP via a linker. In some embodiments, the linker is cleaved under biological conditions.

In some embodiments, the camptothecin or camptothecin derivative makes up at least 5%, 10%, 11%, 12%, 13%, 14%, 15%, 20%, 25%, 30%, or even 35% by weight of the CDP-camptothecin or CDP-camptothecin derivative conjugate.

In some embodiments, the comonomer comprises polyethylene glycol of molecular weight of about 2 to about 5 kDa (e.g., from about 2 to about 4.5 kDa, from about 3 to about 4 kDa, or less than about 4 kDa, (e.g., about 3.4 kDa ± 10%, e.g., about 3060 Da to about 3740 Da)), the cyclodextrin moiety comprises beta-cyclodextrin, the theoretical maximum loading of camptothecin on a CDP-camptothecin conjugate is 13%, and camptothecin is 6-10% by weight of the CDP-camptothecin conjugate.

In some embodiments, the comonomer precursor is a compound containing at least two functional groups through which reaction and thus linkage of the cyclodextrin moieties is achieved. In some embodiments, the functional groups, which may be the same or different, terminal or internal, of each comonomer precursor comprise an amino, acid, imidazole, hydroxyl, thio, acyl halide, -HC=CH-, -C≡C- group, or derivative thereof. In some embodiments, the two functional groups are the same and are located at termini of the comonomer precursor. In some embodiments, a comonomer contains one or more pendant groups with at least one functional group through which reaction and thus linkage of a therapeutic agent is achieved. In some embodiments, the functional groups, which may be the same or different, terminal or internal, of each comonomer pendant group comprise an amino, acid, imidazole, hydroxyl, thiol, acyl halide, ethylene, ethyne group, or derivative thereof. In some embodiments, the pendant group is a substituted or unsubstituted branched, cyclic or straight chain C1-C10 alkyl, or arylalkyl optionally containing one or more heteroatoms within the chain or ring.

In some embodiments, the cyclodextrin moiety comprises an alpha, beta, or gamma cyclodextrin moiety.

In some embodiments, the camptothecin or camptothecin derivative is poorly soluble in water.

In some embodiments, administration of the CDP-camptothecin or camptothecin derivative conjugate, particle or composition, e.g., a CDP-camptothecin or camptothecin derivative conjugate, particle or composition described herein, e.g., CRLX101, to a subject results in release of the a camptothecin or a camptothecin derivative over a period of at least 6 hours. In some embodiments, administration of the CDP-camptothecin or camptothecin derivative conjugate, particle or composition, e.g., a CDP-camptothecin or camptothecin derivative conjugate, particle or composition described herein, e.g., CRLX101, to a subject results in release of the a camptothecin or a camptothecin derivative over a period of 6 hours to a month. In some embodiments, upon administration of the CDP-camptothecin or camptothecin derivative conjugate, particle or composition, e.g., a CDP-camptothecin or camptothecin derivative conjugate, particle or composition described herein, e.g., CRLX101, to a subject the rate of a camptothecin or camptothecin derivative release is dependent primarily upon the rate of hydrolysis as opposed to enzymatic cleavage.

In some embodiments, the CDP-camptothecin or camptothecin derivative conjugate, particle or composition, e.g., a CDP-camptothecin or camptothecin derivative conjugate, particle or composition described herein, e.g., CRLX101, has a molecular weight of 10,000-500,000 amu.

In some embodiments, the cyclodextrin moieties make up at least about 2%, 5%, 10%, 20%, 30%, 50% or 80% of the polymer by weight.

In some embodiments, a CDP-polymer conjugate of the following formula can be made as follows: 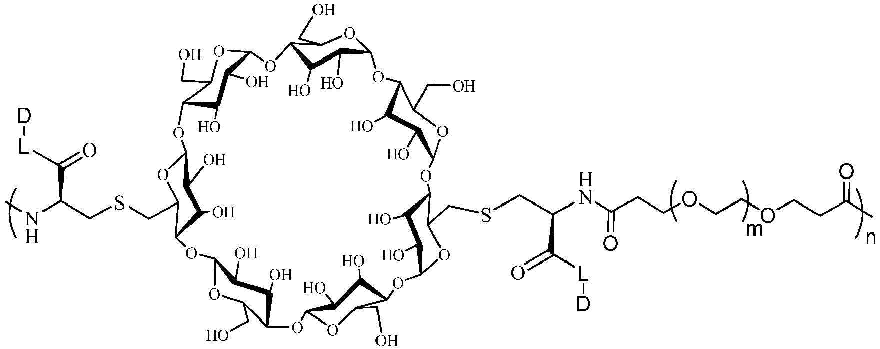 providing a polymer below: 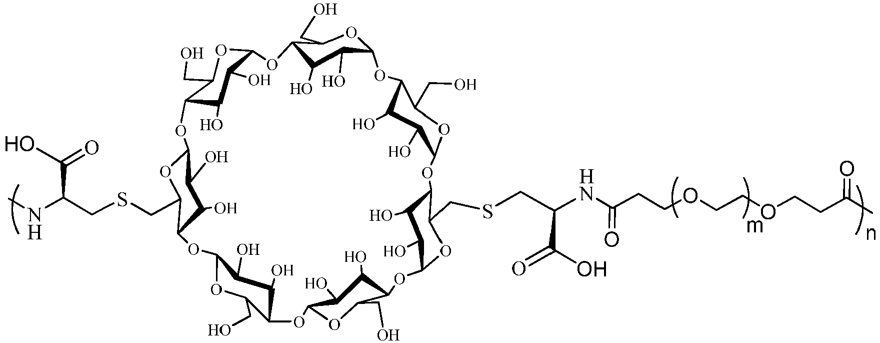 and coupling the polymer with a plurality of L-D moieties, wherein L is a linker, or absent and D is camptothecin or a camptothecin derivative, to provide: 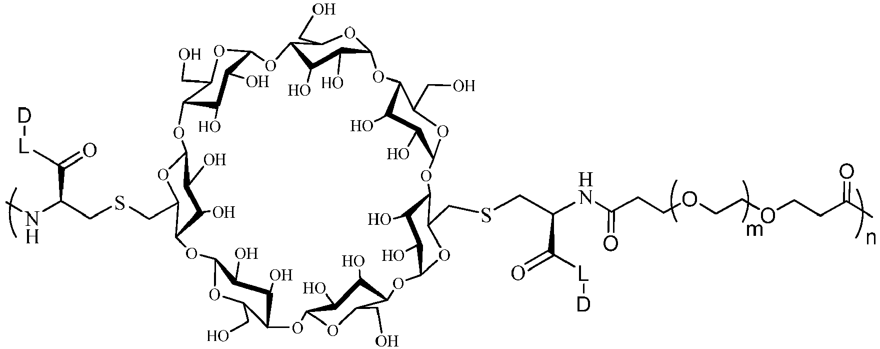 wherein the group 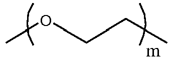 has a Mw of about 2 to about 5 kDa (e.g., from about 2 to about 4.5 kDa, from about 3 to about 4 kDa, or less than about 4 kDa, (e.g., about 3.4 kDa ± 10%, e.g., about 3060 Da to about 3740 Da)) and n is at least 4, wherein on the final product, L can be a linker, a bond, or OH, and D can be camptothecin or a camptothecin derivative or absent.

The reaction scheme as provided above includes embodiments where L-D is absent in one or more positions as provided above. This can be achieved, for example, when less than 100% yield is achieved when coupling the a camptothecin or camptothecin derivative-linker to the polymer and/or when less than an equivalent amount of a camptothecin or camptothecin derivative-linker is used in the reaction. Accordingly, the loading of the a camptothecin or a camptothecin derivative, by weight of the polymer, can vary, for example, the loading of the a camptothecin or a camptothecin derivative can be at least about 3% by weight, e.g., at least about 5%, at least about 8%, at least about 10%, at least about 11%, at least about 12%, at least about 13%, at least about 14%, at least about 15%, or at least about 20%.

In some embodiments, at least a portion of the L moieties of L-D is absent. In some embodiments, each L is independently an amino acid or derivative thereof (e.g., glycine).

In some embodiments, the coupling of the polymer with the plurality of L-D moieties results in the formation of a plurality of amide bonds.

In certain instances, the CDPs are random copolymers, in which the different subunits and/or other monomeric units are distributed randomly throughout the polymer chain. Thus, where the formula Xₘ₋Yₙ-Zₒ appears, wherein X, Y and Z are polymer subunits, these subunits may be randomly interspersed throughout the polymer backbone. In part, the term "random" is intended to refer to the situation in which the particular distribution or incorporation of monomeric units in a polymer that has more than one type of monomeric units is not directed or controlled directly by the synthetic protocol, but instead results from features inherent to the polymer system, such as the reactivity, amounts of subunits and other characteristics of the synthetic reaction or other methods of manufacture, processing, or treatment.

### Pharmaceutical Compositions

In another aspect, the disclosure provides a composition, e.g., a pharmaceutical composition, comprising a CDP-camptothecin or CDP-camptothecin derivative conjugate or particle and a pharmaceutically acceptable carrier or adjuvant.

In some embodiments, a pharmaceutical composition may include a pharmaceutically acceptable salt of a compound described herein, e.g., a CDP-camptothecin or camptothecin derivative conjugate. Pharmaceutically acceptable salts of the compounds described herein include those derived from pharmaceutically acceptable inorganic and organic acids and bases. Examples of suitable acid salts include acetate, adipate, benzoate, benzenesulfonate, butyrate, citrate, digluconate, dodecylsulfate, formate, fumarate, glycolate, hemisulfate, heptanoate, hexanoate, hydrochloride, hydrobromide, hydroiodide, lactate, maleate, malonate, methanesulfonate, 2-naphthalenesulfonate, nicotinate, nitrate, palmoate, phosphate, picrate, pivalate, propionate, salicylate, succinate, sulfate, tartrate, tosylate and undecanoate. Salts derived from appropriate bases include alkali metal (e.g., sodium), alkaline earth metal (e.g., magnesium), ammonium and N-(alkyl)₄⁺ salts. This invention also envisions the quaternization of any basic nitrogen-containing groups of the compounds described herein. Water or oil-soluble or dispersible products may be obtained by such quaternization.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gailate, aipha-tocopherol, and the like; and (3) metal chelating agents, such as citric acid, ethylenediamine tetraacetic acid (EDTA), sorbitol, tartaric acid, phosphoric acid, and the like.

A composition may include a liquid used for suspending a CDP-camptothecin or camptothecin derivative conjugate, particle or composition, e.g., a CDP-camptothecin or camptothecin derivative conjugate, particle or composition described herein, e.g., CRLX101, which may be any liquid solution compatible with the CDP-camptothecin or camptothecin derivative conjugate, particle or composition, e.g., a CDP-camptothecin or camptothecin derivative conjugate, particle or composition described herein, e.g., CRLX101, which is also suitable to be used in pharmaceutical compositions, such as a pharmaceutically acceptable nontoxic liquid. Suitable suspending liquids including but are not limited to suspending liquids selected from the group consisting of water, aqueous sucrose syrups, corn syrups, sorbitol, polyethylene glycol, propylene glycol, and mixtures thereof.

A composition described herein may also include another component, such as an antioxidant, antibacterial, buffer, bulking agent, chelating agent, an inert gas, a tonicity agent and/or a viscosity agent.

In one embodiment, the CDP-camptothecin or camptothecin derivative conjugate, particle or composition, e.g., a CDP-camptothecin or camptothecin derivative conjugate, particle or composition described herein, e.g., CRLX101, is provided in lyophilized form and is reconstituted prior to administration to a subject. The lyophilized CDP-camptothecin or camptothecin derivative conjugate, particle or composition, e.g., a CDP-camptothecin or camptothecin derivative conjugate, particle or composition described herein, e.g., CRLX101, can be reconstituted by a diluent solution, such as a salt or saline solution, e.g., a sodium chloride solution having a pH between 6 and 9, lactated Ringer's injection solution, or a commercially available diluent, such as PLASMA-LYTE A Injection pH 7.4® (Baxter, Deerfield, IL).

In one embodiment, a lyophilized formulation includes a lyoprotectant or stabilizer to maintain physical and chemical stability by protecting the CDP-camptothecin or camptothecin derivative conjugate, particle or composition, e.g., a CDP-camptothecin or camptothecin derivative conjugate, particle or composition described herein, e.g., CRLX101, from damage from crystal formation and the fusion process during freeze-drying. The lyoprotectant or stabilizer can be one or more of polyethylene glycol (PEG), a PEG lipid conjugate (e.g., PEG-ceramide or D-alpha-tocopheryl polyethylene glycol 1000 succinate), poly(vinyl alcohol) (PVA), poly(vinylpyrrolidone) (PVP), polyoxyethylene esters, poloxomers, Tweens, lecithins, saccharides, oligosaccharides, polysaccharides and polyols (e.g., trehalose, mannitol, sorbitol, lactose, sucrose, glucose and dextran), salts and crown ethers. In one embodiment, the lyoprotectant is mannitol.

In some embodiments, the lyophilized CDP-camptothecin or camptothecin derivative conjugate, particle or composition, e.g., a CDP-camptothecin or camptothecin derivative conjugate, particle or composition described herein, e.g., CRLX101, is reconstituted with a mixture of equal parts by volume of Dehydrated Alcohol, USP and a nonionic surfactant, such as a polyoxyethylated castor oil surfactant available from GAF Corporation, Mount Olive, N.J., under the trademark, Cremophor EL. In some embodiments, the lyophilized CDP-camptothecin or camptothecin derivative conjugate, particle or composition, e.g., a CDP-camptothecin or camptothecin derivative conjugate, particle or composition described herein, e.g., CRLX101, is reconstituted in water for infusion. The lyophilized product and vehicle for reconstitution can be packaged separately in appropriately light-protected vials, e.g., amber or other colored vials. To minimize the amount of surfactant in the reconstituted solution, only a sufficient amount of the vehicle may be provided to form a solution having a concentration of about 2 mg/mL to about 4 mg/mL of the CDP-camptothecin or camptothecin derivative conjugate, particle or composition, e.g., a CDP-camptothecin or camptothecin derivative conjugate, particle or composition described herein, e.g., CRLX101. Once dissolution of the drug is achieved, the resulting solution is further diluted prior to injection with a suitable parenteral diluent. Such diluents are well known to those of ordinary skill in the art. These diluents are generally available in clinical facilities. It is, however, within the scope of the present invention to package the subject CDP-camptothecin or camptothecin derivative conjugate, particle or composition, e.g., a CDP-camptothecin or camptothecin derivative conjugate, particle or composition described herein, e.g., CRLX101, with a third vial containing sufficient parenteral diluent to prepare the final concentration for administration. A typical diluent is Lactated Ringer's Injection.

The final dilution of the reconstituted CDP-camptothecin or camptothecin derivative conjugate, particle or composition, e.g., a CDP-camptothecin or camptothecin derivative conjugate, particle or composition described herein, e.g., CRLX101, Lactated Ringer's and Dextrose for Injection (D5W), Sterile Water for Injection, and the like. However, because of its narrow pH range, pH 6.0 to 7.5, Lactated Ringer's Injection is most typical. Per 100 mL, Lactated Ringer's Injection contains Sodium Chloride USP 0.6 g, Sodium Lactate 0.31 g, Potassium chloride USP 0.03 g and Calcium Chloride2H2O USP 0.02 g. The osmolarity is 275 mOsmol/L, which is very close to isotonicity.

The compositions may conveniently be presented in unit dosage form and may be prepared by any methods well known in the art of pharmacy. The dosage form can be, e.g., in a bog, e.g., a bag for infusion or intraperitoneal administration. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

### Routes of Administration

The pharmaceutical compositions described herein may be administered orally, parenterally (e.g., via intravenous, subcutaneous, intracutaneous, intramuscular, intraarticular, intraarterial, intraperitoneal, intrasynovial, intrasternal, intrathecal, intralesional or intracranial injection), topically, mucosally (e.g., rectally or vaginally), nasally, buccally, ophthalmically, via inhalation spray (e.g., delivered via nebulzation, propellant or a dry powder device) or via an implanted reservoir. Typically, the compositions are in the form of injectable or infusible solutions. The preferred mode of administration is, e.g., intravenous, subcutaneous, intraperitoneal, intramuscular.

Pharmaceutical compositions suitable for parenteral administration comprise one or more CDP-camptothecin or camptothecin derivative conjugate(s), particle(s) or composition(s) in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the agent from subcutaneous or intramuscular injection. This may be accomplished by the use of a liquid suspension of crystalline or amorphous material having poor water solubility. The rate of absorption of the CDP-camptothecin or camptothecin derivative conjugate, particle or composition, e.g., a CDP-camptothecin or camptothecin derivative conjugate, particle or composition described herein, e.g., CRLX101, then depends upon its rate of dissolution which, in turn, may depend upon crystal size and crystalline form. Alternatively, delayed absorption of a parenterally administered drug form is accomplished by dissolving or suspending the CDP-camptothecin or camptothecin derivative conjugate, particle or composition, e.g., a CDP-camptothecin or camptothecin derivative conjugate, particle or composition described herein, e.g., CRLX101, in an oil vehicle.

### Dosages and Dosing Regimens

The CDP-camptothecin or camptothecin derivative conjugate, particle or composition, e.g., a CDP-camptothecin or camptothecin derivative conjugate, particle or composition described herein, e.g., CRLX101, can be formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular subject, composition, and mode of administration, without being toxic to the subject.

In one embodiment, the CDP-camptothecin or camptothecin derivative conjugate, particle or composition, e.g., a CDP-camptothecin or camptothecin derivative conjugate, particle or composition described herein, e.g., CRLX101, is administered to a subject at a dosage of, e.g., about 1 to 40 mg/m², about 3 to 35 mg/m², about 9 to 40 mg/m², e.g., about 1, 3, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 mg/m² of the camptothecin or camptothecin derivative. Administration can be at regular intervals, such as weekly, or every 2, 3, 4, 5 or 6 weeks. The administration can be over a period of from about 10 minutes to about 6 hours, e.g., from about 30 minutes to about 2 hours, from about 45 minutes to 90 minutes, e.g., about 30 minutes, 45 minutes, 1 hour, 2 hours, 3 hours, 4 hours, 5 hours or more. The CDP-camptothecin or camptothecin derivative conjugate, particle or composition, e.g., a CDP-camptothecin or camptothecin derivative conjugate, particle or composition described herein, e.g., CRLX101, can be administered, e.g., by intravenous or intraperitoneal administration.

In one embodiment, the CDP-camptothecin or camptothecin derivative conjugate, particle or composition, e.g., a CDP-camptothecin or camptothecin derivative conjugate, particle or composition described herein, e.g., CRLX101, is administered as a bolus infusion or intravenous push, e.g., over a period of 15 minutes, 10 minutes, 5 minutes or less. In one embodiment, the CDP-camptothecin or camptothecin derivative conjugate, particle or composition, e.g., a CDP-camptothecin or camptothecin derivative conjugate, particle or composition described herein, e.g., CRLX101, is administered in an amount such the desired dose of the agent is administered. Preferably the dose of the CDP-camptothecin or camptothecin derivative conjugate, particle or composition, e.g., a CDP-camptothecin or camptothecin derivative conjugate, particle or composition described herein, e.g., CRLX101, is a dose described herein.

In one embodiment, the subject receives 1, 2, 3, up to 10 treatments, or more, or until the disorder or a symptom of the disorder is cured, healed, alleviated, relieved, altered, remedied, ameliorated, palliated, improved or affected. For example, the subject receives an infusion once every 1, 2, 3 or 4 weeks until the disorder or a symptom of the disorder is cured, healed, alleviated, relieved, altered, remedied, ameliorated, palliated, improved or affected. Preferably, the dosing schedule is a dosing schedule described herein.

In one embodiment, the subject has a pathological complete response (pCR), e.g., after one course of treatment. In one embodiment, the subject has a pCR after one course of treatment and the subject is administered one or more additional courses of treatment. In one embodiment, the subject does not have a pathological complete response (pCR), e.g., after one course of treatment. In one embodiment, the subject does not have a pCR after one course of treatment and the subject is administered one or more additional courses of treatment. The term "course" as used herein refers to three administrations of the CDP-camptothecin or camptothecin derivative conjugate, particle or composition, e.g., CRLX101, each of the second and third administrations being between 12, 13, 14, 15 or 16 days, after the previous administration.

The CDP-camptothecin or camptothecin derivative conjugate, particle or composition, e.g., the CDP-camptothecin or camptothecin derivative conjugate, particle or composition described herein, e.g., CRLX101, can be administered as a first line therapy, e.g., alone or in combination with an additional agent or agents. In other embodiments, the CDP-camptothecin or camptothecin derivative conjugate, particle or composition, e.g., the CDP-camptothecin or camptothecin derivative conjugate, particle or composition described herein, e.g., CRLX101, is administered after a subject has developed resistance to, has failed to respond to or has relapsed after a first line therapy. The CDP-camptothecin or camptothecin derivative conjugate, particle or composition, e.g., the CDP-camptothecin or camptothecin derivative conjugate, particle or composition described herein, e.g., CRLX101, can be administered in combination with a second agent. Preferably, the CDP-camptothecin or camptothecin derivative conjugate, particle or composition, e.g., the CDP-camptothecin or camptothecin derivative conjugate, particle or composition described herein, e.g., CRLX101, is administered in combination with a second agent described herein.

### Kits

A CDP-camptothecin or camptothecin derivative conjugate, particle or composition, e.g., a CDP-camptothecin or camptothecin derivative conjugate, particle or composition described herein, e.g., CRLX101, may be provided in a kit. The kit includes a CDP-camptothecin or camptothecin derivative conjugate, particle or composition, e.g., a CDP-camptothecin or camptothecin derivative conjugate, particle or composition described herein, e.g., CRLX101, and, optionally, a container, a pharmaceutically acceptable carrier and/or informational material. The informational material can be descriptive, instructional, marketing or other material that relates to the methods described herein and/or the use of the CDP-camptothecin or camptothecin derivative conjugate, particle or composition, e.g., the CDP-camptothecin or camptothecin derivative conjugate, particle or composition described herein, e.g., CRLX101, for the methods described herein.

The informational material of the kits is not limited in its form. In one embodiment, the informational material can include information about production of the CDP-camptothecin or camptothecin derivative conjugate, particle or composition, e.g., the CDP-camptothecin or camptothecin derivative conjugate, particle or composition described herein, e.g., CRLX101, physical properties of the CDP-camptothecin or camptothecin derivative conjugate, particle or composition, e.g., the CDP-camptothecin or camptothecin derivative conjugate, particle or composition described herein, e.g., CRLX101, concentration, date of expiration, batch or production site information, and so forth. In one embodiment, the informational material relates to methods for administering the CDP-camptothecin or camptothecin derivative conjugate, particle or composition, e.g., the CDP-camptothecin or camptothecin derivative conjugate, particle or composition described herein, e.g., CRLX101, e.g., by a route of administration described herein and/or at a dose and/or dosing schedule described herein.

In one embodiment, the informational material can include instructions to administer a CDP-camptothecin or camptothecin derivative conjugate, particle or composition, e.g., a CDP-camptothecin or camptothecin derivative conjugate, particle or composition described herein, e.g., CRLX101, in a suitable manner to perform the methods described herein, e.g., in a suitable dose, dosage form, or mode of administration (e.g., a dose, dosage form, or mode of administration described herein). In another embodiment, the informational material can include instructions to administer a CDP-camptothecin or camptothecin derivative conjugate, particle or composition, e.g., the CDP-camptothecin or camptothecin derivative conjugate, particle or composition described herein, e.g., CRLX101, to a suitable subject, e.g., a human, e.g., a human having or at risk for a disorder described herein. In another embodiment, the informational material can include instructions to reconstitute a CDP-camptothecin or camptothecin derivative conjugate, particle or composition, e.g., the CDP-camptothecin or camptothecin derivative conjugate, particle or composition described herein, e.g., CRLX101, into a pharmaceutically acceptable composition.

In one embodiment, the kit includes instructions to use the CDP-camptothecin or camptothecin derivative conjugate, particle or composition, e.g., the CDP-camptothecin or camptothecin derivative conjugate, particle or composition described herein, e.g., CRLX101, such as for treatment of a subject. The instructions can include methods for reconstituting or diluting the CDP-camptothecin or camptothecin derivative conjugate, particle or composition, e.g., the CDP-camptothecin or camptothecin derivative conjugate, particle or composition described herein, e.g., CRLX101, for use with a particular subject or in combination with a particular chemotherapeutic agent. The instructions can also include methods for reconstituting or diluting the CDP-camptothecin or camptothecin derivative conjugate, particle or composition, e.g., the CDP-camptothecin or camptothecin derivative conjugate, particle or composition described herein, e.g., CRLX101, for use with a particular means of administration, such as by intravenous infusion or intraperitoneal administration.

In another embodiment, the kit includes instructions for treating a subject with a particular indication, such as a particular cancer, or a cancer at a particular stage. For example, the instructions can be for a cancer or cancer at stage described herein, e.g., colorectal, e.g., rectal cancer. The instructions may also address first line treatment of a subject who has a particular cancer, or cancer at a stage described herein. The instructions can also address treatment of a subject who has been non-responsive to a first line therapy or has become sensitive (e.g., has one or more unacceptable side effect) to a first line therapy, such as a taxane, an anthracycline, an antimetabolite, a vinca alkaloid, a vascular endothelial growth factor (VEGF) pathway inhibitor, an epidermal growth factor (EGF) pathway inhibitor, an alkylating agent, a platinum-based agent, a vinca alkaloid. In another embodiment, the instructions will describe treatment of selected subjects with the CDP-camptothecin or camptothecin derivative conjugate, particle or composition, e.g., the CDP-camptothecin or camptothecin derivative conjugate, particle or composition described herein, e.g., CRLX101.

The informational material of the kits is not limited in its form. In many cases, the informational material, e.g., instructions, is provided in printed matter, e.g., a printed text, drawing, and/or photograph, e.g., a label or printed sheet. However, the informational material can also be provided in other formats, such as Braille, computer readable material, video recording, or audio recording. In another embodiment, the informational material of the kit is contact information, e.g., a physical address, email address, website, or telephone number, where a user of the kit can obtain substantive information about a CDP-camptothecin or camptothecin derivative conjugate, particle or composition, e.g., a CDP-camptothecin or camptothecin derivative conjugate, particle or composition described herein, e.g., CRLX101, and/or its use in the methods described herein. The informational material can also be provided in any combination of formats.

In addition to a CDP-camptothecin or camptothecin derivative conjugate, particle or composition, e.g., a CDP-camptothecin or camptothecin derivative conjugate, particle or composition described herein, e.g., CRLX101, the composition of the kit can include other ingredients, such as a surfactant, a lyoprotectant or stabilizer, an antioxidant, an antibacterial agent, a bulking agent, a chelating agent, an inert gas, a tonicity agent and/or a viscosity agent, a solvent or buffer, a stabilizer, a preservative, a flavoring agent (e.g., a bitter antagonist or a sweetener), a fragrance, a dye or coloring agent, for example, to tint or color one or more components in the kit, or other cosmetic ingredient, a pharmaceutically acceptable carrier and/or a second agent for treating a condition or disorder described herein. Alternatively, the other ingredients can be included in the kit, but in different compositions or containers than a CDP-camptothecin or camptothecin derivative conjugate, particle or composition, e.g., a CDP-camptothecin or camptothecin derivative conjugate, particle or composition described herein, e.g., CRLX101. In such embodiments, the kit can include instructions for admixing a CDP-camptothecin or camptothecin derivative conjugate, particle or composition, e.g., a CDP-camptothecin or camptothecin derivative conjugate, particle or composition described herein, e.g., CRLX101, together with the other ingredients. For example, the kit can include an agent which reduces or inhibits one or more symptom of hypersensitivity, a polysaccharide, and/or an agent which increases urinary excretion and/or neutralizes one or more urinary metabolite.

In another embodiment, the kit includes a second therapeutic agent, such as a second chemotherapeutic agent, e.g., a chemotherapeutic agent or combination of chemotherapeutic agents described herein. In one embodiment, the second agent is in lyophilized or in liquid form. In one embodiment, the CDP-camptothecin or camptothecin derivative conjugate, particle or composition, e.g., the CDP-camptothecin or camptothecin derivative conjugate, particle or composition described herein, e.g., CRLX101, and the second therapeutic agent are in separate containers, and in another embodiment, the CDP-camptothecin or camptothecin derivative conjugate, particle or composition, e.g., the CDP-camptothecin or camptothecin derivative conjugate, particle or composition described herein, e.g., CRLX101, and the second therapeutic agent are packaged in the same container.

In some embodiments, a component of the kit is stored in a sealed vial, e.g., with a rubber or silicone closure (e.g., a polybutadiene or polyisoprene closure). In some embodiments, a component of the kit is stored under inert conditions (e.g., under Nitrogen or another inert gas such as Argon). In some embodiments, a component of the kit is stored under anhydrous conditions (e.g., with a desiccant). In some embodiments, a component of the kit is stored in a light blocking container such as an amber vial.

A CDP-camptothecin or camptothecin derivative conjugate, particle or composition, e.g., a CDP-camptothecin or camptothecin derivative conjugate, particle or composition described herein, e.g., CRLX101, can be provided in any form, e.g., liquid, frozen, dried or lyophilized form. It is preferred that a composition including the conjugate, particle or composition, e.g., a composition comprising a particle or particles that include a conjugate described herein be substantially pure and/or sterile. When a CDP-camptothecin or camptothecin derivative conjugate, particle or composition, e.g., a CDP-camptothecin or camptothecin derivative conjugate, particle or composition described herein, e.g., CRLX101, is provided in a liquid solution, the liquid solution preferably is an aqueous solution, with a sterile aqueous solution being preferred. In one embodiment, the CDP-camptothecin or camptothecin derivative conjugate, particle or composition, e.g., the CDP-camptothecin or camptothecin derivative conjugate, particle or composition described herein, e.g., CRLX101, is provided in lyophilized form and, optionally, a diluent solution is provided for reconstituting the lyophilized agent. The diluent can include for example, a salt or saline solution, e.g., a sodium chloride solution having a pH between 6 and 9, lactated Ringer's injection solution, D5W, or PLASMA-LYTE A Injection pH 7.4® (Baxter, Deerfield, IL).

The kit can include one or more containers for the composition containing a CDP-camptothecin or camptothecin derivative conjugate, particle or composition, e.g., a CDP-camptothecin or camptothecin derivative conjugate, particle or composition described herein, e.g., CRLX101. In some embodiments, the kit contains separate containers, dividers or compartments for the composition and informational material. For example, the composition can be contained in a bottle, vial, IV admixture bag, IV infusion set, piggyback set or syringe, and the informational material can be contained in a plastic sleeve or packet. In other embodiments, the separate elements of the kit are contained within a single, undivided container. For example, the composition is contained in a bottle, vial or syringe that has attached thereto the informational material in the form of a label. In some embodiments, the kit includes a plurality (e.g., a pack) of individual containers, each containing one or more unit dosage forms (e.g., a dosage form described herein) of a CDP-camptothecin or camptothecin derivative conjugate, particle or composition, e.g., the CDP-camptothecin or camptothecin derivative conjugate, particle or composition described herein, e.g., CRLX101. For example, the kit includes a plurality of syringes, ampules, foil packets, or blister packs, each containing a single unit dose of a particle described herein. The containers of the kits can be air tight, waterproof (e.g., impermeable to changes in moisture or evaporation), and/or light-tight.

The kit optionally includes a device suitable for administration of the composition, e.g., a syringe, inhalant, pipette, forceps, measured spoon, dropper (e.g., eye dropper), swab (e.g., a cotton swab or wooden swab), or any such delivery device. In one embodiment, the device is a medical implant device, e.g., packaged for surgical insertion.

### Combination therapy

The CDP-camptothecin or camptothecin derivative conjugate, particle or composition, e.g., the CDP-camptothecin or camptothecin derivative conjugate, particle or composition described herein, e.g., CRLX101, may be used in combination with other known therapies. Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

The CDP-camptothecin or camptothecin derivative conjugate, particle or composition, e.g., the CDP-camptothecin or camptothecin derivative conjugate, particle or composition described herein, e.g., CRLX101, and the at least one additional therapeutic agent can be administered simultaneously, in the same or in separate compositions, or sequentially. For sequential administration, the CDP-camptothecin or camptothecin derivative conjugate, particle or composition, e.g., the CDP-camptothecin or camptothecin derivative conjugate, particle or composition described herein, e.g., CRLX101, can be administered first, and the additional agent can be administered second, or the order of administration can be reversed.

In some embodiments, the CDP-camptothecin or camptothecin derivative conjugate, particle or composition, e.g., the CDP-camptothecin or camptothecin derivative conjugate, particle or composition described herein, e.g., CRLX101, is administered in combination with other therapeutic treatment modalities, including surgery, radiation, cryosurgery, and/or thermotherapy. Such combination therapies may advantageously utilize lower dosages of the administered agent and/or other chemotherapeutic agent, thus avoiding possible toxicities or complications associated with the various monotherapies. The phrase "radiation" includes, but is not limited to, external-beam therapy which involves three dimensional, conformal radiation therapy where the field of radiation is designed to conform to the volume of tissue treated; interstitial-radiation therapy where seeds of radioactive compounds are implanted using ultrasound guidance; and a combination of external-beam therapy and interstitial-radiation therapy.

In some embodiments, the CDP-camptothecin or camptothecin derivative conjugate, particle or composition, e.g., a CDP-camptothecin or camptothecin derivative conjugate, particle or composition described herein, e.g., CRLX101, is administered with at least one additional therapeutic agent, such as a chemotherapeutic agent. In certain embodiments, the CDP-camptothecin or camptothecin derivative conjugate, particle or composition, e.g., a CDP-camptothecin or camptothecin derivative conjugate, particle or composition described herein, e.g., CRLX101, is administered in combination with one or more additional chemotherapeutic agent, e.g., with one or more chemotherapeutic agents described herein. Exemplary classes of chemotherapeutic agents include, e.g., the following:
alkylating agents (including, without limitation, nitrogen mustards, ethylenimine derivatives, alkyl sulfonates, nitrosoureas and triazenes): uracil mustard (Aminouracil Mustard®, Chlorethaminacil®, Demethyldopan®, Desmethyldopan®, Haemanthamine®, Nordopan®, Uracil nitrogen mustard®, Uracillost®, Uracilmostaza®, Uramustin®, Uramustine®), chlormethine (Mustargen®), cyclophosphamide (Cytoxan®, Neosar®, Clafen®, Endoxan®, Procytox®, Revimmune™), ifosfamide (Mitoxana®), melphalan (Alkeran®), Chlorambucil (Leukeran®), pipobroman (Amedel®, Vercyte®), triethylenemelamine (Hemel®, Hexalen®, Hexastat®), triethylenethiophosphoramine, Temozolomide (Temodar®), thiotepa (Thioplex®), busulfan (Busilvex®, Myleran®), carmustine (BiCNU®), lomustine (CeeNU®), streptozocin (Zanosar®), and Dacarbazine (DTIC-Dome®).
anti-EGFR antibodies (e.g., cetuximab (Erbitux®) and panitumumab (Vectibix®).
anti-HER-2 antibodies (e.g., trastuzumab (Herceptin®).
antimetabolites (including, without limitation, folic acid antagonists (also referred to herein as antifolates), pyrimidine analogs, purine analogs and adenosine deaminase inhibitors): methotrexate (Rheumatrex®, Trexall®), 5-fluorouracil (Adrucil®, Efudex®, Fluoroplex®), floxuridine (FUDF®), cytarabine (Cytosar-U®, Tarabine PFS), 6-mercaptopurine (Puri-Nethol®)), 6-thioguanine (Thioguanine Tabloid®), fludarabine phosphate (Fludara®), pentostatin (Nipent®), pemetrexed (Alimta®), raltitrexed (Tomudex®), cladribine (Leustatin®), clofarabine (Clofarex®, Clolar®), mercaptopurine (Puri-Nethol®), capecitabine (Xeloda®), nelarabine (Arranon®), azacitidine (Vidaza®) and gemcitabine (Gemzar®). Preferred antimetabolites include, e.g., 5-fluorouracil (Adrucil®, Efudex®, Fluoroplex®), floxuridine (FUDF®), capecitabine (Xeloda®), pemetrexed (Alimta®), raltitrexed (Tomudex®) and gemcitabine (Gemzar®).
vinca alkaloids: vinblastine (Velban®, Velsar®), vincristine (Vincasar®, Oncovin®), vindesine (Eldisine®), vinorelbine (Navelbine®).
platinum-based agents: carboplatin (Paraplat®, Paraplatin®), cisplatin (Platinol®), oxaliplatin (Eloxatin®).
anthracyclines: daunorubicin (Cerubidine®, Rubidomycin®), doxorubicin (Adriamycin®), epirubicin (Ellence®), idarubicin (Idamycin®), mitoxantrone (Novantrone®), valrubicin (Valstar®). Preferred anthracyclines include daunorubicin (Cerubidine®, Rubidomycin®) and doxorubicin (Adriamycin®).
topoisomerase inhibitors: topotecan (Hycamtin®), irinotecan (Camptosar®), etoposide (Toposar®, VePesid®), teniposide (Vumon®), lamellarin D, SN-38, camptothecin.
taxanes: paclitaxel (Taxol®), docetaxel (Taxotere®), larotaxel, cabazitaxel.
epothilones: ixabepilone, epothilone B, epothilone D, BMS310705, dehydelone, ZK-Epothilone (ZK-EPO).
poly ADP-ribose polymerase (PARP) inhibitors: (e.g., BSI 201, Olaparib (AZD-2281), ABT-888, AG014699, CEP 9722, MK 4827, KU-0059436 (AZD2281), LT-673, 3-aminobenzamide).
antibiotics: actinomycin (Cosmegen®), bleomycin (Blenoxane®), hydroxyurea (Droxia®, Hydrea®), mitomycin (Mitozytrex®, Mutamycin®).
immunomodulators: lenalidomide (Revlimid®), thalidomide (Thalomid®).
immune cell antibodies: alemtuzamab (Campath®), gemtuzumab (Myelotarg®), rituximab (Rituxan®), tositumomab (Bexxar®).
interferons (e.g., IFN-alpha (Alferon®, Roferon-A®, Intron®-A) or IFN-gamma (Actimmune®)).
interleukins: IL-1, IL-2 (Proleukin®), IL-24, IL-6 (Sigosix®), IL-12.
HSP90 inhibitors (e.g., geldanamycin or any of its derivatives). In certain embodiments, the HSP90 inhibitor is selected from geldanamycin, 17-alkylamino-17-desmethoxygeldanamycin ("17-AAG") or 17-(2-dimethylaminoethyl)amino-17-desmethoxygeldanamycin ("17-DMAG").
angiogenesis inhibitors which include, without limitation A6 (Angstrom Pharmacueticals), ABT-510 (Abbott Laboratories), ABT-627 (Atrasentan) (Abbott Laboratories/Xinlay), ABT-869 (Abbott Laboratories), Actimid (CC4047, Pomalidomide) (Celgene Corporation), AdGVPEDF.11D (GenVec), ADH-1 (Exherin) (Adherex Technologies), AEE788 (Novartis), AG-013736 (Axitinib) (Pfizer), AG3340 (Prinomastat) (Agouron Pharmaceuticals), AGX1053 (AngioGenex), AGX51 (AngioGenex), ALN-VSP (ALN-VSP O2) (Alnylam Pharmaceuticals), AMG 386 (Amgen), AMG706 (Amgen), Apatinib (YN968D1) (Jiangsu Hengrui Medicine), AP23573 (Ridaforolimus/MK8669) (Ariad Pharmaceuticals), AQ4N (Novavea), ARQ 197 (ArQule), ASA404 (Novartis/Antisoma), Atiprimod (Callisto Pharmaceuticals), ATN-161 (Attenuon), AV-412 (Aveo Pharmaceuticals), AV-951 (Aveo Pharmaceuticals), Avastin (Bevacizumab) (Genentech), AZD2171 (Cediranib/Recentin) (AstraZeneca), BAY 57-9352 (Telatinib) (Bayer), BEZ235 (Novartis), BIBF1120 (Boehringer Ingelheim Pharmaceuticals), BIBW 2992 (Boehringer Ingelheim Pharmaceuticals), BMS-275291 (Bristol-Myers Squibb), BMS-582664 (Brivanib) (Bristol-Myers Squibb), BMS-690514 (Bristol-Myers Squibb), Calcitriol, CCI-779 (Torisel) (Wyeth), CDP-791 (ImClone Systems), Ceflatonin (Homoharringtonine/HHT) (ChemGenex Therapeutics), Celebrex (Celecoxib) (Pfizer), CEP-7055 (Cephalon/Sanofi), CHIR-265 (Chiron Corporation), NGR-TNF, COL-3 (Metastat) (Collagenex Pharaceuticals), Combretastatin (Oxigene), CP-751,871(Figitumumab) (Pfizer), CP-547,632 (Pfizer), CS-7017 (Daiichi Sankyo Pharma), CT-322 (Angiocept) (Adnexus), Curcumin, Dalteparin (Fragmin) (Pfizer), Disulfiram (Antabuse), E7820 (Eisai Limited), E7080 (Eisai Limited), EMD 121974(Cilengitide) (EMD Pharmaceuticals), ENMD-1198 (EntreMed), ENMD-2076 (EntreMed), Endostar (Simcere), Erbitux (ImClone/Bristol-Myers Squibb), EZN-2208 (Enzon Pharmaceuticals), EZN-2968 (Enzon Pharmaceuticals), GC1008 (Genzyme), Genistein, GSK1363089(Foretinib) (GlaxoSmithKline), GW786034 (Pazopanib) (GlaxoSmithKline), GT-111 (Vascular Biogenics Ltd.), IMC--1121B (Ramucirumab) (ImClone Systems), IMC-18F1 (ImClone Systems), IMC-3G3 (ImClone LLC), INCB007839 (Incyte Corporation), INGN 241 (Introgen Therapeutics), Iressa (ZD1839/Gefitinib), LBH589 (Faridak/Panobinostst) (Novartis), Lucentis (Ranibizumab) (Genentech/Novartis), LY317615 (Enzastaurin) (Eli Lilly and Company), Macugen (Pegaptanib) (Pfizer), MEDI522 (Abegrin) (MedImmune), MLN518(Tandutinib) (Millennium), Neovastat (AE941/Benefin) (Aeterna Zentaris), Nexavar (Bayer/Onyx), NM-3 (Genzyme Corporation), Noscapine (Cougar Biotechnology), NPI-2358 (Nereus Pharmaceuticals), OSI-930 (OSI), Palomid 529 (Paloma Pharmaceuticals, Inc.), Panzem Capsules (2ME2) (EntreMed), Panzem NCD (2ME2) (EntreMed), PF-02341066 (Pfizer), PF-04554878 (Pfizer), PI-88 (Progen Industries/Medigen Biotechnology), PKC412 (Novartis), Polyphenon E (Green Tea Extract) (Polypheno E International, Inc), PPI-2458 (Praecis Pharmaceuticals), PTC299 (PTC Therapeutics), PTK787 (Vatalanib) (Novartis), PXD101 (Belinostat) (CuraGen Corporation), RAD001 (Everolimus) (Novartis), RAF265 (Novartis), Regorafenib (BAY73-4506) (Bayer), Revlimid (Celgene), Retaane (Alcon Research), SN38 (Liposomal) (Neopharm), SNS-032 (BMS-387032) (Sunesis), SOM230(Pasireotide) (Novartis), Squalamine (Genaera), Suramin, Sutent (Pfizer), Tarceva (Genentech), TB-403 (Thrombogenics), Tempostatin (Collard Biopharmaceuticals), Tetrathiomolybdate (Sigma-Aldrich), TG100801 (TargeGen), Thalidomide (Celgene Corporation), Tinzaparin Sodium, TKI258 (Novartis), TRC093 (Tracon Pharmaceuticals Inc.), VEGF Trap (Aflibercept) (Regeneron Pharmaceuticals), VEGF Trap-Eye (Regeneron Pharmaceuticals), Veglin (VasGene Therapeutics), Bortezomib (Millennium), XL184 (Exelixis), XL647 (Exelixis), XL784 (Exelixis), XL820 (Exelixis), XL999 (Exelixis), ZD6474 (AstraZeneca), Vorinostat (Merck), and ZSTK474.
anti-androgens which include, without limitation nilutamide (Nilandron®) and bicalutamide (Caxodex®).
antiestrogens which include, without limitation tamoxifen (Nolvadex®), toremifene (Fareston®), letrozole (Femara®), testolactone (Teslac®), anastrozole (Arimidex®), bicalutamide (Casodex®), exemestane (Aromasin®), flutamide (Eulexin®), fulvestrant (Faslodex®), raloxifene (Evista®, Keoxifene®) and raloxifene hydrochloride.
anti-hypercalcaemia agents which include without limitation gallium (III) nitrate hydrate (Ganite®) and pamidronate disodium (Aredia®).
apoptosis inducers which include without limitation ethanol, 2-[[3-(2,3-dichlorophenoxy)propyl]amino]-(9Cl), gambogic acid, embelin and arsenic trioxide (Trisenox®).
Aurora kinase inhibitors which include without limitation binucleine 2.
Bruton's tyrosine kinase inhibitors which include without limitation terreic acid.
calcineurin inhibitors which include without limitation cypermethrin, deltamethrin, fenvalerate and tyrphostin 8.
CaM kinase II inhibitors which include without limitation 5-Isoquinolinesulfonic acid, 4-[{2S)-2-[(5-isoquinolinylsulfonyl)methylamino]-3-oxo-3-{4-phenyl-1-piperazinyl)propyl]phenyl ester and benzenesulfonamide.
CD45 tyrosine phosphatase inhibitors which include without limitation phosphonic acid.
CDC25 phosphatase inhibitors which include without limitation 1,4-naphthalene dione, 2,3-bis[(2-hydroxyethyl)thio]-(9Cl).
CHK kinase inhibitors which include without limitation debromohymenialdisine.
cyclooxygenase inhibitors which include without limitation 1H-indole-3-acetamide, 1-(4-chlorobenzoyl)-5-methoxy-2-methyl-N-(2-phenylethyl)-(9Cl), 5-alkyl substituted 2-arylaminophenylacetic acid and its derivatives (e.g., celecoxib (Celebrex®), rofecoxib (Vioxx®), etoricoxib (Arcoxia®), lumiracoxib (Prexige®), valdecoxib (Bextra®) or 5-alkyl-2-arylaminophenylacetic acid).
cRAF kinase inhibitors which include without limitation 3-(3,5-dibromo-4-hydroxybenzylidene)-5-iodo-1,3-dihydroindol-2-one and benzamide, 3-(dimethylamino)-N-[3-[(4-hydroxybenzoyl)amino]-4-methylphenyl]-(9Cl).
cyclin dependent kinase inhibitors which include without limitation olomoucine and its derivatives, purvalanol B, roascovitine (Seliciclib®), indirubin, kenpaullone, purvalanol A and indirubin-3'-monooxime.
cysteine protease inhibitors which include without limitation 4-morpholinecarboxamide, N-[(1S)-3-fluoro-2-oxo-1-(2-phenylethyl)propyl]amino]-2-oxo-1-(phenylmethyl)ethyl]-(9Cl).
DNA intercalators which include without limitation plicamycin (Mithracin®) and daptomycin (Cubicin®).
DNA strand breakers which include without limitation bleomycin (Blenoxane®).
E3 ligase inhibitors which include without limitation N-((3,3,3-trifluoro-2-trifluoromethyl)propionyl) sulfanilamide.
EGF Pathway Inhibitors which include, without limitation tyrphostin 46, EKB-569, erlotinib (Tarceva®), gefitinib (Iressa®), lapatinib (Tykerb®) and those compounds that are generically and specifically disclosed in WO 97/02266, EP 0 564 409, WO 99/03854, EP 0 520 722, EP 0 566 226, EP 0 787 722, EP 0 837 063, US 5,747,498, WO 98/10767, WO 97/30034, WO 97/49688, WO 97/38983 and WO 96/33980.
farnesyltransferase inhibitors which include without limitation A-hydroxyfarnesylphosphonic acid, butanoic acid, 2-[(2S)-2-[[(2S,3S)-2-[[(2R)-2-amino-3-mercaptopropyl]amino]-3-methylpentyl]oxy]-1-oxo-3-phenylpropyl]amino]-4-(methylsulfonyl)-1-methylethylester (2S)-(9Cl), and manumycin A.
Flk-1 kinase inhibitors which include without limitation 2-propenamide, 2-cyano-3-[4-hydroxy-3,5-bis(1-methylethyl)phenyl]-N-(3-phenylpropyl)-(2E)-(9Cl).
glycogen synthase kinase-3 (GSK3) inhibitors which include without limitation indirubin-3' -monooxime.
histone deacetylase (HDAC) inhibitors which include without limitation suberoylanilide hydroxamic acid (SAHA), [4-(2-amino-phenylcarbamoyl)-benzyl]-carbamic acid pyridine-3-ylmethylester and its derivatives, butyric acid, pyroxamide, trichostatin A, oxamflatin, apicidin, depsipeptide, depudecin, trapoxin and compounds disclosed in WO 02/22577.
I-kappa B-alpha kinase inhibitors (IKK) which include without limitation 2-propenenitrile, 3-[(4-methylphenyl)sulfonyl]-(2E)-(9Cl).
imidazotetrazinones which include without limitation temozolomide (Methazolastone®, Temodar® and its derivatives (e.g., as disclosed generically and specifically in US 5,260,291) and Mitozolomide.
insulin tyrosine kinase inhibitors which include without limitation hydroxyl-2-naphthalenylmethylphosphonic acid.
c-Jun-N-terminal kinase (JNK) inhibitors which include without limitation pyrazoleanthrone and epigallocatechin gallate.
mitogen-activated protein kinase (MAP) inhibitors which include without limitation benzenesulfonamide, N-[2-[[[3-(4-chlorophenyl)-2-propenyl]methyl]amino]methyl]phenyl]-N-(2-hydroxyethyl)-4-methoxy-(9Cl).
MDM2 inhibitors which include without limitation trans-4-iodo, 4'-boranyl-chalcone.
MEK inhibitors which include without limitation butanedinitrile, bis[amino[2-aminophenyl)thio]methylene]-(9Cl).
MMP inhibitors which include without limitation Actinonin, epigallocatechin gallate, collagen peptidomimetic and non-peptidomimetic inhibitors, tetracycline derivatives marimastat (Marimastat®), prinomastat, incyclinide (Metastat®), shark cartilage extract AE-941 (Neovastat®), Tanomastat, TAA211, MMI270B or AAJ996.
mTor inhibitors which include without limitation rapamycin (Rapamune®), and analogs and derivatives thereof, AP23573 (also known as ridaforolimus, deforolimus, or MK-8669), CCI-779 (also known as temsirolimus) (Torisel®) and SDZ-RAD.
NGFR tyrosine kinase inhibitors which include without limitation tyrphostin AG 879.
p38 MAP kinase inhibitors which include without limitation Phenol, 4-[4-(4-fluorophenyl)-5-(4-pyridinyl)-1H-imidazol-2-yl]-(9Cl), and benzamide, 3-(dimethylamino)-N-[3-[(4-hydroxylbenzoyl)amino]-4-methylphenyl]-(9Cl).
p56 tyrosine kinase inhibitors which include without limitation damnacanthal and tyrphostin 46.
PDGF pathway inhibitors which include without limitation tyrphostin AG 1296, tyrphostin 9, 1,3-butadiene-1,1,3-tricarbonitrile, 2-amino-4-(1H-indol-5-yl)-(9Cl), imatinib (Gleevec®) and gefitinib (Iressa®) and those compounds generically and specifically disclosed in European Patent No.: 0 564 409 and PCT Publication No.: WO 99/03854.
phosphatidylinositol 3-kinase inhibitors which include without limitation wortmannin, and quercetin dihydrate.
phosphatase inhibitors which include without limitation cantharidic acid, cantharidin, and L-leucinamide.
protein phosphatase inhibitors which include without limitation cantharidic acid, cantharidin, L-P-bromotetramisole oxalate, 2(5H)-furanone, 4-hydroxy-5-(hydroxymethyl)-3-(1-oxohexadecyl)-(5R)-(9Cl) and benzylphosphonic acid.
PKC inhibitors which include without limitation 1-H-pyrollo-2,5-dione,3-[1-[3-(dimethylamino)propyl]-1H-indol-3-yl]-4-(1H-indol-3-yl)-(9Cl), Bisindolylmaleimide IX, Sphinogosine, staurosporine, and Hypericin.
PKC delta kinase inhibitors which include without limitation rottlerin.
polyamine synthesis inhibitors which include without limitation DMFO.
proteasome inhibitors which include, without limitation aclacinomycin A, gliotoxin and bortezomib (Velcade®).
PTP1B inhibitors which include without limitation L-leucinamide.
protein tyrosine kinase inhibitors which include, without limitation tyrphostin Ag 216, tyrphostin Ag 1288, tyrphostin Ag 1295, geldanamycin, genistein and 7H-pyrollo[2,3-d]pyrimidine derivatives of formula I as generically and specifically described in PCT Publication No.: WO 03/013541 and U.S. Publication No.: 2008/0139587: 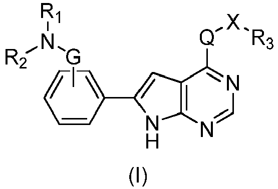
Publication No.: 2008/0139587 discloses the various substituents, e.g., R₁, R₂, etc.
SRC family tyrosine kinase inhibitors which include without limitation PP1 and PP2.
Syk tyrosine kinase inhibitors which include without limitation piceatannol.
Janus (JAK-2 and/or JAK-3) tyrosine kinase inhibitors which include without limitation tyrphostin AG 490 and 2-naphthyl vinyl ketone.
retinoids which include without limitation isotretinoin (Accutane®, Amnesteem®, Cistane®, Claravis®, Sotret®) and tretinoin (Aberel®, Aknoten®, Avita®, Renova®, Retin-A®, Retin-A MICRO®, Vesanoid®).
RNA polymerase II elongation inhibitors which include without limitation 5,6-dichloro-1-beta-D-ribofuranosylbenzimidazole.
serine/threonine kinase inhibitors which include without limitation 2-aminopurine.
sterol biosynthesis inhibitors which include without limitation squalene epoxidase and CYP2D6.
VEGF pathway inhibitors which include without limitation anti-VEGF antibodies, e.g., bevacizumab, and small molecules, e.g., sunitinib (Sutent®), sorafinib (Nexavar®), ZD6474 (also known as vandetanib) (Zactima™), SU6668, CP-547632, AV-951 (tivozanib) and AZD2171 (also known as cediranib) (Recentin™).

Examples of chemotherapeutic agents are also described in the scientific and patent literature, see, e.g., Bulinski (1997) J. Cell Sci. 110:3055-3064; Panda (1997) Proc. Natl. Acad. Sci. USA 94:10560-10564; Muhlradt (1997) Cancer Res. 57:3344-3346; Nicolaou (1997) Nature 387:268-272; Vasquez (1997) Mol. Biol. Cell. 8:973-985; Panda (1996) J. Biol. Chem 271:29807-29812.

In some embodiment, the CDP-camptothecin or camptothecin derivative conjugate, particle or composition, e.g., a CDP-camptothecin or camptothecin derivative conjugate, particle or composition described herein, e.g., CRLX101, is administered as a first line therapy or a second line therapy. For example, the CDP-camptothecin or camptothecin derivative conjugate, particle or composition, e.g., a CDP-camptothecin or camptothecin derivative conjugate, particle or composition described herein, e.g., CRLX101, can be used instead of any of the following topoisomerase inhibitors: a topoisomerase I inhibitor, e.g., camptothecin, irinotecan, SN-38, topotecan, lamellarin D; a topoisomerase II inhibitor, e.g., etoposide, tenoposide, doxorubicin.

In some cases, a hormone and/or steriod can be administered in combination with a CDP-camptothecin or camptothecin derivative conjugate, particle or composition, e.g., a CDP-camptothecin or camptothecin derivative conjugate, particle or composition described herein, e.g., CRLX101. Examples of hormones and steroids include: 17a-ethinylestradiol (Estinyl®, Ethinoral®, Feminone®, Orestralyn®), diethylstilbestrol (Acnestrol®, Cyren A®, Deladumone®, Diastyl®, Domestrol®, Estrobene®, Estrobene®, Estrosyn®, Fonatol®, Makarol®, Milestrol®, Milestrol®, Neo-Oestronol I®, Oestrogenine®, Oestromenin®, Oestromon®, Palestrol®, Stilbestrol®, Stilbetin®, Stilboestroform®, Stilboestrol®, Synestrin®, Synthoestrin®, Vagestrol®), testosterone (Delatestryl®, Testoderm®, Testolin®, Testostroval®, Testostroval-PA®, Testro AQ®), prednisone (Delta-Dome®, Deltasone®, Liquid Pred®, Lisacort®, Meticorten®, Orasone®, Prednicen-M®, Sk-Prednisone®, Sterapred®), Fluoxymesterone (Android-F®, Halodrin®, Halotestin®, Ora-Testryl®, Ultandren®), dromostanolone propionate (Drolban®, Emdisterone®, Masterid®, Masteril®, Masteron®, Masterone®, Metholone®, Permastril®), testolactone (Teslac®), megestrolacetate (Magestin®, Maygace®, Megace®, Megeron®, Megestat®, Megestil®, Megestin®, Nia®, Niagestin®, Ovaban®, Ovarid®, Volidan®), methylprednisolone (Depo-Medrol®, Medlone 21®, Medrol®, Meprolone®, Metrocort®, Metypred®, Solu-Medrol®, Summicort®), methyl-testosterone (Android®, Testred®, Virilon®), prednisolone (Cortalone®, Delta-Cortef®, Hydeltra®, Hydeltrasol®, Meti-derm®, Prelone®), triamcinolone (Aristocort®), chlorotrianisene (Anisene®, Chlorotrisin®, Clorestrolo®, Clorotrisin®, Hormonisene®, Khlortrianizen®, Merbentul®, Metace®, Rianil®, Tace®, Tace-Fn®, Trianisestrol®), hydroxyprogesterone (Delalutin®, Gestiva™), aminoglutethimide (Cytadren®, Elipten®, Orimeten®), estramustine (Emcyt®), medroxyprogesteroneacetate (Provera®, Depo-Provera®), leuprolide (Lupron®, Viadur®), flutamide (Eulexin®), toremifene (Fareston®), and goserelin (Zoladex®).

In certain embodiments, the CDP-camptothecin or camptothecin derivative conjugate, particle or composition, e.g., a CDP-camptothecin or camptothecin derivative conjugate, particle or composition described herein, e.g., CRLX101, is administered in combination with an anti-microbial (e.g., leptomycin B).

In another embodiment, the CDP-camptothecin or camptothecin derivative conjugate, particle or composition, e.g., a CDP-camptothecin or camptothecin derivative conjugate, particle or composition described herein, e.g., CRLX101, is administered in combination with an agent or procedure to mitigate potential side effects from the agent compositions such as cystitis, hypersensitivity, diarrhea, nausea and vomiting.

Cystitis can be mitigated with an agent that increases urinary excretion and/or neutralizes one or more urinary metabolite. For example, cystitis can be mitigated or treated with MESNA.

Diarrhea may be treated with antidiarrheal agents including, but not limited to opioids (e.g., codeine (Codicept®, Coducept®), oxicodeine, percocet, paregoric, tincture of opium, diphenoxylate (Lomotil®), diflenoxin), and loperamide (Imodium A-D®), bismuth subsalicylate, lanreotide, vapreotide (Sanvar®, Sanvar IR®), motiln antagonists, COX2 inhibitors (e.g., celecoxib (Celebrex®), glutamine (NutreStore®), thalidomide (Synovir®, Thalomid®), traditional antidiarrhea remedies (e.g., kaolin, pectin, berberine and muscarinic agents), octreotide and DPP-IV inhibitors.

DPP-IV inhibitors employed in the present invention are generically and specifically disclosed in PCT Publication Nos.: WO 98/19998, DE 196 16 486 A1, WO 00/34241 and WO 95/15309.

Nausea and vomiting may be treated with antiemetic agents such as dexamethasone (Aeroseb-Dex®, Alba-Dex®, Decaderm®, Decadrol®, Decadron®, Decasone®, Decaspray®, Deenar®, Deronil®, Dex-4®, Dexace®, Dexameth®, Dezone®, Gammacorten®, Hexadrol®, Maxidex®, Sk-Dexamethasone®), metoclopramide (Reglan®), diphenylhydramine (Benadryl®, SK-Diphenhydramine®), lorazepam (Ativan®), ondansetron (Zofran®), prochlorperazine (Bayer A 173®, Buccastem®, Capazine®, Combid®, Compazine®, Compro®, Emelent®, Emetiral®, Eskatrol®, Kronocin®, Meterazin®, Meterazin Maleate®, Meterazine®, Nipodal®, Novamin®, Pasotomin®, Phenotil®, Stemetil®, Stemzine®, Tementil®, Temetid®, Vertigon®), thiethylperazine (Norzine®, Torecan®), and dronabinol (Marinol®).

In some embodiments, the CDP-camptothecin or camptothecin derivative conjugate, particle or composition, e.g., the CDP-camptothecin or camptothecin derivative conjugate, particle or composition described herein, e.g., CRLX101, is administered in combination with an immunosuppressive agent. Immunosuppressive agents suitable for the combination include, but are not limited to natalizumab (Tysabri®), azathioprine (Imuran®), mitoxantrone (Novantrone®), mycophenolate mofetil (Cellcept®), cyclosporins (e.g., Cyclosporin A (Neoral®, Sandimmun®, Sandimmune®, SangCya®), cacineurin inhibitors (e.g., Tacrolimus (Prograf®, Protopic®), sirolimus (Rapamune®), everolimus (Afinitor®), cyclophosphamide (Clafen®, Cytoxan®, Neosar®), or methotrexate (Abitrexate®, Folex®, Methotrexate®, Mexate®)), fingolimod, mycophenolate mofetil (CellCept®), mycophenolic acid (Myfortic®), anti-CD3 antibody, anti-CD25 antibody (e.g., Basiliximab (Simulect®) or daclizumab (Zenapax®)), and anti-TNFa antibody (e.g., Infliximab (Remicade®) or adalimumab (Humira®)).

In some embodiments, a CDP-camptothecin or camptothecin derivative conjugate, particle or composition, e.g., a CDP-camptothecin or camptothecin derivative conjugate, particle or composition described herein, e.g., CRLX101, is administered in combination with a CYP3A4 inhibitor (e.g., ketoconazole (Nizoral®, Xolegel®), itraconazole (Sporanox®), clarithromycin (Biaxin®), atazanavir (Reyataz®), nefazodone (Serzone®, Nefadar®), saquinavir (Invirase®), telithromycin (Ketek®), ritonavir (Norvir®), amprenavir (also known as Agenerase, a prodrug version is fosamprenavir (Lexiva®, Telzir®), indinavir (Crixivan®), nelfinavir (Viracept®), delavirdine (Rescriptor®) or voriconazole (Vfend®)).

When employing the methods or compositions, other agents used in the modulation of tumor growth or metastasis in a clinical setting, such as antiemetics, can also be administered as desired.

When formulating the pharmaceutical compositions featured in the invention the clinician may utilize preferred dosages as warranted by the condition of the subject being treated. For example, in one embodiment, a CDP-camptothecin or camptothecin derivative conjugate, particle or composition, e.g., the CDP-camptothecin or camptothecin derivative conjugate, particle or composition described herein, e.g., CRLX101, may be administered at a dosing schedule described herein, e.g., once every one, two, three or four weeks.

Also, in general, a CDP-camptothecin or camptothecin derivative conjugate, particle or composition, e.g., the CDP-camptothecin or camptothecin derivative conjugate, particle or composition described herein, e.g., CRLX101, and an additional chemotherapeutic agent(s) do not have to be administered in the same pharmaceutical composition, and may, because of different physical and chemical characteristics, have to be administered by different routes. For example, the CDP-camptothecin or camptothecin derivative conjugate, particle or composition, e.g., a CDP-camptothecin or camptothecin derivative conjugate, particle or composition described herein, e.g., CRLX101, may be administered intravenously while the chemotherapeutic agent(s) may be administered orally. The determination of the mode of administration and the advisability of administration, where possible, in the same pharmaceutical composition, is well within the knowledge of the skilled clinician. The initial administration can be made according to established protocols known in the art, and then, based upon the observed effects, the dosage, modes of administration and times of administration can be modified by the skilled clinician.

The actual dosage of the CDP-camptothecin or camptothecin derivative conjugate, particle or composition and/or any additional chemotherapeutic agent employed may be varied depending upon the requirements of the subject and the severity of the condition being treated. Determination of the proper dosage for a particular situation is within the skill of the art. Generally, treatment is initiated with smaller dosages which are less than the optimum dose of the compound. Thereafter, the dosage is increased by small amounts until the optimum effect under the circumstances is reached.

In some embodiments, when a CDP- camptothecin or camptothecin derivative conjugate, particle or composition is administered in combination with one or more additional chemotherapeutic agent, the additional chemotherapeutic agent (or agents) is administered at a standard dose. For example, a standard dosage for cisplatin is 75-120 mg/m² administered every three weeks; a standard dosage for carboplatin is within the range of 200-600 mg/m² or an AUC of 0.5-8 mg/ml x min; e.g., at an AUC of 4-6 mg/ml x min; a standard dosage for irinotecan is within 100-125 mg/m², once a week; a standard dosage for gemcitabine is within the range of 80-1500 mg/m² administered weekly; a standard dose for UFT is within a range of 300-400 mg/m² per day when combined with leucovorin administration; a standard dosage for leucovorin is 10-600 mg/m² administered weekly.

The disclosure also encompasses a method for the synergistic treatment of cancer wherein a CDP-camptothecin or camptothecin derivative conjugate, particle or composition, e.g., the CDP-camptothecin or camptothecin derivative conjugate, particle or composition described herein, e.g., CRLX101, is administered in combination with an additional chemotherapeutic agent or agents.

The particular choice of conjugate, particle or composition and anti-proliferative cytotoxic agent(s) or radiation will depend upon the diagnosis of the attending physicians and their judgment of the condition of the subject and the appropriate treatment protocol.

If the CDP-camptothecin or camptothecin derivative conjugate, particle or composition, e.g., a CDP-camptothecin or camptothecin derivative conjugate, particle or composition described herein, e.g., CRLX101, and the chemotherapeutic agent(s) and/or radiation are not administered simultaneously or essentially simultaneously, then the initial order of administration of the CDP-camptothecin or camptothecin derivative conjugate, particle or composition, e.g., a CDP-camptothecin or camptothecin derivative conjugate, particle or composition described herein, e.g., CRLX101, and the chemotherapeutic agent(s) and/or radiation, may be varied. Thus, for example, the CDP-camptothecin or camptothecin derivative conjugate, particle or composition, e.g., a CDP-camptothecin or camptothecin derivative conjugate, particle or composition described herein, e.g., CRLX101, may be administered first followed by the administration of the chemotherapeutic agent(s) and/or radiation; or the chemotherapeutic agent(s) and/or radiation may be administered first followed by the administration of the CDP-camptothecin or camptothecin derivative conjugate, particle or composition, e.g., a CDP-camptothecin or camptothecin derivative conjugate, particle or composition described herein, e.g., CRLX101. This alternate administration may be repeated during a single treatment protocol. The determination of the order of administration, and the number of repetitions of administration of each therapeutic agent during a treatment protocol, is well within the knowledge of the skilled physician after evaluation of the disease being treated and the condition of the subject.

Thus, in accordance with experience and knowledge, the practicing physician can modify each protocol for the administration of a component (CDP-camptothecin or camptothecin derivative conjugate, particle or composition, e.g., the CDP-camptothecin or camptothecin derivative conjugate, particle or composition described herein, e.g., CRLX101, anti-neoplastic agent(s), or radiation) of the treatment according to the individual subject's needs, as the treatment proceeds.

The attending clinician, in judging whether treatment is effective at the dosage administered, will consider the general well-being of the subject as well as more definite signs such as relief of disease-related symptoms, inhibition of tumor growth, actual shrinkage of the tumor, or inhibition of metastasis. Size of the tumor can be measured by standard methods such as radiological studies, e.g., CAT or MRI scan, and successive measurements can be used to judge whether or not growth of the tumor has been retarded or even reversed. Relief of disease-related symptoms such as pain, and improvement in overall condition can also be used to help judge effectiveness of treatment.

### Indications

The disclosed CDP-camptothecin or camptothecin derivative conjugate, particle or composition, e.g., a CDP-camptothecin or camptothecin derivative conjugate, particle or composition described herein, e.g., CRLX101, are useful in treating proliferative disorders, e.g., treating a tumor, e.g., a primary tumor, and/or metastases thereof, wherein the tumor is a primary tumor or a metastases thereof, e.g., a cancer described herein or a metastases of a cancer described herein.

The methods described herein can be used to treat a solid tumor, a soft tissue tumor or a liquid tumor. Exemplary solid tumors include malignancies (e.g., sarcomas and carcinomas (e.g., adenocarcinoma or squamous cell carcinoma)) of the various organ systems, such as those of the gastrointestinal (e.g., colorectal, e.g., rectal). Exemplary adenocarcinomas include colorectal cancers, renal-cell carcinoma, liver cancer, non-small cell carcinoma of the lung, and cancer of the small intestine. The disclosed methods are also useful in evaluating or treating soft tissue tumors such as those of the tendons, muscles or fat, and liquid tumors.

The methods described herein can be used with a cancer, e.g. colorectal cancer, e.g., rectal cancer, for example those described by the National Cancer Institute. Exemplary cancers, e.g. colorectal cancers, e.g., rectal cancers described by the National Cancer Institute include:
Digestive/gastrointestinal cancers such as anal cancer; bile duct cancer; extrahepatic bile duct cancer; appendix cancer; carcinoid tumor, gastrointestinal cancer; colon cancer; colorectal cancer including childhood colorectal cancer; rectal cancer; and small intestine cancer;
In some embodiments, the cancer, e.g. colorectal cancer, e.g., rectal cancer, is an adenocarcinoma. Examples include leiomyosarcoma and neuroendocrine tumors.

Unless otherwise defined, all technical and scientific terms used herein have the same meaning as commonly understood by one of ordinary skill in the art to which this invention belongs. All publications, patent applications, patents, and other references mentioned herein are incorporated by reference in their entirety. In case of conflict, the present specification, including definitions, will control. In addition, the materials, methods, and examples are illustrative only and not intended to be limiting.

### EXAMPLES

### Example 1: CRLX101 with Radiation Therapy in a Head and Neck Xenograft Tumor Model

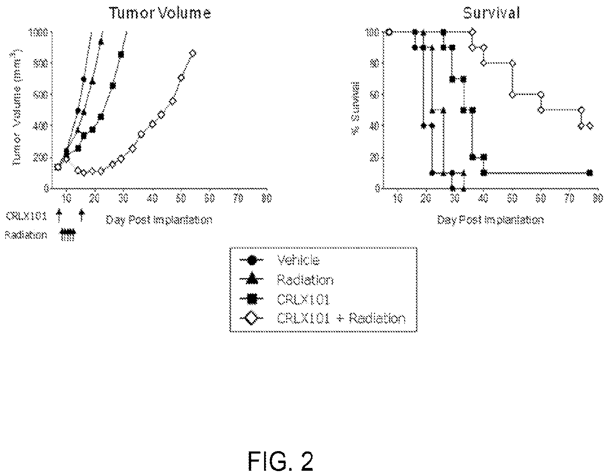

FaDu tumor-bearing mice were treated IV for 2 weeks with CRLX101 (2 mg/kg q7d x 2) and/or X-ray radiation daily (2 Gy qd x 5) at the times indicated. As shown in Figure 2, CRLX101 showed synergy with radiation therapy with respect to tumor growth inhibition and survival. Synergy was observed whether the X-ray radiation was delivered 1 day after the first dose of CRLX101 or 1 day after the second dose of CRLX101.

### Example 2: Human Phase Ib/II Study of CRLX101

The below example describes a human phase Ib and phase II study of CRLX101, a nanopharmaceutical (NP) containing camptothecin, added to neoadjuvant chemoradiotherapy (capecitabine and radiotherapy) in locally advanced rectal cancer. This open label, single-arm multicenter Phase Ib/II study is designed to identify the maximum tolerated dose (MTD) using a traditional 3+3 dose escalation design. In this trial, the MTD will be assigned as the recommended Phase II dose (RP2D). In Phase II, the efficacy of the RP2D will be evaluated and the safety of CRLX101 when combined with capecitabine and radiation therapy prior to surgery in patients with locally advanced rectal carcinoma will be further characterized. Patients in the Phase Ib study with resectable disease and treated at the MTD/RP2D will be included in the Phase II study population for primary endpoint analysis.

Patients in the Phase II trial will be evaluated using a Simon two-stage design, with a primary endpoint of pathological complete response (pCR). Secondary endpoints include pathological response, disease free survival (DFS) and overall survival (OS).

CRLX101 administration in combination with radiation and administration of capecitabine should improve the rate of pCR as compared to historical controls. Current neoadjuvant chemoradiotherapy (CRT) for rectal cancer incorporates a fluoropyrimidine (such as capecitabine) concurrent with radiation. Several large randomized trials have demonstrated that approximately 15-25% of the patients who receive these regimens will achieve pCR after treatment. If the regimen described herein results in a pCR rate of at least 35%, further studies of this combination will be performed.

### Primary Objective Phase Ib

To estimate the MTD (which will be assigned as the RP2D) of CRLX101 when added to standard neoadjuvant chemoradiotherapy consisting of capecitabine and radiotherapy in locally advanced rectal cancer

### Primary Objective Phase II

To estimate the rate of pCR of CRLX101 dosed at its RP2D when combined with capecitabine and radiotherapy in locally advanced rectal cancer

### Secondary Objectives (Phase Ib and Phase II)

To estimate the pathological response rate of CRLX101 in resectable patients when combined with standard neoadjuvant chemoratiotherapy consisting of capecitabine and radiotherapy in locally advanced rectal cancer
To characterize the toxicity and safety profile of CRLX101 when combined with capecitabine and radiotherapy to treat patients with locally advanced rectal cancer

### Secondary Objectives (Phase II only)

To estimate disease-free survival (DFS) of locally advanced rectal cancer patients after receiving neoadjuvant treatment with CRLX101 combined with capecitabine and radiotherapy followed by surgery
To estimate the overall survival (OS) of locally advanced rectal cancer patients receiving neoadjuvant treatment with CRLX101 when combined with capecitabine and radiotherapy followed by surgery
To compare 5 year DFS (and OS) between locally advanced rectal cancer patients who achieve pCR and those who do not post neoadjuvant CRLX101, capecitabine and radiotherapy followed by surgery

### Primary Endpoints

The MTD (RP2D) will be based on the rate of dose-limiting toxicity (DLT); toxicities will be assessed via NCI's CTCAE v4.0 toxicity criteria
Pathological response will be made based on assessment of the surgical specimen at the primary treatment site. A pCR must include no gross or microscopic tumor identified anywhere within the surgical specimen. This must include:
- No evidence of malignant cells in the primary tumor specimen and
- No lymph nodes that contain tumor.

### Secondary Endpoints

For resectable patients who do not meet criteria for a pCR, the extent of response to preoperative therapy will be graded using the Tumor Regression Grade (TRG) schema (see American Joint Committee on Cancer (AJCC) 7^{th} edition)
DFS will be defined as the time from surgical resection date until disease recurrence or death as a result of any cause
OS is defined as the time from surgical resection date until death
Safety will be the reported adverse event (AE) profile characterized by NCI CTCAE v4.0.

### Patient Eligibility

### Inclusion Criteria

Patients will need to meet all of the following inclusion criteria to participate in this study:
ECOG performance score ≤ 2
Phase Ib and II: surgical candidates, with moderate to high-risk pathologically-confirmed rectal cancer (stage cT3-4N0 or cT1-4N+); clinical staging by endoscopic ultrasound (EUS) or magnetic resonance imaging (MRI) is permitted.

### Phase Ib only:

Patients with metastatic rectal cancer are allowed if their primary site meets other eligibility criteria and chemoradiotherapy is recommended as initial therapy for symptom palliation by the multidisciplinary treating team, and no other concurrent chemotherapy is planned for the primary site
Patients with locally advanced unresectable cancers are allowed provided:
There is no evidence of recto-vaginal, recto-vesicular, recto-intestinal fisutalization
Standard dose and schedule chemoradiotherapy is recommended as initial therapy by the multidisciplinary treating team
Age ≥18 years old
Women of childbearing potential (WOCBP) must have negative pregnancy test within 7 days prior to D1 of treatment
Recommendation to undergo concurrent chemoradiation, as determined by the treating physician
Ability to swallow oral medications
As determined by the enrolling physician or protocol designee, ability of the patient to understand and comply with study procedures for the entire length of the study
Informed consent reviewed and signed

### Exclusion Criteria

Patients meeting any of the following exclusion criteria will not be able to participate in this study:
Grade 2 or higher diarrhea within 14 days of the first dose unless deemed by the investigator to be caused by laxatives prescribed for symptomatic partial obstruction (e.g. MiraLAX®)
Not deemed a candidate for concurrent chemoradiation for medical reasons, such as uncontrolled infection (including HIV), uncontrolled diabetes mellitus or cardiac disease which, in the opinion of the treating physician, would make this protocol unreasonably hazardous for the patient.

Specific laboratory exclusion values, including:
Hemoglobin ≤ 10.0 g/dL for males and ≤ 9.0 g/dL for females (transfusion allowed to achieve or maintain levels)
ANC < 1,500/mm³
Platelet count < 100,000/mm³
ALT and AST ≥ 2.5 times upper level of normal (ULN)
Alkaline phosphatase ≥ 2.5 times ULN
Total bilirubin ≥ 1.5 times ULN
Creatinine clearance < 50 mL/min
INR >2
Known dihydropyrimidine dehydrogenase (DPD) deficiency
History of Gilbert's syndrome
Those who require therapeutic anticoagulation with coumarin-derivative anticoagulants
Unable to provide informed consent
Patients with a "currently active" second malignancy other than non-melanoma skin cancers, non-invasive bladder cancer, "low risk" adenocarcinoma of the prostate and carcinoma in situ of the cervix. Patients are not considered to have a "currently active" malignancy if they have completed therapy and are free of disease for ≥ 2 years.
Previous pelvic radiation therapy
Prior treatment with a topoisomerase I inhibitor (i.e. irinotecan, topotecan)

### Study Design

During Phase Ib, the safety will be evaluated and the MTD/RP2D of CRLX101 and capecitabine and radiation therapy (XRT) will be determined in patients with rectal cancer using the traditional 3+3 dose escalation design. Adverse events (AEs) will be evaluated via the CTCAE version 4.0. Patients in Phase Ib will also be followed for pathological response if they have resectable disease.

If CRLX101 can be safely administered in combination with capecitabine and radiation at doses ≥9 mg/m² IV in the Phase Ib study, then the trial will proceed to Phase II with a primary objective of estimating the rate of pCR. Patients with resectable primary disease and treated at the MTD/RP2D in Phase Ib will be included in the Phase II study population.

In Phase II, CRLX101 will be administered at the RP2D in combination with capecitabine and radiation in patients with locally advanced rectal cancer for a total of 5-6 weeks, depending on the total radiation dose. A total of 3 doses of CRLX101 will be administered every other week. Surgery will take place at least 6 weeks after the completion of chemoradiotherapy.

For the Phase II population, postoperative adjuvant therapy is indicated regardless of whether a pCR is achieved or not. While there are a number of regimens used in the adjuvant setting, national guidelines do not specify one of these regimens over the other. Given the consistent application of adjuvant therapies in this population, the Phase II patients will be followed for both DFS and OS.

### Phase Ib

The Phase Ib will follow a traditional 3+3 dose escalation design.

### Dose Escalation Rules

The first 3 patients enrolled (Dose Level 1, see Dose Cohort Table below) will receive 12 mg/m² IV of CRLX101 on D1 of weeks 1, 3 and 5 in combination with 5-6 weeks of radiation and oral capecitabine (Xeloda®). Patients will be enrolled sequentially confirming the absence of DLT within the first 2 weeks after dose 1 before enrolling the subsequent patient. If none of the initial 3 patients experiences DLT within 6 weeks from enrollment or 2 weeks post last dose of CRLX101, whichever is longer, then the next cohort of patients will receive 15 mg/m² of CRLX101 on D1 of weeks 1, 3 and 5 in combination with the study defined doses of capecitabine and radiation.

If 1 of the 3 patients in any cohort experiences DLT, then 3 additional patients will be enrolled into that same cohort. If only 1 of these 6 patients experiences DLT, subsequent patients will enroll into the next cohort. If 2 or more patients in a cohort experience DLT, the MTD is considered to have been exceeded. If 2 patients experience DLT in a cohort, 3 additional patients will be assigned to the lower dose level unless there have been 6 patients already at that dose level, or this occurs in Cohort (-2). NOTE: Dose levels (-1) and (-2) may be opened if dose cohort 1 is above the MTD. Dose level (-1) evaluates CRLX101 at 9 mg/m² with 825 mg/m² of capecitabine, while dose level (-2) reduces the capecitabine to 650 mg/m².

Patients who experience a DLT may continue on study provided they do not require a dose below dose level (-2).

### Dose Cohort Table

| **Dose Cohorts (Level)** | **Capecitabine** | **Radiation** | **CRLX101** |
|---|---|---|---|
| **-2** | Capecitabine 650 mg/m² twice daily five days per week | 180 cGy/day; 5 days (M-F) /week for 5-6 weeks | 9 mg/m² IV dayl of Week 1, 3 and 5 during radiation |
| **-1** | Capecitabine 825 mg/m² twice daily five days per week | 180 cGy/day; 5 days (M-F) /week for 5-6 weeks | 9 mg/m² IV dayl of Week 1, 3 and 5 during radiation |
| **1** | Capecitabine 825 mg/m² twice daily five days per week | 180 cGy/day; 5 days (M-F) /week for 5-6 weeks | 12 mg/m² IV dayl of Week 1, 3 and 5 during radiation |
| **2** | Capecitabine 825 mg/m² twice daily five days per week | 180 cGy/day; 5 days (M-F) /week for 5-6 weeks | 15 mg/m² IV dayl of Week 1, 3 and 5 during radiation |

### Definition and Evaluation of MTD

The MTD of CRLX101 in combination with capecitabine and radiation will be defined as the highest dose at which ≤1 out of 6 patients have experienced a DLT (defined below). No intra-patient dose escalation will be allowed.

### Definition of Dose Limiting Toxicity (DLT)

A DLT is defined as any of the following that occur within the 6 weeks from study start date (or 2 weeks after last dose of CRLX101, whichever is longer) of treatment and is considered related to study treatment:
- Grade ≥3 neutropenia lasting for ≥5 days
- Grade 3 or 4 neutropenia with fever (≥38.5°C)
- Grade 4 anemia not related to cancer-associated bleeding
- Grade 4 thrombocytopenia, or Grade 3 thrombocytopenia in the presence of clinically significant bleeding
- Grade ≥3 nausea or vomiting lasting >48 hours despite use of anti-emetics
- Grade 2 cystitis that does not resolve within 14 days; second occurrence of Grade 2 cystitis ; CRLX101 will be discontinued
- Grade 3 or 4 cystitis; CRLX101 will be discontinued
- Any incidence of diarrhea that requires a dose reduction in CRLX101 (see section 5.4)
- Any other non-hematologic toxicity Grade ≥3 of any duration that requires a dose reduction of either agent (see section 5.5)
- Radiotherapy interruption due to treatment-emergent adverse events ≥5 days; CRLX101 will be discontinued
- Dose interruption or reduction of capecitabine because of treatment-emergent adverse events that leads to administration of less than 50% of the scheduled capecitabine dose for the entire course of therapy.

### Results

The first three patients enrolled (Dose Cohort Level 1, see Dose Cohort Table above) received 12 mg/m² IV of CRLX101 on D1 of weeks 1, 3 and 5 in combination with the study defined doses of capecitabine and radiation. None of the three patients exhibited a DLT and thus, the MTD of CRLX101 was determined to be 15 mg/m2. Furthermore, one of the three patients from this cohort was classified as having a pCR.

The next cohort of patients enrolled (Dose Cohort Level 2, see Dose Cohort Table above) received 15 mg/m² of CRLX101 on D1 of weeks 1, 3 and 5 in combination with the study defined doses of capecitabine and radiation. To date four patients have received at least one dose at 15 mg/m², and two of these four patients have received more than one dose at 15 mg/m². As of September 24, 2014 no DLT has been reported in any of these four patients.

### Phase II:

If CRLX101 can be safely administered in combination with capecitabine and radiation at ≥ 9mg/m² IV then the trial will proceed to Phase II. CRLX101 will be administered at the RP2D in combination with capecitabine and radiation in patients with locally advanced rectal cancer for a total of 5 - 6 weeks. Surgery will take place at least 6 weeks after the completion of chemoradiotherapy.

### Chemoradiotherapy

### Capecitabine

Capecitabine 825mg/m² BID dosing will begin on day 1 of week 1, and continue Monday through Friday of each week of radiation, except for the last week in those with <T4 disease; this group will receive radiation and capecitabine Monday through Wednesday of week 6. Capecitabine should only be taken on radiation days. If radiation is delayed, capecitabine should be held until radiation resumes.

Capecitabine 150 mg and 500 mg tablets will be used for this study. Therefore, the calculated dose should be rounded to the closest 150 or 500 mg dose. The morning dose of capecitabine should be administered with water, and within 30 minutes after a light breakfast, at approximately the same time each day. The second dose of capecitabine should be administered approximately 12 hours later, and within 30 minutes after eating. Premedication is not required for capecitabine. Patients should be instructed to take standard of care anti-emetics and anti-diarrheas as needed.

### CRLX101 Dose and Administration

Each dose should be administered by IV infusion over 60 minutes on Day 1 (D1) of weeks 1, 3 and 5. The first -40 mL of the infusion should be given slowly over the first 10 minutes, and then accelerated to complete the infusion within -60 minutes.

The second and subsequent dose of CRLX101 may be delayed if related AEs have not resolved to grade 1 or better. In addition, if radiation is delayed, CRLX101 should be held until radiation resumes. If a dose of CRLX101 is delayed, then the subsequent dose should be administered 2 weeks later to avoid significant carry-over of unconjugated plasma CPT from one dose to the next. The last dose of CRLX101 should not be administered after the last day of radiation, even if this means dose 3 is omitted.

### Premedication and Hydration

Since hypersensitivity reactions have been reported during infusion of CRLX101, patients will be pre-medicated prior to each dose with a corticosteroid (e.g., oral dexamethasone 20 mg 12 and 6 hours prior, or 20 mg IV 30 minutes prior), an antihistamine (e.g. diphenhydramine 50 mg IV 30-60 minutes prior) and an H2 antagonist (e.g., ranitidine 50 mg or famotidine 20 mg IV 30-60 minutes prior).

Antiemetics with a 5-HT3 antagonist (e.g., dolasetron, granisetron or ondansetron) will also be administered 30-60 minutes prior to each CRLX101 infusion to reduce potential for chemo induced nausea.

To minimize cystitis, an expected toxicity of CRLX101, each patient will receive 1 liter of clinically suitable IV hydration just prior to and immediately following each CRLX101 infusion.

### Radiotherapy

This protocol allows physician discretion as to the use of Intensity Modulated Radiation Therapy (IMRT) or 3D conformal planning techniques.

### Regimen

Radiation begins on D1 of neoadjuvant chemotherapy and continues for 28 (if <T4) or 30 (T4 disease) consecutive weekdays. Patient will receive 1.8 Gy daily fractions of radiotherapy without a break except for weekends and holidays.

| **Field** | **Dose (Gy)** | **Number of fractions** | **Fraction size** | **Rx Length** | **Rx days** |
|---|---|---|---|---|---|
| Initial | 45 | 25 | 1.8 Gy | 25 days | Monday through Friday |
| Boost | 5.4 if <T4 | 3 if <T4 | | 3 days if <T4 | |
| | 9 (T4 disease) | 5 (T4 disease) | | 5 days (T4 disease) | |
| Total | 50.4 if <T4 | 28 if <T4 | | 28 days if <T4 | |
| | 54 (T4 disease) | 30(T4 disease) | | 30 days (T4 disease) | |

### Target Dose

### Prescription Isodose Surface

Dose is to be prescribed to an isodose surface that encompasses the planning target volume (PTV) and that satisfies the dose uniformity guidelines below. The minimum dose to PTV1 and PTV2 shall be no less than 95% of the protocol specified dose for that volume.

### Dose Definition

Dose is to be specified in cGy to muscle.

### Tissue Heterogeneity

Calculations shall take into account the effect of tissue heterogeneities.

### Prescription Dose and Fractionation

| | |
|---|---|
| *PTV1* | The total dose to the PTV1 will be 4,500 cGy in 25 fractions (180 cGy to the PTV1 each day). |
| *PTV2* | A cone down dose of 540 cGy will be delivered to PTV2 in 3 fractions of 180 cGy per day (total dose 5,040 cGy). If patient has T4 disease, investigator can prescribe a cone down dose of 9 Gy to be delivered to PTV2 in 5 fractions of 180 cGy per day (total dose 54 Gy). |

### Dose Uniformity

PTV1 and PTV2 shall both be encompassed within the isodose surface corresponding to 95% of the prescription dose for that volume. The maximal dose should be no more than 110% of the prescription dose; the maximal volume to receive 110% of the prescription dose should be kept below 10% of the PTV, as evaluated by dose volume histogram.

### Fractionation

Treatment shall be given 5 days per week, until the last week, when those with <T4 disease will receive radiation for 3 days.

### Rests and Interruptions

Uninterrupted radiation treatment is intended. Treatment may be interrupted for the development of acute toxicity defined as radiation-related AEs grade ≥3. The specific reason(s) for any treatment interruption must be recorded in the treatment chart and the electronic case report form (e-CRF). Treatment interruptions exceeding fourteen (14) days for reasons other than protocol-mandated interruptions for adverse events will be considered a major protocol deviation. Treatment interruption is mandated for ≥ Grade 3 diarrhea or other regional symptoms of severity > Grade 3 by NCI's Common Terminology Criteria for Adverse Events (CTCAE) v4. No modifications in dose will be made for interruptions in therapy.

### Supportive care during radiation therapy

Routine management of toxicities during combined CRT is allowed. Interventions may include, but are not limited to, the following:

| **Toxicity** | **Management** |
|---|---|
| Skin reactions: | Topical agents such as Aquaphor® or Biofene® |
| Diarrhea/enteritis: | Loperamide, diphenoxylate/atropine |
| Cystitis: | Phenazopyridine |
| Dehydration: | IV fluids as needed |

### Chemotherapy Dosing Delays/Dose Modifications

Each patient will be assessed periodically for the development of any toxicity. Toxicity will be assessed according to the NCI CTCAE v4.

If a patient experiences ≥2 AEs simultaneously that require different dose reductions, the lowest dose should be used. For capecitabine, reduce the dose to 650mg/m² (if at 825 mg/m²). If further dose reductions are required for capecitabine, subsequent doses should be reduced by 20%.

Two dose reductions of CRLX101 are permitted for patients experiencing prolonged adverse events (not including DLT event). Discontinue drug in patients requiring >2 dose reductions, or if doses <9 mg/m² are required or if the patient experiences a dose-limiting toxicity. These patients should continue on standard chemotherapy and radiation, and be followed up per protocol, with collection of tissue etc. if possible. NOTE: If radiation is delayed, capecitabine and CRLX101 should be held until radiation resumes. If either capecitabine or CRLX101 are held for toxicities, radiation should continue if clinically appropriate.

### CRLX101 Infusion Related/Hypersensitivity Reactions

If any infusion-related hypersensitivity reaction occurs, the CRLX101 infusion should be stopped immediately and the patient treated appropriately.

Infusion reactions will be defined according to the National Cancer Institute CTCAE (Version 4.0) definition of an allergic reaction/infusion reaction and anaphylaxis, as defined below:

| **Grade** | **Description** |
|---|---|
| 1 | Transient flushing or rash, drug fever <38° C (<100.4° F); intervention not indicated |
| 2 | Intervention or infusion interruption indicated; responds promptly to symptomatic treatment (e.g., antihistamines,NSAIDS, narcotics); prophylactic medications indicated for <24 hrs |
| 3 | Symptomatic bronchospasm, with or without urticaria; parenteral intervention indicated; allergy-related edema/angioedema; hypotension |
| 4 | Life-threatening consequences; urgent intervention indicated |

For patients who experience ≤Grade 2 infusion reactions, CRLX101 administration may resume at a slower rate of administration (e.g., 40mL/hour, then increased to complete within 2 hours), per the investigator's discretion, after the patient has recovered from the event on the same day. NOTE: the infusion must be completed within 6 hours of drug reconstitution. Infusions should not be restarted in patients experiencing ≥grade 3 infusion reactions.

### Hematologic Toxicity

Dose modifications are based on interval toxicity and day-of-treatment blood counts.

### Neutropenia

| **Neutropenia** | **First occurrence** | **Second occurrence** |
|---|---|---|
| Grade 1 (< lower limit of normal (LLN)-1500 cells/m³) | Maintain dose and schedule of CRLX101 and capecitabine (cape) | Maintain dose and schedule of CRLX101 and cape |
| Grade 2 (1000-<1500 cells/m³) | Maintain dose and schedule of cape | Maintain dose and schedule of cape |
| | Delay dose of CRLX101 until resolves to ≤ Grade 1. Resume at same dose | Delay CRLX101 until resolved ≤ Grade 1; then resume at one dose level below if duration is ≥5 days |
| Grade 3 (500-<1000 xcells/m³) | Interrupt doses of cape and delay CRLX101 until resolves to ≤ Grade 1. | Interrupt doses of cape and delay CRLX101 until resolves to ≤ Grade 1. Do not reduce dose of cape. If duration is ≥5 days then reduce CRLX101 by 1 dose level. |
| | For Phase 1b: If duration is ≥ 5 days, then discontinue CRLX101 (this is a DLT) | |
| | For Phase 2a: Do not reduce dose of cape. If duration is ≥5 days then reduce CRLX101 by 1 dose level. | |
| Grade 4 (<500 cells/m³) | Interrupt cape and delay CRLX101 until resolves to ≤grade 1. | Interrupt cape until resolves to ≤ grade 1, then resume at one dose level below. Discontinue CRLX101. |
| | For Phase 1b: If duration is ≥ 5 days, then discontinue CRLX101 (this is a DLT) | |
| | For Phase 2a: If duration is ≥ 5 days, then resume both at one dose level below | |
| Grade 3 or 4 neutropenia with fever (≥38.5°C | For Phase 1b: Discontinue CRLX101 | For Phase 2a: Interrupt cape until resolves, then resume at one dose level below. Discontinue CRLX101. |
| | For Phase 2a: Interrupt cape and delay CRLX101 until resolves; resume both at one dose level below | |

### Thrombocytopenia

| **Thrombocytopenia** | **Action Required** |
|---|---|
| Grade 1-2 (<75,000 cells/m³) | Maintain dose and schedule of CRLX101 and capecitabine |
| Grade 3 (25,000 to <75,000 cells/m³) with clinically significant bleeding or Grade 4 (<25,000 cells/m³) | Delay CRLX101 until resolves to ≤ Grade 2, then reduce CRLX101 by 1 dose level. |

### Anemia

| **Anemia** | **Action Required** |
|---|---|
| Grade 1-3 (<8 g/dL) | Maintain dose and schedule of CRLX101 and capecitabine |
| Grade 4 not related to cancer associated bleeding (life threatening) | Delay CRLX101 until resolves to ≤ Grade 3, then reduce CRLX101 by 1 dose level. |

### CRLX101-induced Cystitis

Pre- and post-dosing hydration should be administered to mitigate the potential for cystitis. Any patient who experiences persistent Grade 2 that returns to Grade 1 within 14 days will receive CRLX101 at the same dose. If after the next dose, the toxicity returns to ≥ Grade 2, the dose of CRLX101 should be delayed until the toxicity returns to ≤ Grade 1. Subsequent infusions of CRLX101 should be reduced by one dose level. For any Grade 2 cystitis that lasts for >2 weeks or for any incidence of Grade 3 or 4 cystitis, CRLX101 should be delayed until it returns to ≤ Grade 1. Subsequent infusions of CRLX101 should be reduced by one dose level.

### Diarrhea

For any grade diarrhea, see information on management below.

| **Diarrhea Grade** | **First Occurrence** | **Second Occurrence** |
|---|---|---|
| 1 | Maintain dose of capecitabine and CRLX101, refer to information below and in Appendix A for supportive care measures. | Maintain dose of capecitabine and CRLX101, refer to information below and in Appendix A for supportive care measures. |
| (Increase of <4 stools/day over baseline) | | |
| 2 | Interrupt capecitabine until resolved to ≤ grade 1, then resume without reducing the dose; maintain CRLX101 | Interrupt capecitabine and delay CRLX101 until resolved to ≤ grade 1, then resume each at one lower dose level. |
| (Increase of 4-6 stools/day over baseline) | | |
| 3 | Interrupt capecitabine and delay CRLX101 until resolved to ≤ grade 1, then resume capecitabine at one lower dose level; resume CRLX101 at same dose | Interrupt capecitabine and delay CRLX101 until resolved to ≤ grade 1, then resume each at one lower dose level; discontinue each if grade 3 diarrhea recurs a 3^{rd} time. |
| (Increase of ≥7 stools/day over baseline) | | |
| 4 | Interrupt treatment until resolved to ≤ grade 1, then resume each at one lower dose level. | Discontinue both agents and follow-up per protocol. |
| (Life-threatening consequences; urgent intervention indicated) | | |

It is recommended that patients be provided loperamide tablets (or prescription) at the start of their treatment. Patients should be instructed to first notify their physician/healthcare provider at onset of diarrhea of any severity, and to treat diarrhea with loperamide at its earliest occurrence (any grade).

An assessment of frequency, consistency and duration of diarrhea as well as knowledge of other symptoms such as fever, cramping, pain, nausea, vomiting, dizziness and thirst should be taken at baseline. Consequently patients at high risk of diarrhea can be identified. Patients should be educated on signs and symptoms of diarrhea with instructions to report any changes in bowel patterns to the physician.

For grade 3 or 4 diarrhea, or for < grade 3 diarrhea with complicating features (e.g. severe cramping, severe nausea/vomiting, decreased performance status, grade 3 or 4 neutropenia, sepsis, fever, frank bleeding, dehydration), hydrate aggressively with IV fluids as appropriate and consider octreotide therapy. Begin antibiotic therapy as needed for diarrhea longer than 24 hours or if there is fever or neutropenia. Maintain adequate hydration and begin dietary modifications according to institutional guidelines for diarrhea management.

### Other Non-hematologic Toxicity

| **Toxicity** | **First Occurrence** | **Second Recurrence** |
|---|---|---|
| **Hand and foot syndrome** | Interrupt capecitabine until resolution to ≤ grade 1, then resume capecitabine at the current dose level; maintain CRLX101 | Interrupt capecitabine until resolution to ≤ grade 1, then resume capecitabine at the current dose level; maintain CRLX101 |
| Grade 2: Skin changes (e.g., peeling,blisters, bleeding, edema, or hyperkeratosis) with pain; limiting instrumental activities of daily living (IADL) | | |
| **Hand and foot syndrome** | Interrupt capecitabine until resolution to ≤ grade 1, then resume capecitabine at a reduced dose; maintain CRLX101 | Discontinue capecitabine; maintain CRLX101 |
| Grade 3: Severe skin changes (e.g., peeling, blisters, bleeding, edema, or hyperke-ratosis)with pain; limiting self-care ADL | | |
| **Renal toxicity** | Interrupt capecitabine until resolution to ≤ grade 1, then resume capecitabine at the current dose level; maintain CRLX101 | Interrupt capecitabine until resolution to ≤ grade 1, then resume capecitabine at the current dose level; maintain CRLX101 |
| Grade 2: Serum Creatinine >1.5-3x upper limit of normal (ULN) | | |
| **Renal toxicity** | Delay CRLX101 until resolution to ≤ grade 1, then resume at a reduced dose level; **Note**:capecitabine dose should be reduced as it is renally eliminated; dose per investigator discretion. | Discontinue CRLX101 and follow-up per protocol; **Note**:capecitabine dose should be reduced as it is renally eliminated; dose per investigator discretion. |
| ≥ Grade 3: Serum creatinine >3X ULN | | |
| **Liver toxicity** | Interrupt capecitabine and delay CRLX101 treatment until resolution to ≤ grade 1, then resume at the current dose level. | Interrupt capecitabine and delay CRLX101 treatment until resolution to ≤ grade 1, then resume each at one lower dose level |
| Bilirubin ≥Grade 2: >ULN | | |
| **Liver toxicity** | Interrupt capecitabine and CRLX101 treatment until resolution to ≤ grade 1, then resume each at one lower dose level. | Discontinue CRLX101 and follow-up per protocol; capecitabine can continue at discretion of investigator. |
| Transaminase Grade 3 or 4; >5 X ULN | | |
| **Unspecified*clinically significant treatment-emergent non-hematologic toxicity** | Interrupt capecitabine and CRLX101 until resolved to ≤ grade 1, then resume each drug at one lower dose level | Interrupt capecitabine and CRLX101 until resolved to ≤ grade 1, then resume each drug at one lower dose level |
| ≥Grade 2 | | |
| *Clinically signficant as defined at discretion of investigator; study medications do not need to be modified for alopecia, or for any grade 2 nausea or vomiting, or for grade ≥3 nausea or vomiting that is controlled by anti-emetics and lasts ≤48 hours; | | |

### Data Analysis Plans

The rate of pCR will be defined as the total number of patients who are classified as having a pCR, divided by the number of treated evaluable patients. pCR will be determined based on assessment of the surgical specimen at the primary treatment site,. The pCR and pathological response rates will be reported along with its exact 95% confidence interval. Safety and tolerability will be characterized by NCI CTCAE v4.0 and will be summarized using frequency tables. Worst toxicity grades per patient will also be tabulated and reported. DFS and OS will be summarized using the method of Kaplan and Meier. DFS and OS will be compared between patients who achieve a pCR and those who do not using the log-rank test.

## Claims

1. A CDP-camptothecin conjugate, particle or composition for use in a method of treating rectal cancer in a subject, the method comprising:
providing an initial administration of said CDP-camptothecin conjugate, particle or composition to said subject at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m² (wherein said dosage is expressed in mg of drug, as opposed to mg of conjugate),
providing one or more subsequent administrations of said CDP-camptothecin conjugate, particle or composition at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m², wherein each subsequent administration is provided, independently, between 12, 13, 14, 15 or 16 days, after the previous administration,
providing multiple radiation treatments, wherein an initial radiation treatment is administered with the administration of said CDP-camptothecin conjugate, particle or composition of said CDP-camptothecin conjugate, particle or composition, and said radiation treatments are administered daily five days a week on weekdays for at least 25 to 35 days; and
administering multiple doses of a pyrimidine analogue, to thereby treat the rectal cancer.

2. The CDP-camptothecin conjugate, particle or composition for use of claim 1, wherein the rectal cancer is locally advanced rectal cancer, the rectal cancer is stage cT3-4N0 or cT1-4N+, or the rectal cancer is resectable.

3. The CDP-camptothecin conjugate, particle or composition for use of claim 1, wherein the CDP-camptothecin conjugate, particle or composition is administered at a dosage of 9 mg/m², 10 mg/m², 11 mg/m², 12 mg/m², 13 mg/m², 14 mg/m² or 15 mg/m² per administration.

4. The CDP-camptothecin conjugate, particle or composition for use of claim 1, wherein the CDP-camptothecin conjugate, particle or composition is administered at a dosage of 12 mg/m² or 15 mg/m² per administration.

5. The CDP-camptothecin conjugate, particle or composition for use of claim 1, wherein each subsequent administration of the CDP-camptothecin conjugate, particle or composition is provided, independently, 14 days, after the previous administration.

6. The CDP-camptothecin conjugate, particle or composition for use of claim 1, wherein: (i) the radiation treatment is administered at a dosage of 170 cGy to 190 cGy per treatment; (ii) the radiation treatment is administered at a dosage of 180 cGy per treatment; (iii) the radiation treatment is administered at a dosage of 180 cGy per day for five days; (iv) the radiation treatment is administered at a dosage of 180 cGy per day for five days on weekdays for 5 to 6 weeks; or (v) the radiation treatment is administered at a dosage of 180 cGy per day for five days on weekdays for 28 or 30 consecutive weekdays.

7. The CDP-camptothecin conjugate, particle or composition for use of claim 1, wherein the total amount of radiation given during the multiple radiation treatments is from about 4,500 cGy to about 5,400 cGy.

8. The CDP-camptothecin conjugate, particle or composition for use of claim 1, wherein the radiation treatment is pelvic radiation treatment.

9. The CDP-camptothecin conjugate, particle or composition for use of claim 1, wherein the radiation treatment is administered within about 24 hours, within about 22 hours, within about 20 hours, within about 18 hours, within about 16 hours, within about 14 hours, within about 12 hours, within about 10 hours, within about 8 hours, within about 6 hours, within about 4 hours, within about 2 hours or within about 1 hour, of administration of said CDP-camptothecin conjugate, particle or composition.

10. The CDP-camptothecin conjugate, particle or composition for use of claim 1, wherein the pyrimidine analogue is capecitabine.

11. The CDP-camptothecin conjugate, particle or composition for use of claim 10, wherein the capecitabine is administered at a dosage of 825 mg/m² twice daily five days per week on weekdays.

12. The CDP-camptothecin conjugate, particle or composition for use of claim 1, wherein the method further comprises administering an agent which ameliorates a side effect associated with the treatment, and optionally wherein: (i) the agent is administered in an amount sufficient to ameliorate bladder toxicity associated with treatment; (ii) the agent is selected from the group consisting of saline, D5 half normal saline and D5 water; (iii) the agent is administered prior to, during or after administration of the CDP-camptothecin conjugate, particle or composition; (iv) the agent is administered prior to administration of the CDP-camptothecin conjugate, particle or composition; (v) the agent is administered prior to and after administration of the CDP-camptothecin conjugate, particle or composition; (vi) the agent ameliorates a side effect associated with radiation treatment; or (vii) the agent is a radiation protector.

13. The CDP-camptothecin conjugate, particle or composition for use of claim 1, wherein the method further comprises obtaining a sample from the subject after an initial course of treatment, and determining if the subject has a pathological complete response (pCR), and optionally wherein: (i) the sample is a biopsy sample; or (ii) if the subject does not have a pCR after one course of treatment then the subject is administered one or more additional courses of treatment.

14. The CDP-camptothecin conjugate, particle or composition for use of claim 1, wherein the method comprises:
providing an initial administration of said CDP-camptothecin conjugate, particle or composition to said subject at a dosage of 12 mg/m² or 15 mg/m² (wherein said dosage is expressed in mg of drug, as opposed to mg of conjugate),
providing one or more subsequent administrations of said CDP-camptothecin conjugate, particle or composition at a dosage of 12 mg/m² or 15 mg/m², wherein each subsequent administration is provided, independently, between 14 days, after the previous administration,
providing multiple radiation treatments, wherein an initial radiation treatment is administered with the administration of said CDP-camptothecin conjugate, particle or composition and said radiation treatments are administered daily five days a week on weekdays for at least 25 to 35 days, and
administering multiple doses of capecitabine at a dosage of 825 mg/m² for five days on weekdays, to thereby treat the rectal cancer.

15. The CDP-camptothecin conjugate, particle or composition for use of claim 14, wherein the radiation treatment is administered within about 24 hours, within about 22 hours, within about 20 hours, within about 18 hours, within about 16 hours, within about 14 hours, within about 12 hours, within about 10 hours, within about 8 hours, within about 6 hours, within about 4 hours, within about 2 hours or within about 1 hour, of administration of said CDP-camptothecin conjugate, particle or composition.

## Patentansprüche

1. CDP-Camptothecin-Konjugat, -Partikel oder -Zusammensetzung zur Verwendung in einem Verfahren zur Behandlung des Rektumkrebses bei einem Patienten, wobei das Verfahren Folgendes umfasst:
Bereitstellen einer initialen Verabreichung von genanntem/genannten/genannter CDP-Camptothecin-Konjugat, -Partikeln oder -Zusammensetzung an den genannten Patienten in einer Dosierung von 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² oder 18 mg/m² (wobei genannte Dosierung in mg Arzneimittel im Gegensatz zu mg Konjugat ausgedrückt ist),
Bereitstellen von einer oder mehr anschließenden Verabreichung(en) von genanntem/genannten/genannter CDP-Camptothecin-Konjugat, -Partikeln oder -Zusammensetzung in einer Dosierung von 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² oder 18 mg/m2, wobei jede anschließende Verabreichung, unabhängig, zwischen 12, 13, 14, 15 oder 16 Tagen, nach der vorherigen Verabreichung bereitgestellt wird,
Bereitstellen mehrfacher Strahlenbehandlungen, wobei eine initiale Strahlenbehandlung mit der Verabreichung von genanntem/genannten/genannter CDP-Camptothecin-Konjugat, -Partikeln oder -Zusammensetzung von genanntem/genannten/genannter CDP-Camptothecin-Konjugat, -Partikeln oder -Zusammensetzung verabreicht wird und die genannten Strahlenbehandlungen täglich fünf Tage in der Woche an Wochentagen für mindestens 25 bis 35 Tage verabreicht werden; und
Verabreichen mehrfacher Dosen eines Pyrimidin-Analogons, um auf diese Weise den Rektumkrebs zu behandeln.

2. CDP-Camptothecin-Konjugat, -Partikel oder -Zusammensetzung zur Verwendung nach Anspruch 1, wobei der Rektumkrebs ein lokal fortgeschrittener Rektumkrebs ist, der Rektumkrebs sich im Stadium cT3-4N0 oder cT1-4N+ befindet, oder der Rektumkrebs resezierbar ist.

3. CDP-Camptothecin-Konjugat, -Partikel oder -Zusammensetzung zur Verwendung nach Anspruch 1, wobei das/die CDP-Camptothecin-Konjugat, -Partikel oder -Zusammensetzung in einer Dosierung von 9 mg/m², 10 mg/m², 11 mg/m², 12 mg/m², 13 mg/m², 14 mg/m² oder 15 mg/m² pro Verabreichung verabreicht wird/werden.

4. CDP-Camptothecin-Konjugat, -Partikel oder -Zusammensetzung zur Verwendung nach Anspruch 1, wobei das/die CDP-Camptothecin-Konjugat, -Partikel oder -Zusammensetzung in einer Dosierung von 12 mg/m² oder 15 mg/m² pro Verabreichung verabreicht wird/werden.

5. CDP-Camptothecin-Konjugat, -Partikel oder -Zusammensetzung zur Verwendung nach Anspruch 1, wobei jede anschließende Verabreichung des/der CDP-Camptothecin-Konjugats, -Partikel oder -Zusammensetzung, unabhängig, 14 Tage, nach der vorherigen Verabreichung bereitgestellt wird.

6. CDP-Camptothecin-Konjugat, -Partikel oder -Zusammensetzung zur Verwendung nach Anspruch 1, wobei: (i) die Strahlenbehandlung in einer Dosierung von 170 cGy bis 190 cGy pro Behandlung verabreicht wird; (ii) die Strahlenbehandlung in einer Dosierung von 180 cGy pro Behandlung verabreicht wird; (iii) die Strahlenbehandlung in einer Dosierung von 180 cGy pro Tag für 5 Tage verabreicht wird; (iv) die Strahlenbehandlung in einer Dosierung von 180 cGy pro Tag für fünf Tage an Wochentagen für 5 bis 6 Wochen verabreicht wird; oder (v) die Strahlenbehandlung in einer Dosierung von 180 cGy pro Tag für fünf Tage an Wochentagen für 28 oder 30 aufeinanderfolgende Wochentage verabreicht wird.

7. CDP-Camptothecin-Konjugat, -Partikel oder -Zusammensetzung zur Verwendung nach Anspruch 1, wobei die Gesamtmenge der während der mehrfachen Strahlenbehandlungen gegebenen Bestrahlung von ca. 4.500 cGy bis ca. 5.400 cGy beträgt.

8. CDP-Camptothecin-Konjugat, -Partikel oder -Zusammensetzung zur Verwendung nach Anspruch 1, wobei die Strahlenbehandlung eine Beckenstrahlenbehandlung ist.

9. CDP-Camptothecin-Konjugat, -Partikel oder -Zusammensetzung zur Verwendung nach Anspruch 1, wobei die Strahlenbehandlung innerhalb ca. 24 Stunden, innerhalb ca. 22 Stunden, innerhalb ca. 20 Stunden, innerhalb ca. 18 Stunden, innerhalb ca. 16 Stunden, innerhalb ca. 14 Stunden, innerhalb ca. 12 Stunden, innerhalb ca. 10 Stunden, innerhalb ca. 8 Stunden, innerhalb ca. 6 Stunden, innerhalb ca. 4 Stunden, innerhalb ca. 2 Stunden oder innerhalb ca. 1 Stunde der Verabreichung von genanntem/genannten/genannter CDP-Camptothecin-Konjugat, -Partikeln oder -Zusammensetzung verabreicht wird.

10. CDP-Camptothecin-Konjugat, -Partikel oder -Zusammensetzung zur Verwendung nach Anspruch 1, wobei das Pyrimidin-Analogon Capecitabin ist.

11. CDP-Camptothecin-Konjugat, -Partikel oder -Zusammensetzung zur Verwendung nach Anspruch 10, wobei das Capecitabin in einer Dosierung von 825 mg/m² zweimal täglich fünf Tage pro Woche an Wochentagen verabreicht wird.

12. CDP-Camptothecin-Konjugat, -Partikel oder -Zusammensetzung zur Verwendung nach Anspruch 1, wobei das Verfahren weiter die Verabreichung eines Mittels umfasst, welches eine behandlungsbedingte Nebenwirkung bessert, und gegebenenfalls wobei: (i) das Mittel in einer Menge verabreicht wird, die zur Besserung der behandlungsbedingten Harnblasentoxizität ausreichend ist; (ii) das Mittel aus der Gruppe ausgewählt ist, bestehend aus einer Salzlösung, D5 Halbnormal-Salzlösung und D5 Wasser; (iii) das Mittel vor, während oder nach Verabreichung des/der CDP-Camptothecin-Konjugats, -Partikel oder -Zusammensetzung verabreicht wird; (iv) das Mittel vor Verabreichung des/der CDP-Camptothecin-Konjugats, -Partikel oder -Zusammensetzung verabreicht wird; (v) das Mittel vor und nach Verabreichung des/der CDP-Camptothecin-Konjugats, -Partikel oder -Zusammensetzung verabreicht wird; (vi) das Mittel eine strahlenbehandlungsbedingte Nebenwirkung bessert, oder (vii) das Mittel ein Strahlenschutzmittel ist.

13. CDP-Camptothecin-Konjugat, -Partikel oder -Zusammensetzung zur Verwendung nach Anspruch 1, wobei das Verfahren weiter die Entnahme einer Probe von dem Patienten nach einem initialen Behandlungszyklus umfasst, und Bestimmung, ob bei dem Patienten eine pathologisch komplette Remission (pCR) vorliegt, und gegebenenfalls wobei: (i) die Probe eine Biopsieprobe ist; oder (ii) falls der Patient nach einem Behandlungszyklus keine pCR aufweist, dem Patienten dann ein oder mehr zusätzliche(r) Behandlungszyklus/Behandlungszyklen verabreicht wird/werden.

14. CDP-Camptothecin-Konjugat, -Partikel oder -Zusammensetzung zur Verwendung nach Anspruch 1, wobei das Verfahren Folgendes umfasst:
Bereitstellen einer initialen Verabreichung von genanntem/genannten/genannter CDP-Camptothecin-Konjugat, -Partikeln oder -Zusammensetzung an den genannten Patienten in einer Dosierung von 12 mg/m² oder 15 mg/m² (wobei genannte Dosierung in mg Arzneimittel im Gegensatz zu mg Konjugat ausgedrückt ist),
Bereitstellen von einer oder mehr anschließenden Verabreichung(en) von genanntem/genannten/genannter CDP-Camptothecin-Konjugat, -Partikeln oder -Zusammensetzung in einer Dosierung von 12 mg/m² oder 15 mg/m², wobei jede anschließende Verabreichung, unabhängig, zwischen 14 Tagen nach der vorherigen Verabreichung bereitgestellt wird,
Bereitstellen mehrfacher Strahlenbehandlungen, wobei eine initiale Strahlenbehandlung mit der Verabreichung von genanntem/genannten/genannter CDP-Camptothecin-Konjugat, -Partikeln oder -Zusammensetzung verabreicht wird und die genannten Strahlenbehandlungen täglich fünf Tage in der Woche an Wochentagen für mindestens 25 bis 35 Tage verabreicht werden, und
Verabreichen mehrfacher Dosen Capecitabin in einer Dosierung von 825 mg/m² für fünf Tage an Wochentagen, um auf diese Weise den Rektumkrebs zu behandeln.

15. CDP-Camptothecin-Konjugat, -Partikel oder -Zusammensetzung zur Verwendung nach Anspruch 14, wobei die Strahlenbehandlung innerhalb ca. 24 Stunden, innerhalb ca. 22 Stunden, innerhalb ca. 20 Stunden, innerhalb ca. 18 Stunden, innerhalb ca. 16 Stunden, innerhalb ca. 14 Stunden, innerhalb ca. 12 Stunden, innerhalb ca. 10 Stunden, innerhalb ca. 8 Stunden, innerhalb ca. 6 Stunden, innerhalb ca. 4 Stunden, innerhalb ca. 2 Stunden oder innerhalb ca. 1 Stunde der Verabreichung des/der genannten CDP-Camptothecin-Konjugats, -Partikel oder -Zusammensetzung verabreicht wird.

## Revendications

1. Conjugué, particule ou composition de CDP-camptothécine destiné(e) à une utilisation dans une méthode de traitement d'un cancer rectal chez un sujet, la méthode comprenant :
le fait de pratiquer une administration initiale dudit conjugué, de ladite particule ou de ladite composition de CDP-camptothécine audit sujet à une dose de 6 mg/m², 7 mg/m², 8 mg/m², 9 mg/m², 10 mg/m², 11 mg/m², 12 mg/m² , 13 mg/m², 14 mg/m², 15 mg/m², 16 mg/m², 17 mg/m² ou 18 mg/m² (ladite dose étant exprimée en mg de médicament, et non en mg de conjugué),
le fait de pratiquer une ou plusieurs administrations ultérieures dudit conjugué, de ladite particule ou de ladite composition de CDP-camptothécine à une dose de 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² ou 18 mg/m², chaque administration ultérieure étant pratiquée, indépendamment, entre 12, 13, 14, 15 ou 16 jours, après l'administration précédente,
le fait de pratiquer de multiples traitements par radiothérapie, un traitement par radiothérapie initial étant administré avec l'administration dudit conjugué, de ladite particule ou de ladite composition de CDP-camptothécine, et lesdits traitements par radiothérapie étant administrés quotidiennement cinq jours par semaine en semaine pendant au moins 25 à 35 jours ; et
l'administration de doses multiples d'un analogue de la pyrimidine, pour traiter de ce fait le cancer rectal.

2. Conjugué, particule ou composition de CDP-camptothécine destiné(e) à une utilisation selon la revendication 1, le cancer rectal étant un cancer rectal localement avancé, le cancer rectal étant au stade cT3-4N0 ou cT1-4N+, ou le cancer rectal étant résécable.

3. Conjugué, particule ou composition de CDP-camptothécine destiné(e) à une utilisation selon la revendication 1, le conjugué, la particule ou la composition de CDP-camptothécine étant administré(e) à une dose de 9 mg/m², 10 mg/m², 11 mg/m², 12 mg/m², 13 mg/m², 14 mg/m² ou 15 mg/m² par administration.

4. Conjugué, particule ou composition de CDP-camptothécine destiné(e) à une utilisation selon la revendication 1, le conjugué, la particule ou la composition de CDP-camptothécine étant administré(e) à une dose de 12 mg/m² ou 15 mg/m² par administration.

5. Conjugué, particule ou composition de CDP-camptothécine destiné(e) à une utilisation selon la revendication 1, chaque administration ultérieure du conjugué, de la particule ou de la composition de CDP-camptothécine étant pratiquée, indépendamment, 14 jours après l'administration précédente.

6. Conjugué, particule ou composition de CDP-camptothécine destiné(e) à une utilisation selon la revendication 1, (i) le traitement par radiothérapie étant administré à une dose de 170 cGy à 190 cGy par traitement ; (ii) le traitement par radiothérapie étant administré à une dose de 180 cGy par traitement ; (iii) le traitement par radiothérapie étant administré à une dose de 180 cGy par jour pendant cinq jours ; (iv) le traitement par radiothérapie étant administré à une dose de 180 cGy par jour pendant cinq jours en semaine pendant 5 à 6 semaines ; ou (v) le traitement par radiothérapie étant administré à une dose de 180 cGy par jour pendant cinq jours en semaine pendant 28 ou 30 jours en semaine consécutifs.

7. Conjugué, particule ou composition de CDP-camptothécine destiné(e) à une utilisation selon la revendication 1, la quantité totale de rayonnement administrée durant les multiples traitements par radiothérapie étant d'environ 4 500 cGy à environ 5 400 cGy.

8. Conjugué, particule ou composition de CDP-camptothécine destiné(e) à une utilisation selon la revendication 1, le traitement par radiothérapie étant un traitement par radiothérapie pelvienne.

9. Conjugué, particule ou composition de CDP-camptothécine destiné(e) à une utilisation selon la revendication 1, le traitement par radiothérapie étant administré dans les 24 heures environ, dans les 22 heures environ, dans les 20 heures environ, dans les 18 heures environ, dans les 16 heures environ, dans les 14 heures environ, dans les 12 heures environ, dans les 10 heures environ, dans les 8 heures environ, dans les 6 heures environ, dans les 4 heures environ, dans les 2 heures environ, ou dans une période de 1 heure environ, suivant l'administration dudit conjugué, de ladite particule ou de ladite composition de CDP-camptothécine.

10. Conjugué, particule ou composition de CDP-camptothécine destiné(e) à une utilisation selon la revendication 1, l'analogue de la pyrimidine étant la capécitabine.

11. Conjugué, particule ou composition de CDP-camptothécine destiné(e) à une utilisation selon la revendication 10, la capécitabine étant administrée à une dose de 825 mg/m² deux fois par jour cinq jours par semaine en semaine.

12. Conjugué, particule ou composition de CDP-camptothécine destiné(e) à une utilisation selon la revendication 1, la méthode comprenant en outre l'administration d'un agent qui améliore un effet secondaire associé au traitement et, optionnellement, (i) l'agent étant administré dans une quantité suffisante pour améliorer une toxicité vésicale associée au traitement ; (ii) l'agent étant sélectionné dans le groupe constitué d'une solution saline, d'une solution saline demi-normale D5 et d'eau D5 ; (iii) l'agent étant administré avant, pendant ou après l'administration du conjugué, de la particule ou de la composition de CDP-camptothécine ; (iv) l'agent étant administré avant l'administration du conjugué, de la particule ou de la composition de CDP-camptothécine ; (v) l'agent étant administré avant et après l'administration du conjugué, de la particule ou de la composition de CDP-camptothécine ; (vi) l'agent améliorant un effet secondaire associé au traitement par radiothérapie ; ou (vii) l'agent ayant un effet de protection contre les rayonnements.

13. Conjugué, particule ou composition de CDP-camptothécine destiné(e) à une utilisation selon la revendication 1, la méthode comprenant en outre l'obtention d'un échantillon à partir du sujet après une série initiale de traitements, et le fait de déterminer si le sujet présente une réponse pathologique complète (RPC), et optionnellement, (i) l'échantillon étant un échantillon de biopsie ; ou (ii) si le sujet ne présente pas de RPC après une série de traitements, une ou plusieurs séries de traitements additionnelles étant administrées au sujet.

14. Conjugué, particule ou composition de CDP-camptothécine destiné(e) à une utilisation selon la revendication 1, la méthode comprenant :
le fait de pratiquer une administration initiale dudit conjugué, de ladite particule ou de ladite composition de CDP-camptothécine audit sujet à une dose de 12 mg/m² ou 15 mg/m² (ladite dose étant exprimée en mg de médicament, et non en mg de conjugué),
le fait de pratiquer une ou plusieurs administrations ultérieures dudit conjugué, de ladite particule ou de ladite composition de CDP-camptothécine à une dose de 12 mg/m² ou 15 mg/m², chaque administration ultérieure étant pratiquée, indépendamment, entre 14 jours, après l'administration précédente,
le fait de pratiquer de multiples traitements par radiothérapie, un traitement par radiothérapie initial étant administré avec l'administration dudit conjugué, de ladite particule ou de ladite composition de CDP-camptothécine, et lesdits traitements par radiothérapie étant administrés quotidiennement cinq jours par semaine en semaine pendant au moins 25 à 35 jours, et
l'administration de doses multiples de capécitabine à une dose de 825 mg/m² pendant cinq jours en semaine, pour traiter de ce fait le cancer rectal.

15. Conjugué, particule ou composition de CDP-camptothécine destiné(e) à une utilisation selon la revendication 14, le traitement par radiothérapie étant administré dans les 24 heures environ, dans les 22 heures environ, dans les 20 heures environ, dans les 18 heures environ, dans les 16 heures environ, dans les 14 heures environ, dans les 12 heures environ, dans les 10 heures environ, dans les 8 heures environ, dans les 6 heures environ, dans les 4 heures environ, dans les 2 heures environ, ou dans une période de 1 heure environ, suivant l'administration dudit conjugué, de ladite particule ou de ladite composition de CDP-camptothécine.
